# EUROPEAN PATENT APPLICATION

(11) **EP 4 635 492 A2**
(43) Date of publication of application: **22.10.2025**
(21) Application number: 25199520.5
(22) Date of filing: 09.08.2023
(51) Int. Cl.: A61K 31/33

(54) **TRYPTAMINES AND METHODS OF TREATING MOOD DISORDERS**

(30) Priority: 11.08.2022 US 202263397037 P
(62) Divisional of application: 23853304.6
(71) Applicant: Gilgamesh Pharmaceuticals, Inc., New York, NY 10003 (US)
(72) Inventor: KRUEGEL, Andrew Carry, Millington, 07946 (US); CUNNINGHAM, Michael, Asheville, 28804 (US)
(74) Representative: Modiano, Gabriella Diana

(57) **Abstract**

Novel tryptamine compounds according to Formulas (1), (2), (3), (4), (5), and (6) and sub-formulas thereof, pharmaceutical salts thereof, and pharmaceutical compositions thereof, and methods of treating mood disorders, including depressive disorders and bipolar disorders, comprising administering a therapeutically effective amount of a compound according to any one of Formulas (1), (2), (3), (4), (5), and (6) or a pharmaceutical composition thereof to a subject in need thereof.

## Description

### BACKGROUND

Depression is a common psychological problem and refers to a mental state of low mood and aversion to activity. Various symptoms associated with depression include persistent anxious or sad feelings, feelings of helplessness, hopelessness, pessimism, and/or worthlessness, low energy, restlessness, irritability, fatigue, loss of interest in pleasurable activities or hobbies, excessive sleeping, overeating, appetite loss, Insomnia, thoughts of suicide, and suicide attempts. The presence, severity, frequency, and duration of the above-mentioned symptoms vary on a case-by-case basis.

Approximately one third of patients with major depressive disorder (MDD) fail to achieve remission of their symptoms, even after multiple rounds of treatment with several known classes of antidepressants, including selective serotonin reuptake inhibitors (SSRIs) (Rush *et al.* 2006). This high prevalence of treatment-resistant depression (TRD) makes clear the need for new, more efficacious pharmacotherapies for depression that will target new mechanisms and/or patient populations.

Tryptamines are monoamine alkaloids that contain an indole ring and are structurally similar to the amino acid tryptophan, from which the name derives. There are a significant number of tryptamine compounds that include naturally occurring compounds and chemical derivatives with similar structure that may be ring unsubstituted or ring substituted. Many tryptamines are 5-HT2A receptor agonists and/or modulators of other serotonin receptors and are known to be psychoactive and, in many cases, cause prolonged hallucinations. The most well-known tryptamines are psychedelic compounds, including compounds derived from entheogenic fungi (psilocybin and psilocin), N,N-dimethyltryptamine (DMT), lysergic acid diethylamide (LSD), 5-methoxy-N,N-dimethyltryptamine (5-MeO-DMT), bufotenin, and ibogaine. These compounds are known to have significant effects on thought, perception, and behavior. However, these compounds are currently classified as Schedule I drugs under the Controlled Substances Act due to their high abuse potential, no accepted medical use, and lack of established safety. Moreover, tryptamines are metabolized by a number of pathways, in some cases including monoamine oxidase, limiting the oral bioavailability of some compounds and resulting in very short durations of action. Conversely, other tryptamines have very long durations of action, which makes them challenging to use in a guided therapy setting, where supervised sessions of many hours in duration are costly and inconvenient for patients and healthcare providers. Accordingly, there remains a need for safe and effective tryptamine compounds that can reliably be used for the treatment of mood disorders.

### SUMMARY

In a first aspect, the present disclosure is directed to compounds of the following structure: 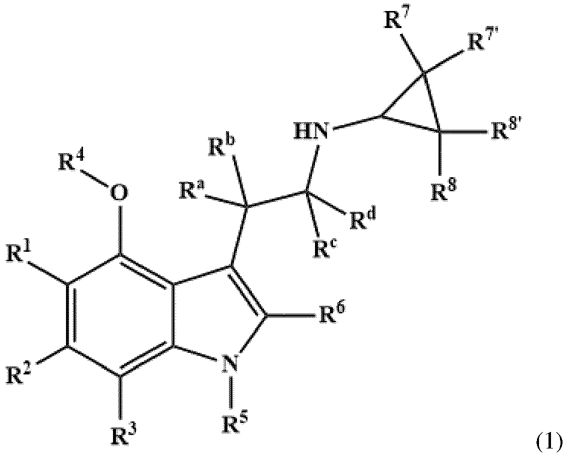 and pharmaceutically acceptable salts thereof.

In Formula (1), R¹, R², R³, and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms; R⁴ is selected from the group consisting of H and esterifying groups that result in OR⁴ being an ester group; R⁵ is selected from the group consisting of H and hydrocarbon groups containing 1-5 carbon atoms (R); R⁷, R^{7'}, R⁸, and R^{8'} are independently selected from H and F atoms; and R^{a}, R^{b}, R^{c}, and R^{d} are independently selected from H and D atoms. The present disclosure also includes pharmaceutical compositions of compounds of Formula (1) and methods of using the same.

In a second aspect, the present disclosure is directed to compounds of the following structure: 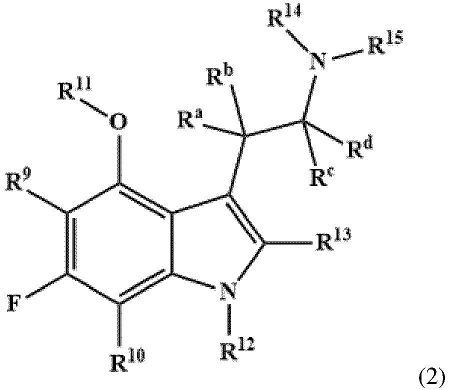 and pharmaceutically acceptable salts thereof.

In Formula (2), R⁹, R¹⁰, and R¹³ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms; R¹¹ is selected from the group consisting of H and esterifying groups that result in OR¹¹ being an ester group; R¹² is isopropyl or cyclopropyl with optional substitution with one or more fluorine atoms; R¹⁴ and R¹⁵ are independently selected from the group consisting of H and hydrocarbon groups containing 1-5 carbon atoms (R), which are optionally substituted with one or more fluorine atoms; and R^{a}, R^{b}, R^{c}, and R^{d} are independently selected from H and D atoms; provided that, when R⁹, R¹⁰, R¹¹, R¹³, R^{a}, R^{b}, R^{c}, and R^{d} are H and R¹² is isopropyl, then R¹⁴ and R¹⁵ are not both methyl. The present disclosure also includes pharmaceutical compositions of compounds of Formula (2) and methods of using the same.

In a third aspect, the present disclosure is directed to compounds of the following structure: 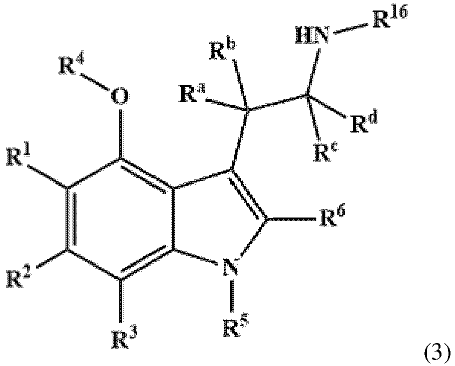 and pharmaceutically acceptable salts thereof.

In Formula (3), R¹, R², R³, and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms; R⁴ is selected from the group consisting of H and esterifying groups that result in OR⁴ being an ester group; R⁵ is selected from the group consisting of H and hydrocarbon groups containing 1-5 carbon atoms (R); R^{a}, R^{b}, R^{c}, and R^{d} are independently selected from H and D atoms; and R¹⁶ is an allyl or propargyl group. The present disclosure also includes pharmaceutical compositions of compounds of Formula (3) and methods of using the same.

In a fourth aspect, the present disclosure is directed to compounds of the following structure:

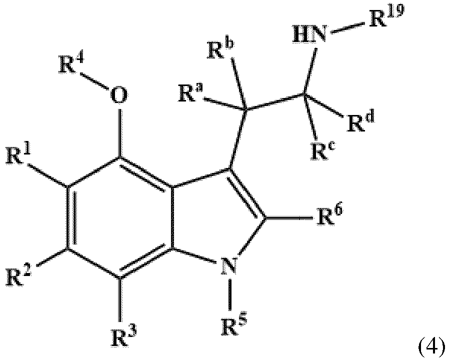

In Formula (4), R¹ is selected from the group consisting of: F, Cl, and methyl; R², R³, and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms; R⁴ is selected from the group consisting of H and esterifying groups that result in OR⁴ being an ester group; R⁵ is selected from the group consisting of H and hydrocarbon groups containing 1-5 carbon atoms (R); R^{a}, R^{b}, R^{c}, and R^{d} are independently selected from H and D atoms; and R¹⁹ is selected from the group consisting of H and hydrocarbon groups containing 1-5 carbon atoms (R), which are optionally substituted with one or more fluorine atoms. In embodiments, the following first provision applies: when R¹ is F or methyl, and R², R³, R⁴, R⁶, R¹⁹, R^{a}, R^{b}, R^{c}, and R^{d} are all H, then R⁵ is not H or methyl. In embodiments, the following second provision applies: when R¹ is Cl, and R², R³, R⁴, R⁶, R¹⁹, R^{a}, R^{b}, R^{c}, and R^{d} are all H, then R⁵ is not H, methyl, or ethyl. In embodiments, the following third provision applies: when R¹ is F, and R², R³, R⁴, R⁶, R^{a}, R^{b}, R^{c}, and R^{d} are all H, then R⁵ and R¹⁹ are not both methyl. Any one, two, or all three of the foregoing first, second, and third provisions may be combined. The present disclosure also includes pharmaceutical compositions of compounds of Formula (4) and methods of using the same.

In a fifth aspect, the present disclosure is directed to compounds of the following structure:

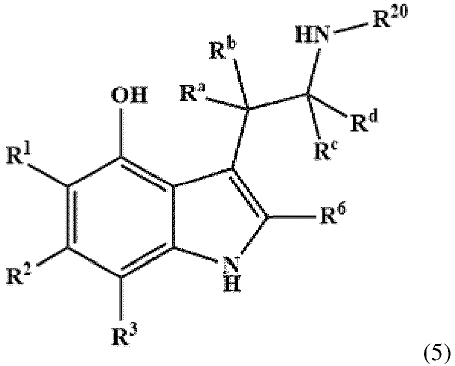

In Formula (5), R¹, R³, and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms; R² is selected from the group consisting of: F, Cl, and methyl; R^{a}, R^{b}, R^{c}, and R^{d} are independently selected from H and D atoms; and R²⁰ is selected from the group consisting of H and hydrocarbon groups containing 1-5 carbon atoms (R), which are optionally substituted with one or more fluorine atoms. In embodiments, the following provision applies: when R² is F, R³ is not methyl or OH. The present disclosure also includes pharmaceutical compositions of compounds of Formula (5) and methods of using the same.

In a sixth aspect, the present disclosure is directed to compounds of the following structure:

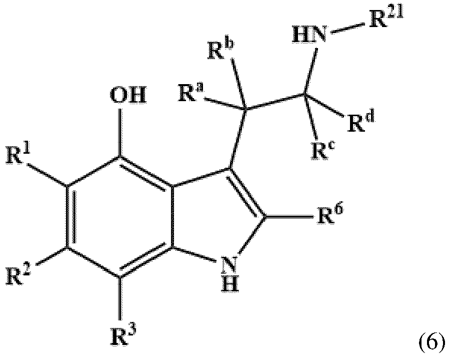

In Formula (6), R¹, R², and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms; R³ is selected from the group consisting of: F, Cl, and methyl; R^{a}, R^{b}, R^{c}, and R^{d} are independently selected from H and D atoms; and R²¹ is selected from the group consisting of H and hydrocarbon groups containing 1-5 carbon atoms (R), which are optionally substituted with one or more fluorine atoms. In embodiments, the following first provision applies: when R³ is methyl, R² is not F and R⁶ is not methyl. In embodiments, the following second provision applies: when R³ is methyl, and R¹, R², R⁶, R^{a}, R^{b}, R^{c}, and R^{d} are all H, then R²¹ is not H, methyl, or ethyl. In embodiments, the following third provision applies: when R³ is F or Cl, and R¹, R², R⁶, R^{a}, R^{b}, R^{c}, and R^{d} are all H, then R²¹ is not H. Any one, two, or all three of the foregoing first, second, and third provisions may be combined. The present disclosure also includes pharmaceutical compositions of compounds of Formula (6) and methods of using the same.

In a seventh aspect, the present disclosure includes methods of treating mood disorders in a patient in need thereof comprising administering a therapeutically effective amount of any compound of Formula (1), (2), (3), (4), (5), or (6), or a pharmaceutical composition thereof. In embodiments, the methods and compositions may treat mood disorders that include depressive disorders, bipolar and related disorders, substance-related disorders, and/or anxiety disorders. In embodiments, the depressive disorder is a treatment-resistant depressive disorder. In embodiments, the methods and compositions may treat mood disorders that include obsessive-compulsive and related disorders. In embodiments, the methods and compositions may treat mood disorders that include trauma- and stressor-related disorders. In embodiments, the methods and compositions may treat mood disorders that include feeding and eating disorders. In embodiments, the methods and compositions may treat mood disorders that include neurocognitive disorders. In embodiments, the methods and compositions may treat mood disorders that include neurodevelopmental disorders. In embodiments, the methods and compositions may treat mood disorders that include personality disorders. In embodiments, the methods and compositions may treat mood disorders that include sexual dysfunctions. In embodiments, the methods and compositions may treat mood disorders that include gender dysphoria.

### BRIEF DESCRIPTION OF THE DRAWINGS

The file of this patent or application contains at least one drawing executed in color. Copies of this patent or application publication with color drawing(s) will be provided by the Patent and Trademark Office upon request and payment of the necessary fee.

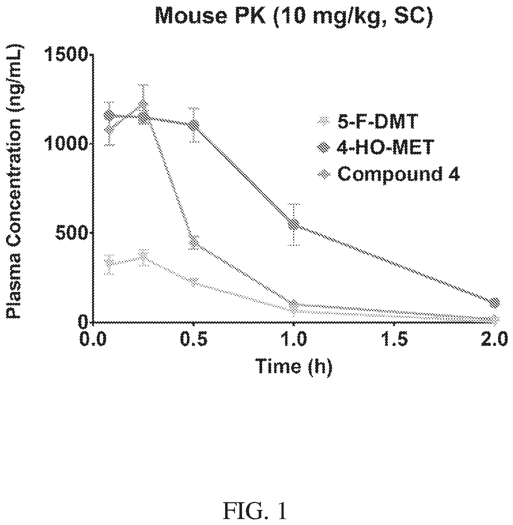

**FIG.** 1 depicts the plasma pharmacokinetics of test compounds in male C57BL/6 mice after subcutaneous (SC) administration (10 mg/kg).

### DETAILED DESCRIPTION

As used herein, the term "hydrocarbon group" (also denoted by the group R) is defined as a chemical group composed solely of carbon and hydrogen, except that the hydrocarbon group may (i.e., optionally) be substituted with one or more fluorine atoms to result in partial or complete fluorination of the hydrocarbon group. In some embodiments, the hydrocarbon group is composed of only carbon and hydrogen atoms, i.e., with fluorine atoms excluded. In other embodiments, the hydrocarbon group is a partially or fully fluorinated hydrocarbon group, such as fluorinated linear, branched, or cyclic alkyl group, as described in further detail below. The hydrocarbon group typically contains one, two, three, four, or five carbon atoms or a number of carbon atoms within a range bounded by any two of the foregoing values (e.g., 1-5, 1-4, 1-3, or 1-2 carbon atoms). Hydrocarbon groups in different compounds described herein, or in different positions of a compound, may possess the same or different number (or preferred range thereof) of carbon atoms in order to independently adjust or optimize the physical properties or treatment efficacy of the compound.

In one embodiment, the hydrocarbon group (R) is a linear, branched, or cyclic alkyl group. Some examples of linear alkyl groups containing 1-5 carbon atoms include methyl, ethyl, n-propyl, n-butyl, and n-pentyl and partially or fully fluorinated versions thereof. Some specific examples of fluorinated linear alkyl groups include fluoromethyl, difluoromethyl, trifluoromethyl, 2-fluoroethyl, 1-fluoroethyl, 2,2-difluoroethyl, 1,1-difluoroethyl, 1,2-difluoroethyl, 2,2,2-trifluoroethyl, pentafluoroethyl (perfluoroethyl), 3-fluoro-n-propyl, 3,3-difluoro-n-propyl, 3,3,3-trifluoro-n-propyl, 1,1,-difluoro-n-propyl, heptafluoro-n-propyl, 4-fluoro-n-butyl, 4,4-difluoro-n-butyl, 4,4,4-trifluoro-n-butyl, 1,1-difluoro-n-butyl, perfluoro-n-propyl, 5-fluoro-n-pentyl, 5,5-difluoro-n-pentyl, 5,5,5-trifluoro-n-pentyl, 1,1-difluoro-n-pentyl, 1,1,2,2-tetrafluoro-n-pentyl, and perfluoro-n-pentyl). Some examples of branched alkyl groups containing 1-5 carbon atoms include isopropyl (2-propyl), isobutyl (2-methylprop-1-yl), sec-butyl (2-butyl), t-butyl (1,1-dimethylethyl-1-yl), 2-pentyl, 3-pentyl, isopentyl (3-methylbut-1-yl), 1,2-dimethylprop-1-yl, 1,1-dimethylprop-1-yl, and neopentyl (2,2-dimethylprop-1-yl) and partially or fully fluorinated versions thereof. Some specific examples of fluorinated branched alkyl groups include 2-fluoroisopropyl, 1-fluoroisopropyl, 1,2-difluoroisopropyl, 1,1'-difluoroisopropyl, 1,1,1-trifluoroisopropyl, 1,1,1,1',1',1'-hexafluoroisopropyl, perfluoroisopropyl, 3-fluoroisobutyl, 3,3-difluoroisobutyl, 3,3,3-trifluoroisobutyl, and 3,3,3,3',3',3'-hexafluoroisobutyl. Some examples of cyclic alkyl groups containing 3-5 carbon atoms include cyclopropyl, cyclobutyl, and cyclopentyl and partially or fully fluorinated versions thereof. Some specific examples of fluorinated cyclic alkyl groups include 2-fluorocycloprop-1-yl, 1-fluorocycloprop-1-yl, 2,2-difluorocycloprop-1-yl, 2,3-difluorocycloprop-1-yl, 2,2,3-difluorocycloprop-1-yl, 2,2,3,3-tetrafluorocycloprop-1-yl, 2-fluorocyclobut-1-yl, 3-fluorocyclobut-1-yl, 2,3-difluorocyclobut-1-yl, 2,2-difluorocyclobut-1-yl, 3,3-difluorocyclobut-1-yl, 2,4-difluorocyclobut-1-yl, 2,3,4-trifluorocyclobut-1-yl, 2,2,4,4-tetrafluorocyclobut-1-yl, 2,2,3,3,4,4-hexafluorocyclobut-1-yl, 2-fluorocyclopent-1-yl, 2,5-difluorocyclopent-1-yl, 2,2-difluorocyclopent-1-yl, 3,4-difluorocyclopent-1-yl, 3,3-difluorocyclopent-1-yl, and 3,3,4,4-tetrafluorocyclobut-1-yl. In some embodiments, any one or more of the foregoing alkyl groups or fluorinated alkyl groups are excluded from one or more groups in any formula disclosed in this application.

In another embodiment, the hydrocarbon group (R) is a linear, branched, or cyclic alkenyl or alkynyl group. Some examples of linear alkenyl groups containing 2-5 carbon atoms include vinyl, propen-1-yl (allyl), 3-buten-1-yl (CH₂=CH-CH₂-CH₂-), 2-buten-1-yl (CH₂-CH=CH-CH₂-), butadienyl, 4-penten-1-yl, 3-penten-1-yl, 2-penten-1-yl, and 2,4-pentadien-1-yl. Some examples of branched alkenyl groups include propen-2-yl (CH₂=C.-CH₃), 1-buten-2-yl (CH₂=C.-CH₂-CH₃), 1-buten-3-yl (CH₂=CH-CH.-CH₃), 1-propen-2-methyl-3-yl (CH₂=C(CH₃)-CH₂-), 1-penten-4-yl, 1-penten-3-yl, 1-penten-2-yl, 2-penten-2-yl, 2-penten-3-yl, 2-penten-4-yl, and 1,4-pentadien-3-yl, and partially or fully fluorinated versions thereof (e.g., 2-fluorovinyl, 2,2-difluorovinyl, 1,2-difluorovinyl, and 1,2,2-trifluorovinyl), wherein the dot in any of the foregoing groups indicates a point of attachment. Some examples of cyclic alkenyl groups containing 3-5 carbon atoms include cyclopropenyl, cyclobutenyl, cyclopentenyl, and cyclopentadienyl. Some examples of alkynyl groups containing 2-5 carbon atoms include ethynyl, propargyl (2-propynyl), and 3-butynyl, and partially or fully fluorinated versions thereof. The hydrocarbon group may also contain a cyclic portion attached to an acyclic portion, e.g., methylene group attached to a cyclopropyl or cyclobutyl group.

In a first aspect, the present disclosure is directed to compounds of the following structure:

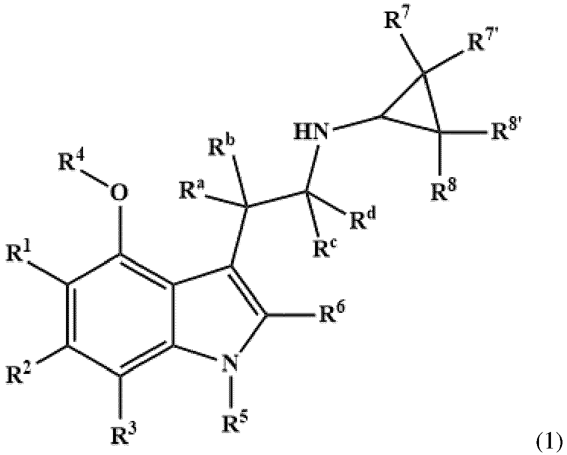

In Formula (1), R¹, R², R³, and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms. Pharmaceutically acceptable salts thereof are also within the scope of Formula (1) and all sub-formulas disclosed in this application. In a first set of embodiments, any one, two, three (or all) of R¹, R², R³, and R⁶ are hydrogen atoms. In a second set of embodiments, any one or more of R¹, R², R³, and R⁶ is independently selected from any of the hydrocarbon groups (R) described anywhere above and below, including any of the linear, branched, or cyclic alkyl or alkenyl groups described above having 1-5 carbon atoms or sub-range therein (e.g., 1-4, 1-3, 1-2, 2-5, 2-4, 2-3, 3-5, or 3-4 carbon atoms). In a third set of embodiments, any one or more of R¹, R², R³, and R⁶ is independently selected from an alkoxy group (OR), wherein R is selected from any of the hydrocarbon groups (R) described anywhere above and below, including any of the linear, branched, or cyclic alkyl or alkenyl groups described above having 1-5 carbon atoms or sub-range therein (e.g., 1-4, 1-3, 1-2, 2-5, 2-4, 2-3, 3-5, or 3-4 carbon atoms). In a fourth set of embodiments, any one or more of R¹, R², R³, and R⁶ is independently selected from a thioalkoxy group (SR), wherein R is selected from any of the hydrocarbon groups (R) described anywhere above and below, including any of the linear, branched, or cyclic alkyl or alkenyl groups described above having 1-5 carbon atoms or sub-range therein (e.g., 1-4, 1-3, 1-2, 2-5, 2-4, 2-3, 3-5, or 3-4 carbon atoms). In a fifth set of embodiments, any one or more of R¹, R², R³, and R⁶ is a nitrile (CN) group. In a sixth set of embodiments, any one or more of R¹, R², R³, and R⁶ is an amino (NH₂) group. In a seventh set of embodiments, any one or more of R¹, R², R³, and R⁶ is a hydroxy (OH) group. In an eighth set of embodiments, any one or more of R¹, R², R³, and R⁶ is a halogen atom (e.g., F, Cl, Br, or I atom). Any two or three of the foregoing first through eighth embodiments may also be combined, thereby requiring R¹, R², R³, and R⁶ to be selected from two or three different groups described above. In other embodiments, any one or more of the groups (i)-(viii) provided above may be independently excluded for R¹, R², R³, and/or R⁶.

In a first particular embodiment, R¹ in Formula (1) is independently selected from H, methyl, ethyl, n-propyl, isopropyl, cyclopropyl, methoxy, ethoxy, F, and OH, or a sub-grouping of these. In a second particular embodiment, R² is independently selected from H, methyl, ethyl, n-propyl, isopropyl, cyclopropyl, methoxy, ethoxy, F, and OH, or a sub-grouping of these. In a third particular embodiment, R³ is independently selected from H, methyl, ethyl, n-propyl, isopropyl, cyclopropyl, methoxy, ethoxy, F, and OH, or a sub-grouping of these. In a fourth particular embodiment, R⁶ is independently selected from H, methyl, ethyl, n-propyl, isopropyl, cyclopropyl, methoxy, ethoxy, F, and OH, or a sub-grouping of these. Any one of the first through fourth particular embodiments provided above may also be combined.

The group R⁴ in Formula (1) is selected from H and esterifying groups that result in OR⁴ being an ester group. When R⁴ is H, OR⁴ is a hydroxy (OH) group. When R⁴ is an esterifying group, OR⁴ is an ester group. The esterifying group (R⁴) may result in OR⁴ being any type of ester group, including, for example, a carboxy ester, phosphoester, or sulfonic ester. For example, R⁴ may be -C(O)R to result in OR⁴ being -OC(O)R (carboxy ester); or R⁴ may be -P(O)(OR')₂, wherein R' is independently selected from H and R, to result in OR⁴ being -OP(O)(OR')₂ (phospho ester); or R⁴ may be -SO₂R to result in OR⁴ being -OSO₂R (sulfonic ester). Moreover, any one of the foregoing embodiments for R⁴ may be combined with any one or more embodiments provided earlier above for R¹, R², R³, and R⁶.

The group R⁵ in Formula (1) is selected from H and hydrocarbon groups containing 1-5 carbon atoms (R). In one set of embodiments, R⁵ is H. In another set of embodiments, R⁵ is a hydrocarbon group (R) containing 1-5 carbon atoms, such as any of the R groups described in detail anywhere above and below, including any of the linear, branched, or cyclic alkyl or alkenyl groups described above having 1-5 carbon atoms or sub-range therein (e.g., 1-4, 1-3, 1-2, 2-5, 2-4, 2-3, 3-5, or 3-4 carbon atoms). In some embodiments, R⁵ is selected from one or more of methyl, ethyl, n-propyl, isopropyl, n-butyl, sec-butyl, isobutyl, t-butyl, cyclopropyl, and cyclobutyl. In some embodiments, any one or more of the foregoing R groups is excluded for R⁵. Moreover, any one of the foregoing embodiments for R⁵ may be combined with any one or more embodiments provided earlier above for R¹, R², R³, R⁴, and R⁶.

The groups R⁷, R^{7'}, R⁸, and R^{8'} in Formula (1) are independently selected from H and F atoms. In one embodiment, R⁷, R^{7'}, R⁸, and R^{8'} are H atoms. In other embodiments, R⁷ or R⁸ is an F atom, while R^{7'} and R^{8'} are H atoms. In another embodiment, R⁷ and R⁸ are F atoms, while R^{7'} and R^{8'} are H atoms. In other embodiments, R⁷ and R^{7'} are F atoms, while R⁸ and R^{8'} are H atoms (or R⁸ and R^{8'} are F atoms, while R⁷ and R^{7'} are H atoms). In another embodiment, R⁷, R^{7'}, R⁸, and R^{8'} are F atoms. Moreover, any of the foregoing embodiments for R⁷, R^{7'}, R⁸, and R^{8'} may be combined with any of the embodiments provided for R¹, R², R³, and R⁶, any of the embodiments provided for R⁴, and any of the embodiments provided for R⁵ as provided above.

The groups R^{a}, R^{b}, R^{c}, and R^{d} in Formula (1) are independently selected from H and D atoms. In one embodiment, R^{a}, R^{b}, R^{c}, and R^{d} are all H atoms. In another embodiment, precisely or at least one or two of R^{a}, R^{b}, R^{c}, and R^{d} are D atoms, the rest (if any) being H atoms. In another embodiment, R^{a}, R^{b}, R^{c}, and R^{d} are all D atoms.

In some embodiments, the compound of Formula (1) has the following structure: 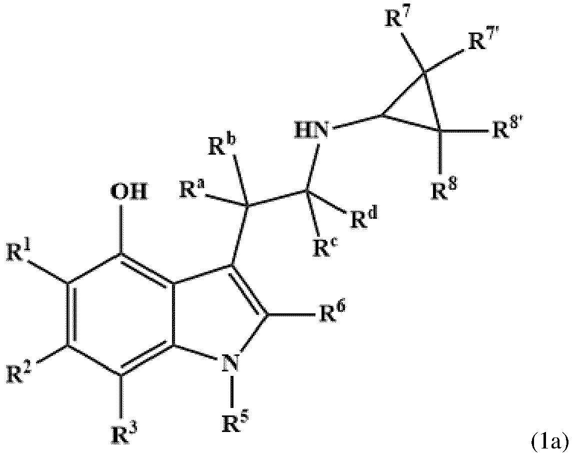 wherein R¹, R², R³, R⁵, R⁶, R⁷, R⁸, R^{7'}, R^{8'}, R^{a}, R^{b}, R^{c}, and R^{d} are independently as defined under Formula (1) and any sub-embodiments thereof and combinations thereof, as provided above.

In some embodiments, the compound of Formula (1) has the following structure: 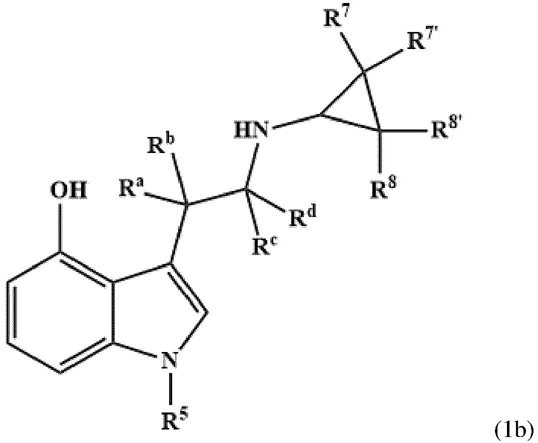 wherein R⁵, R⁷, R⁸, R^{7'}, R^{8'}, R^{a}, R^{b}, R^{c}, and R^{d} are independently as defined under Formula (1) and any sub-embodiments thereof and combinations thereof, as provided above.

In some embodiments, the compound of Formula (1) has the following structure: 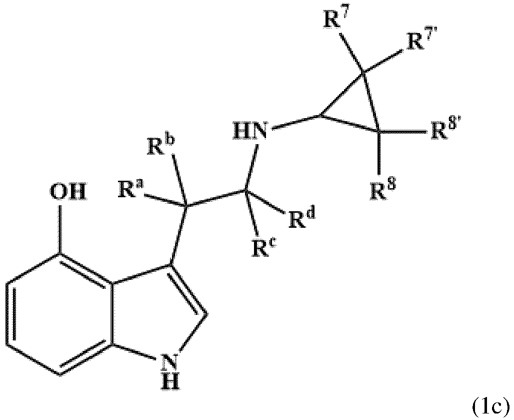 wherein R⁷, R⁸, R^{7'}, R^{8'}, R^{a}, R^{b}, R^{c}, and R^{d} are independently as defined under Formula (1) and any sub-embodiments thereof and combinations thereof, as provided above.

In some embodiments, the compound of Formula (1) has the following structure: 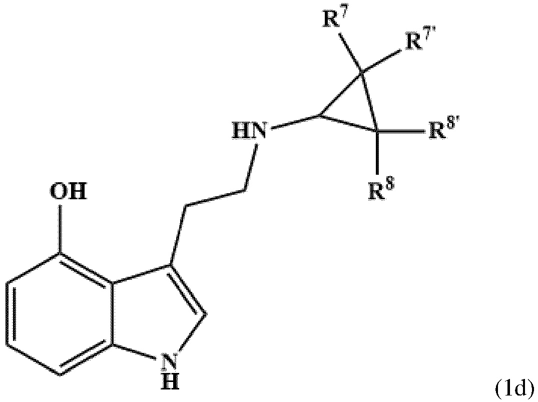 wherein R⁷, R⁸, R^{7'}, and R^{8'} are independently as defined under Formula (1) and any sub-embodiments thereof and combinations thereof, as provided above.

In some embodiments, the compound of Formula (1) has the following structure: 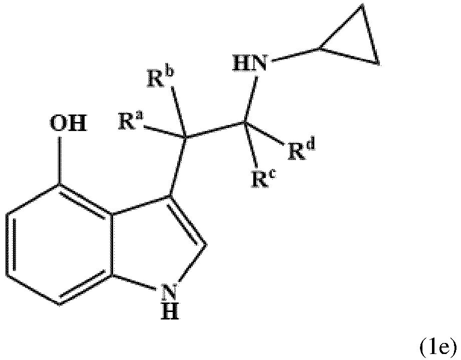 wherein R^{a}, R^{b}, R^{c}, and R^{d} are independently as defined under Formula (1) and any sub-embodiments thereof and combinations thereof, as provided above.

In some embodiments, the compound of Formula (1e) has the following specific structure:

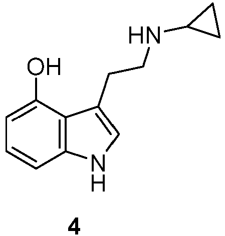

In other embodiments, the compound of Formula (1e) has the following specific structure: 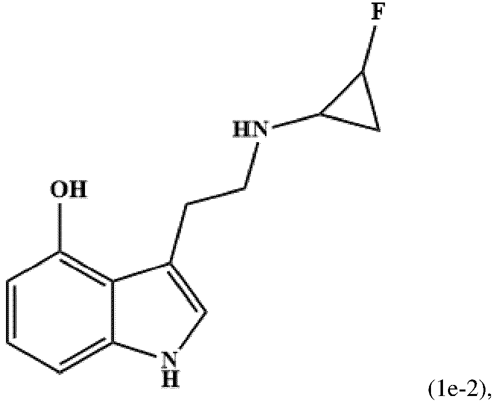 which may be racemic or enantiomerically (or diastereomerically) enriched or pure.

In some embodiments, the compound of Formula (1e-2) may be or include any of the following specific diastereomers: 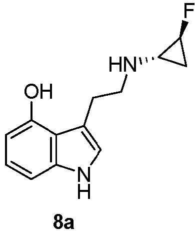 and

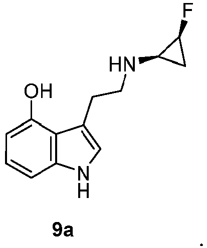

Notably, although a specific trans and cis diastereomer is shown above, the present disclosure also considers the corresponding enantiomer of each diastereomer, as follows: 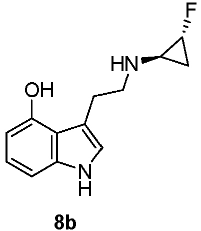 and

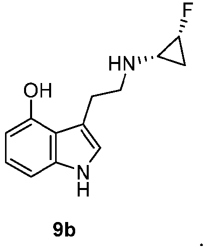

Any enantiomerically enriched or isolated form of compound 8a, 8b, 9a, or 9b is considered herein, as well as mixtures of any of these, e.g., a racemic mixture of compounds 8a and 8b or a racemic mixture of compounds 9a and 9b.

In a second aspect, the present disclosure is directed to compounds of the following structure:

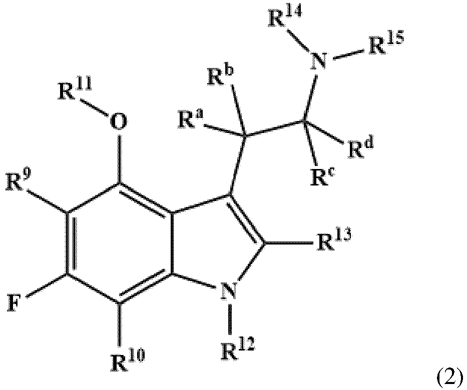

In Formula (2), R⁹, R¹⁰, and R¹³ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms. Pharmaceutically acceptable salts thereof are also within the scope of Formula (2) and all sub-formulas disclosed in this application. In a first set of embodiments, any one, two, three (or all) of R⁹, R¹⁰, and R¹³ are hydrogen atoms. In a second set of embodiments, any one or more of R⁹, R¹⁰, and R¹³ is independently selected from any of the hydrocarbon groups (R) described anywhere above and below, including any of the linear, branched, or cyclic alkyl or alkenyl groups described above having 1-5 carbon atoms or sub-range therein (e.g., 1-4, 1-3, 1-2, 2-5, 2-4, 2-3, 3-5, or 3-4 carbon atoms). In a third set of embodiments, any one or more of R⁹, R¹⁰, and R¹³ is independently selected from an alkoxy group (OR), wherein R is selected from any of the hydrocarbon groups (R) described anywhere above and below, including any of the linear, branched, or cyclic alkyl or alkenyl groups described above having 1-5 carbon atoms or sub-range therein (e.g., 1-4, 1-3, 1-2, 2-5, 2-4, 2-3, 3-5, or 3-4 carbon atoms). In a fourth set of embodiments, any one or more of R⁹, R¹⁰, and R¹³ is independently selected from a thioalkoxy group (SR), wherein R is selected from any of the hydrocarbon groups (R) described anywhere above and below, including any of the linear, branched, or cyclic alkyl or alkenyl groups described above having 1-5 carbon atoms or sub-range therein (e.g., 1-4, 1-3, 1-2, 2-5, 2-4, 2-3, 3-5, or 3-4 carbon atoms). In a fifth set of embodiments, any one or more of R⁹, R¹⁰, and R¹³ is a nitrile (CN) group. In a sixth set of embodiments, any one or more of R⁹, R¹⁰, and R¹³ is an amino (NH₂) group. In a seventh set of embodiments, any one or more of R⁹, R¹⁰, and R¹³ is a hydroxy (OH) group. In an eighth set of embodiments, any one or more of R⁹, R¹⁰, and R¹³ is a halogen atom (e.g., F, Cl, Br, or I atom). Any two or three of the foregoing first through eighth embodiments may also be combined, thereby requiring R⁹, R¹⁰, and R¹³ to be selected from two or three different groups described above. In other embodiments, any one or more of the groups (i)-(viii) provided above may be independently excluded for R⁹, R¹⁰, and R¹³.

In a first particular embodiment of Formula (2), R⁹ is independently selected from H, methyl, ethyl, n-propyl, isopropyl, cyclopropyl, methoxy, ethoxy, F, and OH, or a sub-grouping of these. In a second particular embodiment, R¹⁰ is independently selected from H, methyl, ethyl, n-propyl, isopropyl, cyclopropyl, methoxy, ethoxy, F, and OH, or a sub-grouping of these. In a third particular embodiment, R¹³ is independently selected from H, methyl, ethyl, n-propyl, isopropyl, cyclopropyl, methoxy, ethoxy, F, and OH, or a sub-grouping of these. Any one of the first through third particular embodiments provided above may also be combined.

The group R¹¹ in Formula (2) is selected from H and esterifying groups that result in OR¹¹ being an ester group. When R¹¹ is H, OR¹¹ is a hydroxy (OH) group. When R¹¹ is an esterifying group, OR¹¹ is an ester group. The esterifying group (R¹¹) may result in OR¹¹ being any type of ester group, including, for example, a carboxy ester, phosphoester, or sulfonic ester. For example, R¹¹ may be -C(O)R to result in OR¹¹ being -OC(O)R (carboxy ester); or R¹¹ may be -P(O)(OR')₂, wherein R' is independently selected from H and R, to result in OR¹¹ being -OP(O)(OR')₂ (phospho ester); or R¹¹ may be -SO₂R to result in OR¹¹ being -OSO₂R (sulfonic ester). Moreover, any one of the foregoing embodiments for R¹¹ may be combined with any one or more embodiments provided earlier above for R⁹, R¹⁰, and R¹³.

The group R¹² in Formula (2) is isopropyl or cyclopropyl with optional substitution with one or more fluorine atoms. In one set of embodiments, R¹² is not substituted with fluorine atoms. In another set of embodiments, R¹² is substituted with one, two, or more fluorine atoms, in which case R¹² may be partially or completely fluorinated. Moreover, any one of the foregoing embodiments for R¹² may be combined with any one or more embodiments provided earlier above for R⁹, R¹⁰, R¹¹, and R¹³.

The groups R¹⁴ and R¹⁵ in Formula (2) are independently selected from H and hydrocarbon groups containing 1-5 carbon atoms (R), which are optionally substituted with one or more fluorine atoms. In one set of embodiments, R¹⁴ and R¹⁵ are both H. In another set of embodiments, one of R¹⁴ and R¹⁵ is selected from among any of the hydrocarbon groups (R) described anywhere above and below, including any of the linear, branched, or cyclic alkyl or alkenyl groups described above having 1-5 carbon atoms or sub-range therein (e.g., 1-4, 1-3, 1-2, 2-5, 2-4, 2-3, 3-5, or 3-4 carbon atoms). In another set of embodiments, R¹⁴ and R¹⁵ are both independently selected from among any of the hydrocarbon groups (R) described anywhere above and below, including any of the linear, branched, or cyclic alkyl or alkenyl groups described above having 1-5 carbon atoms or sub-range therein (e.g., 1-4, 1-3, 1-2, 2-5, 2-4, 2-3, 3-5, or 3-4 carbon atoms). In a first particular embodiment, R¹⁴ or R¹⁵ is independently selected from H, methyl, ethyl, n-propyl, isopropyl, cyclopropyl, methoxy, ethoxy, F, and OH, or a sub-grouping of these. In a second particular embodiment, R¹⁴ and R¹⁵ are independently selected from H, methyl, ethyl, n-propyl, isopropyl, cyclopropyl, methoxy, ethoxy, F, and OH, or a sub-grouping of these. Moreover, any one of the foregoing embodiments for R¹⁴ and R¹⁵ may be combined with any one or more embodiments provided earlier above for R⁹, R¹⁰, R¹¹, R¹², and R¹³.

The groups R^{a}, R^{b}, R^{c}, and R^{d} in Formula (2) are independently selected from H and D atoms. In one embodiment, R^{a}, R^{b}, R^{c}, and R^{d} are all H atoms. In another embodiment, precisely or at least one or two of R^{a}, R^{b}, R^{c}, and R^{d} are D atoms, the rest (if any) being H atoms. In another embodiment, R^{a}, R^{b}, R^{c}, and R^{d} are all D atoms.

In some embodiments, when R⁹, R¹⁰, R¹¹, R¹³, R^{a}, R^{b}, R^{c}, and R^{d} are H and R¹² is isopropyl, then R¹⁴ and R¹⁵ are not both methyl. In other embodiments, R¹⁴ and R¹⁵ may both be methyl only when R⁹, R¹⁰, R¹¹, R¹³, R^{a}, R^{b}, R^{c}, and R^{d} are not all H and/or when R¹² is not isopropyl (or when R¹² is cyclopropyl).

In some embodiments, the following compound is excluded from the scope of Formula (2):

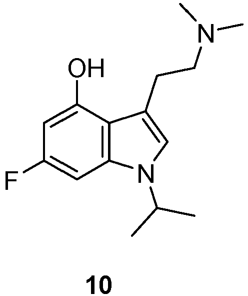

In some embodiments, the compound of Formula (2) has the following structure: 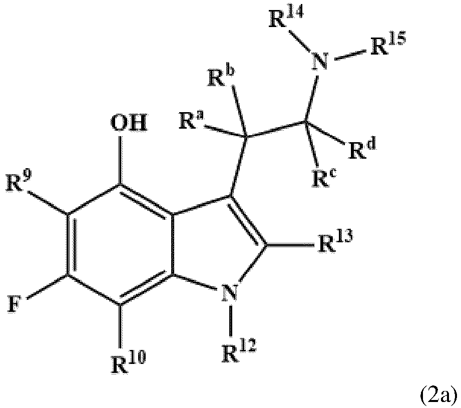 wherein R⁹, R¹⁰, R¹², R¹³, R¹⁴, R¹⁵, R^{a}, R^{b}, R^{c}, and R^{d} are independently as defined under Formula (2) and any sub-embodiments thereof and combinations thereof, as provided above.

In some embodiments, the compound of Formula (2) has the following structure: 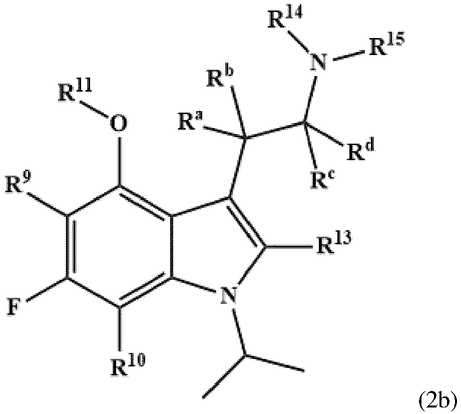 wherein R⁹, R¹⁰, R¹¹, R¹³, R¹⁴, R¹⁵, R^{a}, R^{b}, R^{c}, and R^{d} are independently as defined under Formula (2) and any sub-embodiments thereof and combinations thereof, as provided above.

In some embodiments, the compound of Formula (2b) has the following structure: 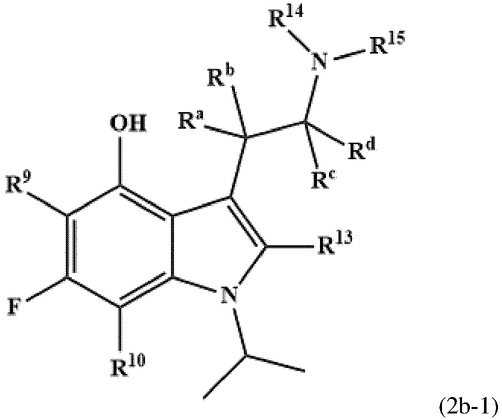 wherein R⁹, R¹⁰, R¹³, R¹⁴, R¹⁵, R^{a}, R^{b}, R^{c}, and R^{d} are independently as defined under Formula (2) and any sub-embodiments thereof and combinations thereof, as provided above.

In some embodiments, the compound of Formula (2) has the following structure: 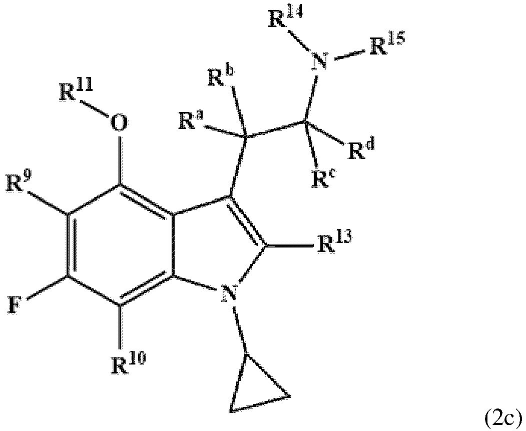 wherein R⁹, R¹⁰, R¹¹, R¹³, R¹⁴, R¹⁵, R^{a}, R^{b}, R^{c}, and R^{d} are independently as defined under Formula (2) and any sub-embodiments thereof and combinations thereof, as provided above.

In some embodiments, the compound of Formula (2c) has the following structure: 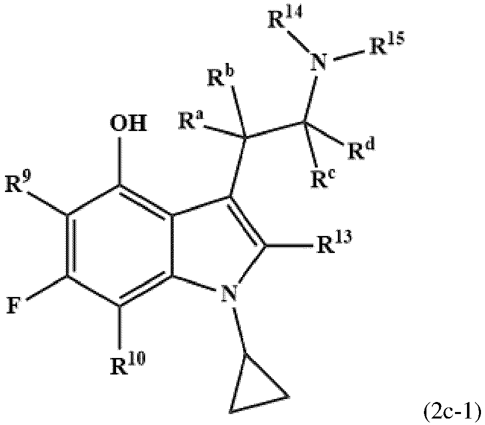 wherein R⁹, R¹⁰, R¹³, R¹⁴, R¹⁵, R^{a}, R^{b}, R^{c}, and R^{d} are independently as defined under Formula (2) and any sub-embodiments thereof and combinations thereof, as provided above.

In some embodiments, the compound of Formula (2) is selected from any of the following specific compounds:

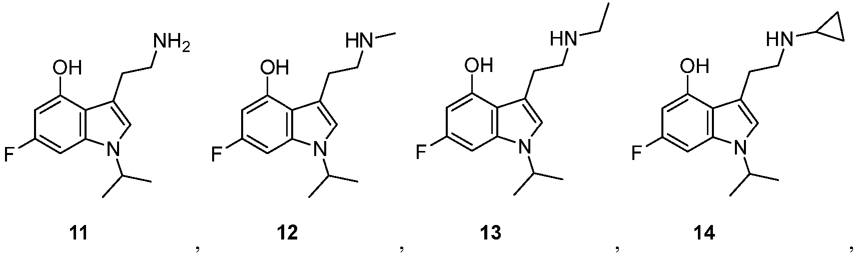

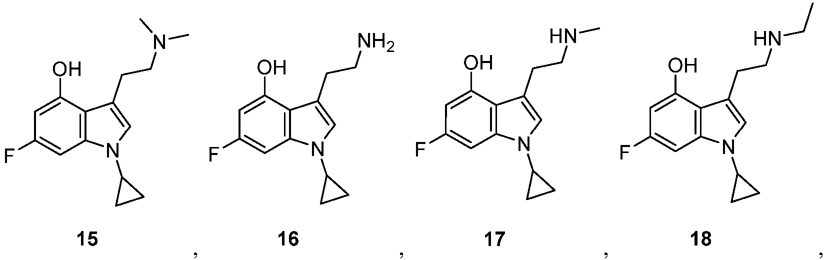

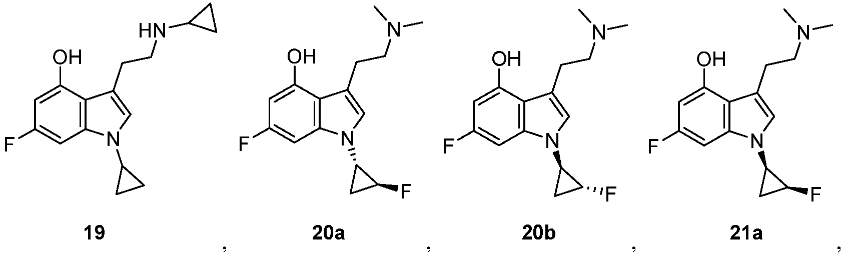

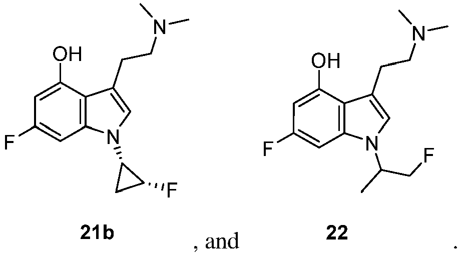

Any enantiomerically or diastereomerically enriched or isolated form of any of the above compounds, such as compounds 20a, 20b, 21a, or 21b, is considered herein, as well as mixtures of any of these, e.g., a racemic mixture of compounds 20a and 20b or a racemic mixture of compounds 21a and 21b.

In a third aspect, the present disclosure is directed to compounds of the following structure:

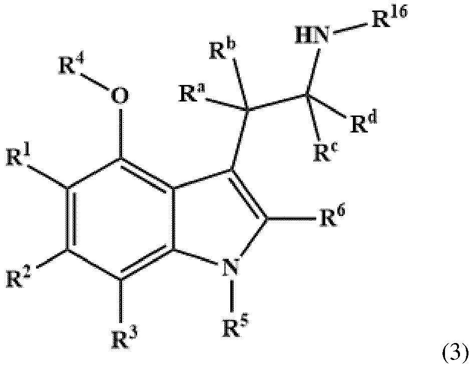

In Formula (3), R¹, R², R³, and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms. Pharmaceutically acceptable salts thereof are also within the scope of Formula (3) and all sub-formulas disclosed in this application. In a first set of embodiments, any one, two, three (or all) of R¹, R², R³, and R⁶ are hydrogen atoms. In a second set of embodiments, any one or more of R¹, R², R³, and R⁶ is independently selected from any of the hydrocarbon groups (R) described anywhere above and below, including any of the linear, branched, or cyclic alkyl or alkenyl groups described above having 1-5 carbon atoms or sub-range therein (e.g., 1-4, 1-3, 1-2, 2-5, 2-4, 2-3, 3-5, or 3-4 carbon atoms). In a third set of embodiments, any one or more of R¹, R², R³, and R⁶ is independently selected from an alkoxy group (OR), wherein R is selected from any of the hydrocarbon groups (R) described anywhere above and below, including any of the linear, branched, or cyclic alkyl or alkenyl groups described above having 1-5 carbon atoms or sub-range therein (e.g., 1-4, 1-3, 1-2, 2-5, 2-4, 2-3, 3-5, or 3-4 carbon atoms). In a fourth set of embodiments, any one or more of R¹, R², R³, and R⁶ is independently selected from a thioalkoxy group (SR), wherein R is selected from any of the hydrocarbon groups (R) described anywhere above and below, including any of the linear, branched, or cyclic alkyl or alkenyl groups described above having 1-5 carbon atoms or sub-range therein (e.g., 1-4, 1-3, 1-2, 2-5, 2-4, 2-3, 3-5, or 3-4 carbon atoms). In a fifth set of embodiments, any one or more of R¹, R², R³, and R⁶ is a nitrile (CN) group. In a sixth set of embodiments, any one or more of R¹, R², R³, and R⁶ is an amino (NH₂) group. In a seventh set of embodiments, any one or more of R¹, R², R³, and R⁶ is a hydroxy (OH) group. In an eighth set of embodiments, any one or more of R¹, R², R³, and R⁶ is a halogen atom (e.g., F, Cl, Br, or I atom). Any two or three of the foregoing first through eighth embodiments may also be combined, thereby requiring R¹, R², R³, and R⁶ to be selected from two or three different groups described above. In other embodiments, any one or more of the groups (i)-(viii) provided above may be independently excluded for R¹, R², R³, and/or R⁶.

In a first particular embodiment of Formula (3), R¹ is independently selected from H, methyl, ethyl, n-propyl, isopropyl, cyclopropyl, methoxy, ethoxy, F, and OH, or a sub-grouping of these. In a second particular embodiment, R² is independently selected from H, methyl, ethyl, n-propyl, isopropyl, cyclopropyl, methoxy, ethoxy, F, and OH, or a sub-grouping of these. In a third particular embodiment, R³ is independently selected from H, methyl, ethyl, n-propyl, isopropyl, cyclopropyl, methoxy, ethoxy, F, and OH, or a sub-grouping of these. In a fourth particular embodiment, R⁶ is independently selected from H, methyl, ethyl, n-propyl, isopropyl, cyclopropyl, methoxy, ethoxy, F, and OH, or a sub-grouping of these. Any one of the first through fourth particular embodiments provided above may also be combined.

The group R⁴ in Formula (3) is selected from H and esterifying groups that result in OR⁴ being an ester group. When R⁴ is H, OR¹¹ is a hydroxy (OH) group. When R⁴ is an esterifying group, OR⁴ is an ester group. The esterifying group (R⁴) may result in OR⁴ being any type of ester group, including, for example, a carboxy ester, phosphoester, or sulfonic ester. For example, R⁴ may be -C(O)R to result in OR⁴ being -OC(O)R (carboxy ester); or R⁴ may be -P(O)(OR')₂, wherein R' is independently selected from H and R, to result in OR⁴ being -OP(O)(OR')₂ (phospho ester); or R⁴ may be -SO₂R to result in OR⁴ being -OSO₂R (sulfonic ester). Moreover, any one of the foregoing embodiments for R⁴ may be combined with any one or more embodiments provided earlier above for R¹, R², R³, and R⁶.

The group R⁵ in Formula (3) is selected from H and hydrocarbon groups containing 1-5 carbon atoms (R). In one set of embodiments, R⁵ is H. In another set of embodiments, R⁵ is a hydrocarbon group (R) containing 1-5 carbon atoms, such as any of the R groups described in detail anywhere above and below, including any of the linear, branched, or cyclic alkyl or alkenyl groups described above having 1-5 carbon atoms or sub-range therein (e.g., 1-4, 1-3, 1-2, 2-5, 2-4, 2-3, 3-5, or 3-4 carbon atoms). In some embodiments, R⁵ is selected from one or more of methyl, ethyl, n-propyl, isopropyl, n-butyl, sec-butyl, isobutyl, t-butyl, cyclopropyl, and cyclobutyl. In some embodiments, any one or more of the foregoing R groups is excluded for R⁵. Moreover, any one of the foregoing embodiments for R⁵ may be combined with any one or more embodiments provided earlier above for R¹, R², R³, R⁴, and R⁶.

The group R¹⁶ in Formula (3) is an allyl or propargyl group. The allyl group has the structure -CH₂-CH=CH₂. The propargyl group has the structure -CH₂-C≡CH. Moreover, any one of the foregoing embodiments for R¹⁶ may be combined with any one or more embodiments provided earlier above for R¹, R², R³, R⁴, R⁵, and R⁶.

The groups R^{a}, R^{b}, R^{c}, and R^{d} in Formula (3) are independently selected from H and D atoms. In one embodiment, R^{a}, R^{b}, R^{c}, and R^{d} are all H atoms. In another embodiment, precisely or at least one or two of R^{a}, R^{b}, R^{c}, and R^{d} are D atoms, the rest (if any) being H atoms. In another embodiment, R^{a}, R^{b}, R^{c}, and R^{d} are all D atoms.

In some embodiments, the compound of Formula (3) has the following structure: 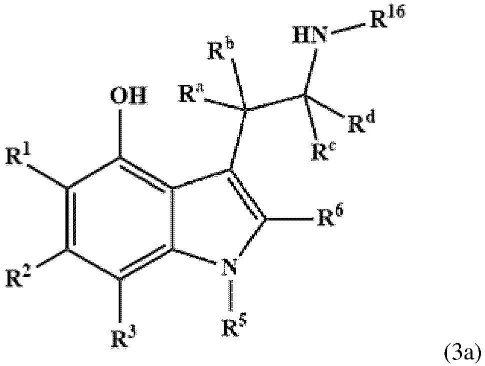 wherein R¹, R², R³, R⁵, R⁶, R¹⁶, R^{a}, R^{b}, R^{c}, and R^{d} are independently as defined under Formula (3) and any sub-embodiments thereof and combinations thereof, as provided above.

In some embodiments, the compound of Formula (3) has the following structure: 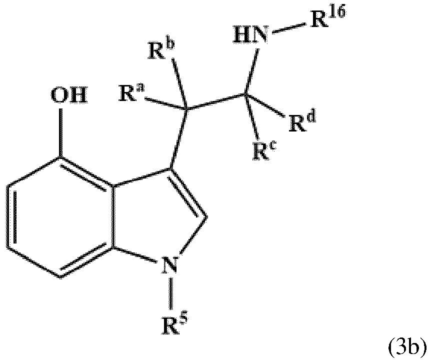 wherein R⁵, R¹⁶, R^{a}, R^{b}, R^{c}, and R^{d} are independently as defined under Formula (3) and any sub-embodiments thereof and combinations thereof, as provided above.

In some embodiments, the compound of Formula (3) has the following structure: 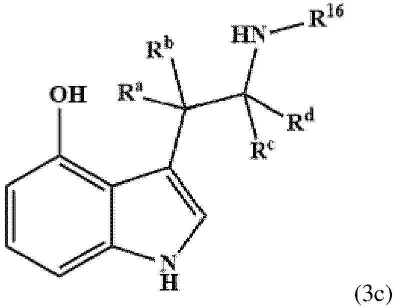 wherein R¹⁶, R^{a}, R^{b}, R^{c}, and R^{d} are independently as defined under Formula (3) and any sub-embodiments thereof and combinations thereof, as provided above.

In some embodiments, the compound of Formula (3) has the following structure: 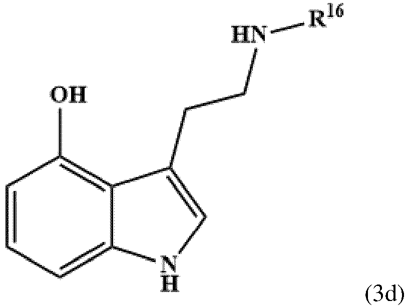 wherein R¹⁶ is as defined under Formula (3) and any sub-embodiments thereof and combinations thereof, as provided above.

In some embodiments, the compound of Formula (3) has the following structure: 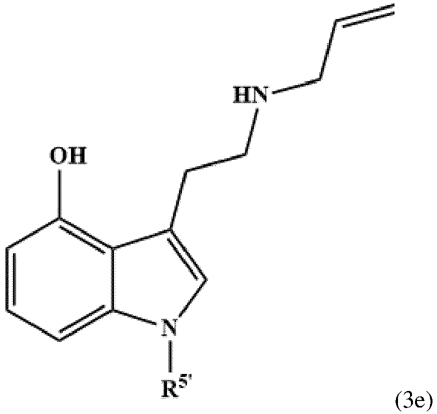 wherein R^{5'} is selected from hydrocarbon groups (R) containing 1, 2, 3, 4, or 5 carbon atoms.

In some embodiments, the compound of Formula (3d) has the following specific structure:

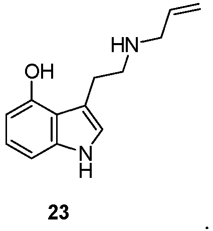

In some embodiments, Formula (3) excludes the above compound 23.

In other embodiments, the compound of Formula (3d) has the following specific structure:

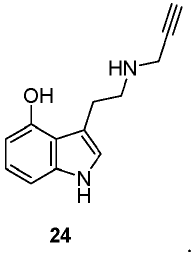

In some embodiments of Formula (3), when R⁴ is OH, and R¹, R², R³, R⁵, R⁶, R^{a}, R^{b}, R^{c}, and R^{d} are all H, then R¹⁶ is not allyl.

In a fourth aspect, the present disclosure is directed to compounds of the following structure:

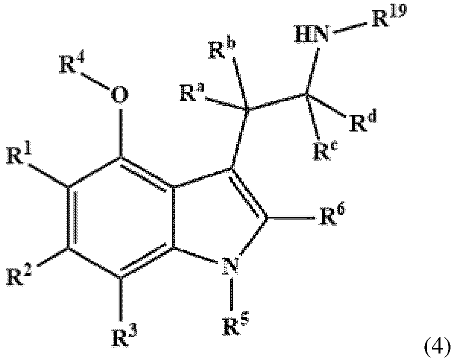

In Formula (4), R¹ is selected from the group consisting of: F, Cl, and methyl; R², R³, and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms; R⁴ is selected from the group consisting of H and esterifying groups that result in OR⁴ being an ester group; R⁵ is selected from the group consisting of H and hydrocarbon groups containing 1-5 carbon atoms (R); R^{a}, R^{b}, R^{c}, and R^{d} are independently selected from H and D atoms; and R¹⁹ is selected from the group consisting of H and hydrocarbon groups containing 1-5 carbon atoms (R), which are optionally substituted with one or more fluorine atoms. Pharmaceutically acceptable salts thereof are also within the scope of Formula (4) and all sub-formulas disclosed in this application.

In a first set of embodiments, any one, two, or all of R², R³, and R⁶ are hydrogen atoms. In a second set of embodiments, any one or more of R², R³, and R⁶ is independently selected from any of the hydrocarbon groups (R) described anywhere above and below, including any of the linear, branched, or cyclic alkyl or alkenyl groups described above having 1-5 carbon atoms or sub-ranges therein (e.g., 1-4, 1-3, 1-2, 2-5, 2-4, 2-3, 3-5, or 3-4 carbon atoms). In a third set of embodiments, any one or more of R², R³, and R⁶ is independently selected from an alkoxy group (OR), wherein R is selected from any of the hydrocarbon groups (R) described anywhere above and below, including any of the linear, branched, or cyclic alkyl or alkenyl groups described above having 1-5 carbon atoms or sub-ranges therein (e.g., 1-4, 1-3, 1-2, 2-5, 2-4, 2-3, 3-5, or 3-4 carbon atoms). In a fourth set of embodiments, any one or more of R², R³, and R⁶ is independently selected from a thioalkoxy group (SR), wherein R is selected from any of the hydrocarbon groups (R) described anywhere above and below, including any of the linear, branched, or cyclic alkyl or alkenyl groups described above having 1-5 carbon atoms or sub-ranges therein (e.g., 1-4, 1-3, 1-2, 2-5, 2-4, 2-3, 3-5, or 3-4 carbon atoms). In a fifth set of embodiments, any one or more of R², R³, and R⁶ is a nitrile (CN) group. In a sixth set of embodiments, any one or more of R², R³, and R⁶ is an amino (NH₂) group. In a seventh set of embodiments, any one or more of R², R³, and R⁶ is a hydroxy (OH) group. In an eighth set of embodiments, any one or more of R², R³, and R⁶ is a halogen atom (e.g., F, Cl, Br, or I atom). Any two or three of the foregoing first through eighth embodiments may also be combined, thereby requiring R², R³, and R⁶ to be selected from two or three different groups described above. In other embodiments, any one or more of the groups (i)-(viii) provided above may be independently excluded for R², R³, and/or R⁶.

In some embodiments, for any of the first through eighth embodiments or combinations thereof described above, R¹ is F. In other embodiments, for any of the first through eighth embodiments or combinations thereof described above, R¹ is Cl. In other embodiments, for any of the first through eighth embodiments or combinations thereof described above, R¹ is methyl.

In a first particular embodiment of Formula (4), R² is independently selected from H, methyl, ethyl, n-propyl, isopropyl, cyclopropyl, methoxy, ethoxy, F, and OH, or a sub-grouping of these. In a second particular embodiment, R³ is independently selected from H, methyl, ethyl, n-propyl, isopropyl, cyclopropyl, methoxy, ethoxy, F, and OH, or a sub-grouping of these. In a third particular embodiment, R⁶ is independently selected from H, methyl, ethyl, n-propyl, isopropyl, cyclopropyl, methoxy, ethoxy, F, and OH, or a sub-grouping of these. Any one of the first through third particular embodiments provided above may also be combined with each other and with embodiments of R¹ described above.

The group R⁴ in Formula (4) is selected from H and esterifying groups that result in OR⁴ being an ester group. When R⁴ is H, OR⁴ is a hydroxy (OH) group. When R⁴ is an esterifying group, OR⁴ is an ester group. The esterifying group (R⁴) may result in OR⁴ being any type of ester group, including, for example, a carboxy ester, phosphoester, or sulfonic ester. For example, R⁴ may be -C(O)R to result in OR⁴ being -OC(O)R (carboxy ester); or R⁴ may be -P(O)(OR')₂, wherein R' is independently selected from H and R, to result in OR⁴ being -OP(O)(OR')₂ (phospho ester); or R⁴ may be -SO₂R to result in OR⁴ being -OSO₂R (sulfonic ester). In some embodiments, any one or more of the foregoing groups is excluded for R⁴. Moreover, any one of the foregoing embodiments for R⁴ may be combined with any one or more embodiments provided earlier above for R¹, R², R³, and R⁶.

The group R⁵ in Formula (4) is selected from H and hydrocarbon groups containing 1-5 carbon atoms (R). In one set of embodiments, R⁵ is H. In another set of embodiments, R⁵ is a hydrocarbon group (R) containing 1-5 carbon atoms, such as any of the R groups described in detail anywhere above and below, including any of the linear, branched, or cyclic alkyl or alkenyl groups described above having 1-5 carbon atoms or sub-ranges therein (e.g., 1-4, 1-3, 1-2, 2-5, 2-4, 2-3, 3-5, or 3-4 carbon atoms). In some embodiments, R⁵ is selected from one or more of methyl, ethyl, n-propyl, isopropyl, n-butyl, sec-butyl, isobutyl, t-butyl, cyclopropyl, and cyclobutyl. In some embodiments, any one or more of the foregoing groups is excluded for R⁵. Moreover, any one of the foregoing embodiments for R⁵ may be combined with any one or more embodiments provided earlier above for R¹, R², R³, R⁴, and R⁶.

The group R¹⁹ in Formula (4) is selected from H and hydrocarbon groups containing 1-5 carbon atoms (R), which are optionally substituted with one or more fluorine atoms. In one set of embodiments, R¹⁹ is H. In another set of embodiments, R¹⁹ is selected from among any of the hydrocarbon groups (R) described anywhere above and below, including any of the linear, branched, or cyclic alkyl or alkenyl groups described above having 1-5 carbon atoms or sub-ranges therein (e.g., 1-4, 1-3, 1-2, 2-5, 2-4, 2-3, 3-5, or 3-4 carbon atoms). In some embodiments, R¹⁹ is selected from one or more of methyl, ethyl, n-propyl, isopropyl, n-butyl, sec-butyl, isobutyl, t-butyl, cyclopropyl, and cyclobutyl. In some embodiments, any one or more of the foregoing groups is excluded for R¹⁹. Moreover, any one of the foregoing embodiments for R¹⁹ may be combined with any one or more embodiments provided earlier above for R¹, R², R³, R⁴, R⁵, and R⁶.

The groups R^{a}, R^{b}, R^{c}, and R^{d} in Formula (4) are independently selected from H and D atoms. In one embodiment, R^{a}, R^{b}, R^{c}, and R^{d} are all H atoms. In another embodiment, precisely or at least one or two of R^{a}, R^{b}, R^{c}, and R^{d} are D atoms, the rest (if any) being H atoms. In another embodiment, R^{a}, R^{b}, R^{c}, and R^{d} are all D atoms. Moreover, any one of the foregoing embodiments for R^{a}, R^{b}, R^{c}, and R^{d} may be combined with any one or more embodiments provided earlier above for R¹, R², R³, R⁴, R⁵, R⁶, and R¹⁹.

In embodiments of Formula (4), the following first provision applies: when R¹ is F or methyl, and R², R³, R⁴, R⁶, R¹⁹, R^{a}, R^{b}, R^{c}, and R^{d} are all H, then R⁵ is not H or methyl. In embodiments of Formula (4), the following second provision applies: when R¹ is Cl, and R², R³, R⁴, R⁶, R¹⁹, R^{a}, R^{b}, R^{c}, and R^{d} are all H, then R⁵ is not H, methyl, or ethyl. In embodiments of Formula (4), the following third provision applies: when R¹ is F, and R², R³, R⁴, R⁶, R^{a}, R^{b}, R^{c}, and R^{d} are all H, then R⁵ and R¹⁹ are not both methyl. Any one, two, or all three of the foregoing first, second, and third provisions may be combined.

In some embodiments, the compound of Formula (4) has the following structure: 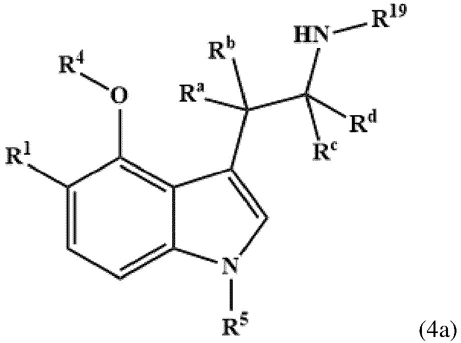 wherein R¹, R⁴, R⁵, R¹⁹, R^{a}, R^{b}, R^{c}, and R^{d} are independently as defined under Formula (4) and any sub-embodiments thereof and combinations thereof, as provided above. Formula (4a) also includes all pharmaceutical salts thereof. In some embodiments of Formula (4a), R¹ is F. In other embodiments of Formula (4a), R¹ is Cl. In other embodiments of Formula (4a), R¹ is methyl.

In some embodiments, the compound of Formula (4) has the following structure: 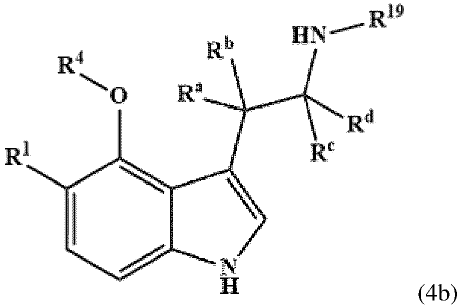 wherein R¹, R⁴, R¹⁹, R^{a}, R^{b}, R^{c}, and R^{d} are independently as defined under Formula (4) and any sub-embodiments thereof and combinations thereof, as provided above. Formula (4b) also includes all pharmaceutical salts thereof. In some embodiments of Formula (4b), R¹ is F. In other embodiments of Formula (4b), R¹ is Cl. In other embodiments of Formula (4b), R¹ is methyl.

In some embodiments, the compound of Formula (4) has the following structure: 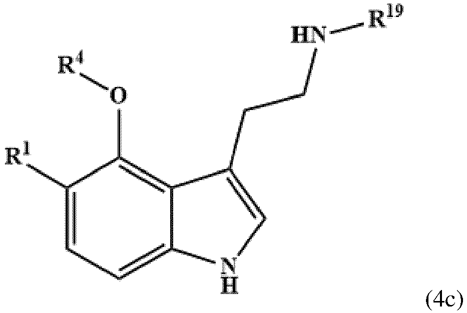 wherein R¹, R⁴, and R¹⁹, are independently as defined under Formula (4) and any sub-embodiments thereof and combinations thereof, as provided above. Formula (4c) also includes all pharmaceutical salts thereof. In some embodiments of Formula (4c), R¹ is F. In other embodiments of Formula (4c), R¹ is Cl. In other embodiments of Formula (4c), R¹ is methyl.

In some embodiments, the compound of Formula (4) has any of the following specific structures: 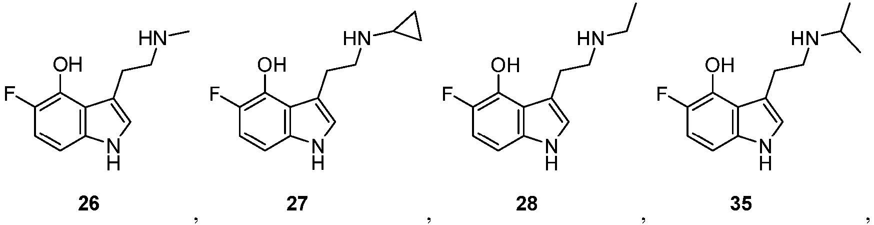 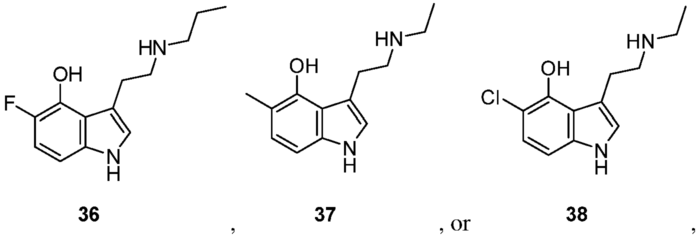 and pharmaceutically acceptable salts thereof.

In more specific embodiments, any one or more of the following compounds may be specifically excluded from Formula (4):

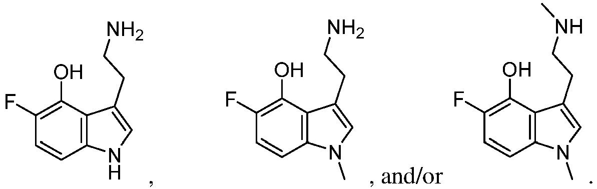

In a fifth aspect, the present disclosure is directed to compounds of the following structure:

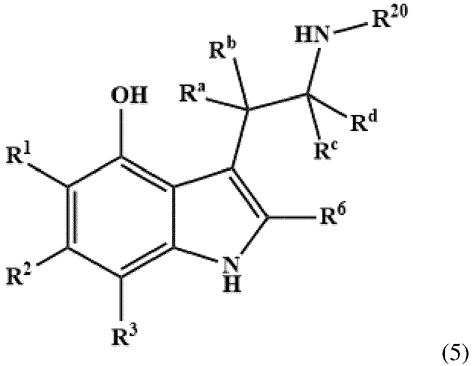

In Formula (5), R¹, R³, and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms; R² is selected from the group consisting of: F, Cl, and methyl; R^{a}, R^{b}, R^{c}, and R^{d} are independently selected from H and D atoms; and R²⁰ is selected from the group consisting of H and hydrocarbon groups containing 1-5 carbon atoms (R), which are optionally substituted with one or more fluorine atoms. Pharmaceutically acceptable salts thereof are also within the scope of Formula (5) and all sub-formulas disclosed in this application.

In a first set of embodiments, any one, two, or all of R¹, R³, and R⁶ are hydrogen atoms. In a second set of embodiments, any one or more of R¹, R³, and R⁶ is independently selected from any of the hydrocarbon groups (R) described anywhere above and below, including any of the linear, branched, or cyclic alkyl or alkenyl groups described above having 1-5 carbon atoms or sub-ranges therein (e.g., 1-4, 1-3, 1-2, 2-5, 2-4, 2-3, 3-5, or 3-4 carbon atoms). In a third set of embodiments, any one or more of R¹, R³, and R⁶ is independently selected from an alkoxy group (OR), wherein R is selected from any of the hydrocarbon groups (R) described anywhere above and below, including any of the linear, branched, or cyclic alkyl or alkenyl groups described above having 1-5 carbon atoms or sub-ranges therein (e.g., 1-4, 1-3, 1-2, 2-5, 2-4, 2-3, 3-5, or 3-4 carbon atoms). In a fourth set of embodiments, any one or more of R¹, R³, and R⁶ is independently selected from a thioalkoxy group (SR), wherein R is selected from any of the hydrocarbon groups (R) described anywhere above and below, including any of the linear, branched, or cyclic alkyl or alkenyl groups described above having 1-5 carbon atoms or sub-ranges therein (e.g., 1-4, 1-3, 1-2, 2-5, 2-4, 2-3, 3-5, or 3-4 carbon atoms). In a fifth set of embodiments, any one or more of R¹, R³, and R⁶ is a nitrile (CN) group. In a sixth set of embodiments, any one or more of R¹, R³, and R⁶ is an amino (NH₂) group. In a seventh set of embodiments, any one or more of R¹, R³, and R⁶ is a hydroxy (OH) group. In an eighth set of embodiments, any one or more of R¹, R³, and R⁶ is a halogen atom (e.g., F, Cl, Br, or I atom). Any two or three of the foregoing first through eighth embodiments may also be combined, thereby requiring R¹, R³, and R⁶ to be selected from two or three different groups described above. In other embodiments, any one or more of the groups (i)-(viii) provided above may be independently excluded for R¹, R³, and/or R⁶.

The variable R² in Formula (5) is selected from the group consisting of: F, Cl, and methyl. In some embodiments, for any of the first through eighth embodiments or combinations thereof described above, R² is F. In other embodiments, for any of the first through eighth embodiments or combinations thereof described above, R² is Cl. In other embodiments, for any of the first through eighth embodiments or combinations thereof described above, R² is methyl.

In a first particular embodiment of Formula (5), R¹ is independently selected from H, methyl, ethyl, n-propyl, isopropyl, cyclopropyl, methoxy, ethoxy, F, and OH, or a sub-grouping of these. In a second particular embodiment, R³ is independently selected from H, methyl, ethyl, n-propyl, isopropyl, cyclopropyl, methoxy, ethoxy, F, and OH, or a sub-grouping of these. In a third particular embodiment, R⁶ is independently selected from H, methyl, ethyl, n-propyl, isopropyl, cyclopropyl, methoxy, ethoxy, F, and OH, or a sub-grouping of these. Any one of the first through third particular embodiments provided above may also be combined with each other and with embodiments of R² described above.

The group R²⁰ in Formula (5) is selected from H and hydrocarbon groups containing 1-5 carbon atoms (R), which are optionally substituted with one or more fluorine atoms. In one set of embodiments, R²⁰ is H. In another set of embodiments, R²⁰ is selected from among any of the hydrocarbon groups (R) described anywhere above and below, including any of the linear, branched, or cyclic alkyl or alkenyl groups described above having 1-5 carbon atoms or sub-ranges therein (e.g., 1-4, 1-3, 1-2, 2-5, 2-4, 2-3, 3-5, or 3-4 carbon atoms). In some embodiments, R²⁰ is selected from one or more of methyl, ethyl, n-propyl, isopropyl, n-butyl, sec-butyl, isobutyl, t-butyl, cyclopropyl, and cyclobutyl. In some embodiments, any one or more of the foregoing groups is excluded for R²⁰. Moreover, any one of the foregoing embodiments for R²⁰ may be combined with any one or more embodiments provided earlier above for R¹, R², R³, and R⁶.

The groups R^{a}, R^{b}, R^{c}, and R^{d} in Formula (5) are independently selected from H and D atoms. In one embodiment, R^{a}, R^{b}, R^{c}, and R^{d} are all H atoms. In another embodiment, precisely or at least one or two of R^{a}, R^{b}, R^{c}, and R^{d} are D atoms, the rest (if any) being H atoms. In another embodiment, R^{a}, R^{b}, R^{c}, and R^{d} are all D atoms. Moreover, any one of the foregoing embodiments for R^{a}, R^{b}, R^{c}, and R^{d} may be combined with any one or more embodiments provided earlier above for R¹, R², R³, R⁶, and R²⁰.

In any of the above embodiments of Formula (5), the following provision may apply: when R² is F, R³ is not methyl or OH.

In some embodiments, the compound of Formula (5) has the following structure: 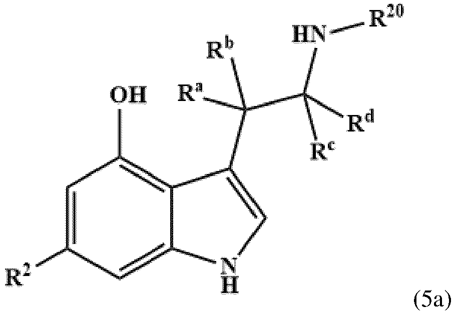 wherein R², R²⁰, R^{a}, R^{b}, R^{c}, and R^{d} are independently as defined under Formula (5) and any sub-embodiments thereof and combinations thereof, as provided above. Formula (5a) also includes all pharmaceutical salts thereof. In some embodiments of Formula (5a), R² is F. In other embodiments of Formula (5a), R² is Cl. In other embodiments of Formula (5a), R² is methyl.

In some embodiments, the compound of Formula (5) has the following structure: 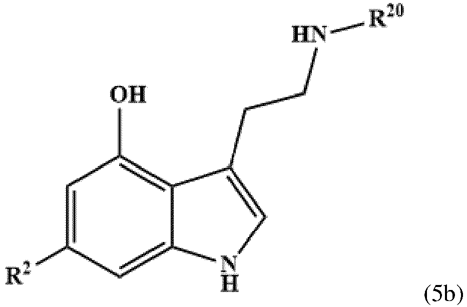 wherein R² and R²⁰ are independently as defined under Formula (5) and any sub-embodiments thereof and combinations thereof, as provided above. Formula (5b) also includes all pharmaceutical salts thereof. In some embodiments of Formula (5b), R² is F. In other embodiments of Formula (5b), R² is Cl. In other embodiments of Formula (5b), R² is methyl.

In some embodiments, the compound of Formula (5) has any of the following specific structures: 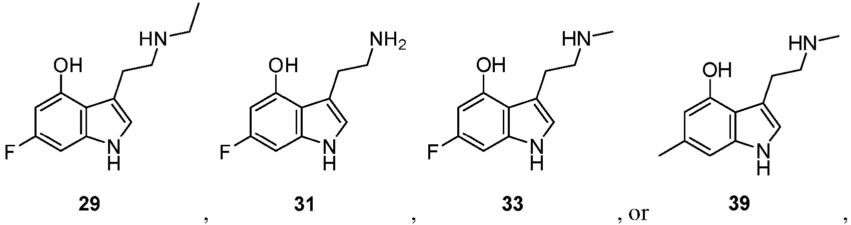 and pharmaceutically acceptable salts thereof.

In a sixth aspect, the present disclosure is directed to compounds of the following structure:

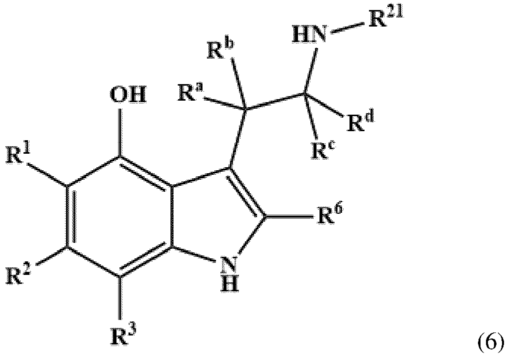

In Formula (6), R¹, R², and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms; R³ is selected from the group consisting of: F, Cl, and methyl; R^{a}, R^{b}, R^{c}, and R^{d} are independently selected from H and D atoms; and R²¹ is selected from the group consisting of H and hydrocarbon groups containing 1-5 carbon atoms (R), which are optionally substituted with one or more fluorine atoms. Pharmaceutically acceptable salts thereof are also within the scope of Formula (6) and all sub-formulas disclosed in this application.

In a first set of embodiments, any one, two, or all of R¹, R², and R⁶ are hydrogen atoms. In a second set of embodiments, any one or more of R¹, R², and R⁶ is independently selected from any of the hydrocarbon groups (R) described anywhere above and below, including any of the linear, branched, or cyclic alkyl or alkenyl groups described above having 1-5 carbon atoms or sub-ranges therein (e.g., 1-4, 1-3, 1-2, 2-5, 2-4, 2-3, 3-5, or 3-4 carbon atoms). In a third set of embodiments, any one or more of R¹, R², and R⁶ is independently selected from an alkoxy group (OR), wherein R is selected from any of the hydrocarbon groups (R) described anywhere above and below, including any of the linear, branched, or cyclic alkyl or alkenyl groups described above having 1-5 carbon atoms or sub-ranges therein (e.g., 1-4, 1-3, 1-2, 2-5, 2-4, 2-3, 3-5, or 3-4 carbon atoms). In a fourth set of embodiments, any one or more of R¹, R², and R⁶ is independently selected from a thioalkoxy group (SR), wherein R is selected from any of the hydrocarbon groups (R) described anywhere above and below, including any of the linear, branched, or cyclic alkyl or alkenyl groups described above having 1-5 carbon atoms or sub-ranges therein (e.g., 1-4, 1-3, 1-2, 2-5, 2-4, 2-3, 3-5, or 3-4 carbon atoms). In a fifth set of embodiments, any one or more of R¹, R², and R⁶ is a nitrile (CN) group. In a sixth set of embodiments, any one or more of R¹, R², and R⁶ is an amino (NH₂) group. In a seventh set of embodiments, any one or more of R¹, R², and R⁶ is a hydroxy (OH) group. In an eighth set of embodiments, any one or more of R¹, R², and R⁶ is a halogen atom (e.g., F, Cl, Br, or I atom). Any two or three of the foregoing first through eighth embodiments may also be combined, thereby requiring R¹, R², and R⁶ to be selected from two or three different groups described above. In other embodiments, any one or more of the groups (i)-(viii) provided above may be independently excluded for R¹, R², and/or R⁶.

The variable R³ in Formula (6) is selected from the group consisting of: F, Cl, and methyl. In some embodiments, for any of the first through eighth embodiments or combinations thereof described above, R³ is F. In other embodiments, for any of the first through eighth embodiments or combinations thereof described above, R³ is Cl. In other embodiments, for any of the first through eighth embodiments or combinations thereof described above, R³ is methyl.

In a first particular embodiment of Formula (6), R¹ is independently selected from H, methyl, ethyl, n-propyl, isopropyl, cyclopropyl, methoxy, ethoxy, F, and OH, or a sub-grouping of these. In a second particular embodiment, R² is independently selected from H, methyl, ethyl, n-propyl, isopropyl, cyclopropyl, methoxy, ethoxy, F, and OH, or a sub-grouping of these. In a third particular embodiment, R⁶ is independently selected from H, methyl, ethyl, n-propyl, isopropyl, cyclopropyl, methoxy, ethoxy, F, and OH, or a sub-grouping of these. Any one of the first through third particular embodiments provided above may also be combined with each other and with embodiments of R³ described above.

The group R²¹ in Formula (6) is selected from H and hydrocarbon groups containing 1-5 carbon atoms (R), which are optionally substituted with one or more fluorine atoms. In one set of embodiments, R²¹ is H. In another set of embodiments, R²¹ is selected from among any of the hydrocarbon groups (R) described anywhere above and below, including any of the linear, branched, or cyclic alkyl or alkenyl groups described above having 1-5 carbon atoms or sub-ranges therein (e.g., 1-4, 1-3, 1-2, 2-5, 2-4, 2-3, 3-5, or 3-4 carbon atoms). In some embodiments, R²¹ is selected from one or more of methyl, ethyl, n-propyl, isopropyl, n-butyl, sec-butyl, isobutyl, t-butyl, cyclopropyl, and cyclobutyl. In some embodiments, any one or more of the foregoing groups is excluded for R²¹. Moreover, any one of the foregoing embodiments for R²¹ may be combined with any one or more embodiments provided earlier above for R¹, R², R³, and R⁶.

The groups R^{a}, R^{b}, R^{c}, and R^{d} in Formula (6) are independently selected from H and D atoms. In one embodiment, R^{a}, R^{b}, R^{c}, and R^{d} are all H atoms. In another embodiment, precisely or at least one or two of R^{a}, R^{b}, R^{c}, and R^{d} are D atoms, the rest (if any) being H atoms. In another embodiment, R^{a}, R^{b}, R^{c}, and R^{d} are all D atoms. Moreover, any one of the foregoing embodiments for R^{a}, R^{b}, R^{c}, and R^{d} may be combined with any one or more embodiments provided earlier above for R¹, R², R³, R⁶, and R²¹.

In embodiments of Formula (6), the following first provision applies: when R³ is methyl, R² is not F and R⁶ is not methyl. In embodiments of Formula (6), the following second provision applies: when R³ is methyl, and R¹, R², R⁶, R^{a}, R^{b}, R^{c}, and R^{d} are all H, then R²¹ is not H, methyl, or ethyl. In embodiments of Formula (6), the following third provision applies: when R³ is F or Cl, and R¹, R², R⁶, R^{a}, R^{b}, R^{c}, and R^{d} are all H, then R²¹ is not H. Any one, two, or all three of the foregoing first, second, and third provisions may be combined.

In some embodiments, the compound of Formula (6) has the following structure: 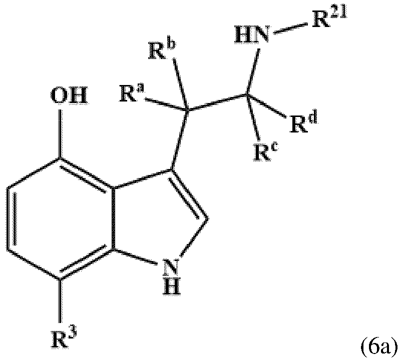 wherein R³, R²¹, R^{a}, R^{b}, R^{c}, and R^{d} are independently as defined under Formula (6) and any sub-embodiments thereof and combinations thereof, as provided above. Formula (6a) also includes all pharmaceutical salts thereof. In some embodiments of Formula (6a), R³ is F. In other embodiments of Formula (6a), R³ is Cl. In other embodiments of Formula (6a), R³ is methyl.

In some embodiments, the compound of Formula (6) has the following structure: 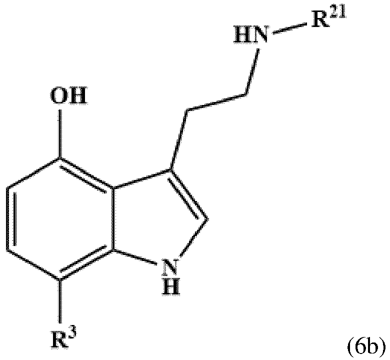 wherein R³ and R²¹ are independently as defined under Formula (6) and any sub-embodiments thereof and combinations thereof, as provided above. Formula (6b) also includes all pharmaceutical salts thereof. In some embodiments of Formula (6b), R³ is F. In other embodiments of Formula (6b), R³ is Cl. In other embodiments of Formula (6b), R³ is methyl.

In some embodiments, the compound of Formula (6b) has the following specific structure: 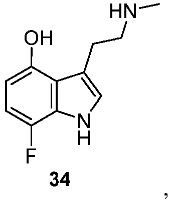 and pharmaceutically acceptable salts thereof.

In a seventh aspect, the present disclosure is directed to a compound having the following specific structure:

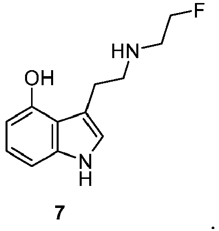

In an eighth aspect, the present disclosure is directed to any of the following specific compounds:

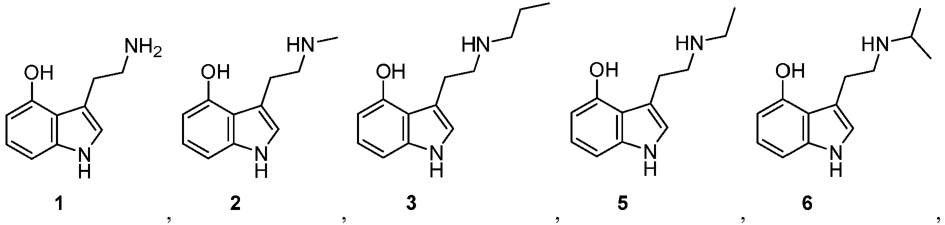

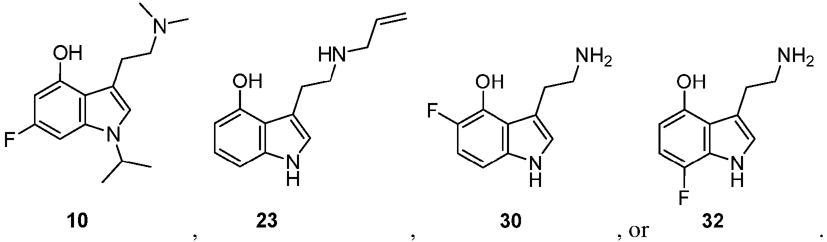

The present disclosure is also directed to pharmaceutical compositions, including dosage forms, of any of the compounds disclosed in this application. Pharmaceutical compositions typically include a pharmaceutically acceptable carrier in which the one or more active compounds are contained (e.g., dissolved, suspended, or admixed). As well known, the pharmaceutically acceptable carrier is composed of one or more substances that are considered safe and effective. The carrier includes all components present in the pharmaceutical formulation other than the active ingredient(s). The term "carrier" includes, but is not limited to, diluents, binders, lubricants, glidants, disintegrants, fillers, and coating compositions.

Suitable dosage forms for a compound disclosed herein include, but are not limited to, oral forms, such as tablets, hard or soft gelatin capsules, powders, granules and oral solutions, syrups or suspensions, troches, as well as sublingual, buccal, intratracheal, intraocular, or intranasal forms, forms adapted to inhalation, topical forms, transdermal forms, or parenteral forms, for example, forms adapted for intravenous, intra-arterial, intraperitoneal, intrathecal, intraventricular, intramuscular, or subcutaneous administration. In embodiments, for such parenteral administration, it may be in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

Pharmaceutical compositions include those suitable for oral, rectal, nasal, topical (including transdermal, buccal and sublingual), vaginal or parenteral (including subcutaneous, intramuscular, intravenous and intradermal) administration or administration via an implant. The compositions may be prepared by any method well known in the art of pharmacy. In commercial form, the pharmaceutical composition may be supplied with packaging material, including instructions for the use of the composition as herein described.

Pharmaceutical compositions herein may also be provided with immediate release, delayed release, extended release, or modified release profiles. In embodiments, pharmaceutical compositions with different drug release profiles may be combined to create a two-phase or three-phase release profile. For example, pharmaceutical compositions may be provided with an immediate release and an extended-release profile. In embodiments, pharmaceutical compositions may be provided with an extended release and delayed release profile. Such composition may be provided as pulsatile formulations, multilayer tablets, or capsules containing tablets, beads, granules, or the like.

The pharmaceutical composition may also include one or more auxiliary agents. The auxiliary agent(s), also may also be referred to as accessory ingredient(s), include any of the additional conventional substances of the art, such as fillers, binders, diluents, disintegrants, lubricants, colorants, flavoring agents, anti-oxidants, and wetting agents. Such auxiliary agents are suitably selected with respect to the intended form and route of administration and as consistent with conventional pharmaceutical practices.

Pharmaceutical compositions suitable for oral administration may be formulated as discrete dosage units, such as pills, tablets, dragées or capsules, or as a powder or granules, or as a solution or suspension. The active ingredient may also be presented as a bolus or paste. The compositions can further be processed into a suppository or enema for rectal administration.

Tablets may contain the active ingredient compound(s) and suitable binders, lubricants, disintegrating agents, coloring agents, flavoring agents, flow-inducing agents, and melting agents. Gelatin capsules may contain the active ingredient compound(s) and powdered carriers, such as lactose, starch, cellulose derivatives, magnesium stearate, stearic acid, and the like. Similar diluents can be used to make compressed tablets. Compressed tablets can be sugar coated or film coated to mask any unpleasant taste and protect the tablet from the atmosphere, or enteric coated for selective disintegration in the gastrointestinal tract. For instance, for oral administration in the dosage unit form of a tablet or capsule, the active drug component can be combined with an oral, non-toxic, pharmaceutically acceptable, inert carrier such as lactose, gelatin, agar, starch, sucrose, glucose, methyl cellulose, magnesium stearate, dicalcium phosphate, calcium sulfate, mannitol, sorbitol and the like. Suitable binders include starch, gelatin, natural sugars such as glucose or beta-lactose, corn sweeteners, natural and synthetic gums such as acacia, tragacanth, or sodium alginate, carboxymethylcellulose, polyethylene glycol, waxes, and the like. Lubricants used in these dosage forms include sodium oleate, sodium stearate, magnesium stearate, sodium benzoate, sodium acetate, sodium chloride, and the like. Disintegrators include, without limitation, starch, methyl cellulose, agar, bentonite, xanthan gum, and the like.

For oral administration in liquid dosage form, the oral drug components are combined with any oral, non-toxic, pharmaceutically acceptable inert carrier, such as ethanol, glycerol, water, and the like. Examples of suitable liquid dosage forms include, but are not limited to, solutions or suspensions in water, pharmaceutically acceptable fats and oils, alcohols or other organic solvents, including esters, emulsions, syrups or elixirs, suspensions, solutions and/or suspensions reconstituted from non-effervescent granules and effervescent preparations reconstituted from effervescent granules. Such liquid dosage forms may contain, for example, suitable solvents, preservatives, emulsifying agents, suspending agents, diluents, sweeteners, thickeners, and melting agents. Liquid dosage forms for oral administration can contain coloring and flavoring to increase patient acceptance.

For parenteral administration, suitable compositions include aqueous and non-aqueous sterile solutions. In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water-soluble salt of the active ingredient, suitable stabilizing agents, and if necessary, buffer substances. Anti-oxidizing agents, such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl- or propyl-paraben, and chlorobutanol. The compositions may be presented in unit-dose or multi-dose containers, such as sealed vials and ampoules, and may be stored in a freeze-dried (lyophilized) condition requiring only the addition of a sterile liquid carrier, typically water or an aqueous solution, prior to use. For transdermal administration, gels, patches, or sprays, among others, may be used. Compositions or formulations suitable for pulmonary administration, such as by nasal inhalation, include fine dusts or mists which may be generated by means of metered dose pressurized aerosols, nebulizers or insufflators. Parenteral and intravenous forms may also include minerals and other materials to make them compatible with the type of injection or delivery system chosen.

The compounds used in the method of the present disclosure may also be administered in the form of liposome delivery systems, such as small unilamellar vesicles, large unilamellar vesicles, and multilamellar vesicles. Liposomes can be formed from a variety of phospholipids, such as cholesterol, stearylamine, or phosphatidylcholines. The compounds may be administered as components of tissue-targeted emulsions.

The compounds used in the method of the present disclosure may also be coupled to soluble polymers as targetable drug carriers or as prodrugs. Such polymers include polyvinylpyrrolidone, pyran copolymer, polyhydroxylpropylmethacrylamide-phenol, polyhydroxyethylasparta-midephenol, or polyethyleneoxide-polylysine substituted with palmitoyl residues. Furthermore, the compounds may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacylates, and crosslinked or amphipathic block copolymers of hydrogels.

Pharmaceutical compositions herein may be provided with abuse deterrent features by techniques know in the art, for example, by making a tablet that is difficult to crush or to dissolve in water.

In another aspect, the present disclosure is directed to methods for treating a mood disorder. In the method, one or more compounds described herein, typically in the form of a pharmaceutical composition, is administered to a subject in need thereof in a therapeutically effective amount. The therapeutically effective amount is an amount that provides an improvement (typically, a lessening of or a shortening of the duration of) one or more symptoms of the mood disorder or that prevents a recurrence of one or more symptoms of the mood disorder.

In embodiments, the mood disorder is a depressive disorder. Some examples of depressive disorders include major depressive disorder, persistent depressive disorder, postpartum depression, premenstrual dysphoric disorder, seasonal affective disorder, psychotic depression, disruptive mood dysregulation disorder, substance/medication-induced depressive disorder, and depressive disorder due to another medical condition. The depressive disorder may also be a treatment-resistant depressive disorder. Any one or more compounds described in this disclosure may be administered to treat a depressive disorder, including any of those specifically pointed out above.

In some embodiments, depression conditions include major depressive disorder and dysthymic disorder. In some embodiments, depression conditions develop under unique circumstances, including, but are not limited to, psychotic depression, postpartum depression, seasonal affective disorder (SAD), mood disorder, depressions caused by chronic medical conditions such as cancer or chronic pain, chemotherapy, chronic stress, post traumatic stress disorders, and bipolar disorder (or manic depressive disorder). In some embodiments, depression conditions that are expected to be treated according to this aspect of the present disclosure include, but are not limited to, major depressive disorder, dysthymic disorder, psychotic depression, postpartum depression, premenstrual syndrome, premenstrual dysphoric disorder, seasonal affective disorder (SAD), anxiety, mood disorder, depressions caused by chronic medical conditions such as cancer or chronic pain, chemotherapy, chronic stress, post traumatic stress disorders, and bipolar disorder (or manic depressive disorder). Any one or more compounds described in this disclosure may be administered to treat any of the depressive disorders specifically pointed out above.

Also provided herein are methods of treating refractory depression, e.g., patients suffering from a depressive disorder that does not, and/or has not, responded to adequate courses of at least one, or at least two, other antidepressant compounds or therapeutics. For example, provided herein is a method of treating depression in a treatment-resistant patient, comprising a) optionally identifying the patient as treatment resistant and b) administering an effective dose of a disclosed compound. As used herein, the term "depressive disorder" encompasses refractory depression. In some embodiments, refractory depression occurs in patients suffering from depression who are resistant to standard pharmacological treatments, including tricyclic antidepressants, MAOIs, SSRIs, and double and triple uptake inhibitors and/or anxiolytic drugs, as well non-pharmacological treatments such as psychotherapy, electroconvulsive therapy, vagus nerve stimulation and/or transcranial magnetic stimulation. In some embodiments, a treatment-resistant patient may be identified as one who fails to experience alleviation of one or more symptoms of depression (e.g., persistent anxious or sad feelings, feelings of helplessness, hopelessness, pessimism), despite undergoing one or more standard pharmacological or non-pharmacological treatments. In certain embodiments, a treatment-resistant patient is one who fails to experience alleviation of one or more symptoms of depression despite undergoing treatment with two different antidepressant drugs. In other embodiments, a treatment-resistant patient is one who fails to experience alleviation of one or more symptoms of depression despite undergoing treatment with four different antidepressant drugs. In some embodiments, a treatment-resistant patient may also be identified as one who is unwilling or unable to tolerate the side effects of one or more standard pharmacological or non-pharmacological treatment. Any one or more compounds described in this disclosure may be administered to treat a refractory or treatment-resistant depressive disorder, including any of those specifically pointed out above.

In some embodiments, symptoms associated with depression include, but are not limited to, persistent anxious or sad feelings, feelings of helplessness, hopelessness, pessimism, and/or worthlessness, low energy, restlessness, irritability, fatigue, loss of interest in pleasurable activities or hobbies, excessive sleeping, overeating, appetite loss, insomnia, thoughts of suicide, or suicide attempts. In some embodiments, various symptoms associated with anxiety include fear, panic, heart palpitations, shortness of breath, fatigue, nausea, and headaches among others. In addition, patients suffering from any form of depression often experience anxiety. It is expected that the methods of the present condition can be used to treat anxiety or any of the symptoms thereof. In some embodiments, presence, severity, frequency, and duration of symptoms of depression vary on a case-to-case basis.

In other embodiments, the mood disorder is a bipolar or related disorder. Some examples of bipolar and related disorders include bipolar I disorder, bipolar II disorder, cyclothymic disorder, substance/medication-induced bipolar and related disorder, and bipolar and related disorder due to another medical condition. Any one or more compounds described in this disclosure may be administered to treat a bipolar or related disorder, including any of those specifically pointed out above.

In other embodiments, the mood disorder is a substance-related disorder. Substance use disorders typically involve abuse of psychoactive compounds, such as alcohol, caffeine, cannabis, inhalants, opioids, sedatives, hypnotics, anxiolytics, stimulants, nicotine and tobacco. As used herein "substance" or "substances" are psychoactive compounds that can be addictive, such as alcohol, caffeine, cannabis, hallucinogens, inhalants, opioids, sedatives, hypnotics, anxiolytics, stimulants, nicotine and tobacco. The method described herein may be used for treating or preventing a substance use craving, diminishing a substance use craving, and/or facilitating substance use cessation or withdrawal. In some embodiments, the method may be used to facilitate smoking cessation or cessation of opioid use. Any one or more compounds described in this disclosure may be administered to treat a substance-related disorder, including any of those specifically pointed out above.

In other embodiments, the mood disorder is an anxiety disorder. Some examples of anxiety disorders include separation anxiety disorder, selective mutism, specific phobia, social anxiety disorder (social phobia), panic disorder, panic attack, agoraphobia, generalized anxiety disorder, substance/medication-induced anxiety disorder, and anxiety disorder due to another medical condition. Any one or more compounds described in this disclosure may be administered to treat an anxiety disorder, including any of those specifically pointed out above.

In other embodiments, the mood disorder is an obsessive-compulsive or related disorder, such as obsessive-compulsive disorder, body dysmorphic disorder, hoarding disorder, trichotillomania (hair-pulling disorder), excoriation (skin-picking) disorder, substance/medication-induced obsessive-compulsive and related disorder, and obsessive-compulsive and related disorder due to another medical condition. Any one or more compounds described in this disclosure may be administered to treat an obsessive-compulsive or related disorder, including any of those specifically pointed out above.

In other embodiments, the mood disorder is a trauma- or stressor-related disorders. Some examples of such mood disorders include reactive attachment disorder, disinhibited social engagement disorder, posttraumatic stress disorder, acute stress disorder, and adjustment disorders. Any one or more compounds described in this disclosure may be administered to treat a trauma- or stressor-related disorder, including any of those specifically pointed out above.

In other embodiments, the mood disorder is a feeding and eating disorder. Some examples of such mood disorders include anorexia nervosa, bulimia nervosa, binge-eating disorder, pica, rumination disorder, and avoidant/restrictive food intake disorder. Any one or more compounds described in this disclosure may be administered to treat a feeding or eating disorder, including any of those specifically pointed out above.

In other embodiments, the mood disorder is a neurocognitive disorder. Some examples of neurocognitive disorders include delirium, major neurocognitive disorder, mild neurocognitive disorder, major or mild neurocognitive disorder due to Alzheimer's disease, major or mild frontotemporal neurocognitive disorder, major or mild neurocognitive disorder with Lewy bodies, major or mild vascular neurocognitive disorder, major or mild neurocognitive disorder due to traumatic brain injury, substance/medication-induced major or mild neurocognitive disorder, major or mild neurocognitive disorder due to HIV infection, major or mild neurocognitive disorder due to prion disease, major or mild neurocognitive disorder due to Parkinson's disease, major or mild neurocognitive disorder due to Huntington's disease, major or mild neurocognitive disorder due to another medical condition, and major or mild neurocognitive disorder due to multiple etiologies. Any one or more compounds described in this disclosure may be administered to treat a neurocognitive disorder, including any of those specifically pointed out above.

In other embodiments, the mood disorder is a neurodevelopmental disorder. Some examples of neurodevelopmental disorders include autism spectrum disorder, attention-deficit/hyperactivity disorder, stereotypic movement disorder, tic disorders, Tourette's disorder, persistent (chronic) motor or vocal tic disorder, and provisional tic disorder. In some embodiments, a variety of other neurological conditions may be treated according to the methods of the present disclosure. In some embodiments, neurological conditions include, but are not limited to, a learning disorder, autistic disorder (e.g., autism spectrum disorder), attention-deficit hyperactivity disorder, Tourette's syndrome, phobia, post-traumatic stress disorder, dementia, AIDS dementia, Alzheimer's disease, Parkinson's disease, spasticity, myoclonus, muscle spasm, bipolar disorder, a substance abuse disorder, urinary incontinence, and schizophrenia. Any one or more compounds described in this disclosure may be administered to treat a neurodevelopmental disorder, including any of those specifically pointed out above.

In other embodiments, the mood disorder is a personality disorder. The personality disorder may be within any of the known clusters, e.g., clusters A, B, or C. Some examples of personality disorders include paranoid personality disorder, schizoid personality disorder, schizotypal personality disorder, borderline personality disorder, antisocial personality disorder, narcissistic personality disorder, histrionic personality disorder, avoidant personality disorder, obsessive-compulsive personality disorder, and dependent personality disorder. Any one or more compounds described in this disclosure may be administered to treat a personality disorder, including any of those specifically pointed out above.

In other embodiments, the mood disorder may be associated with a sexual dysfunction. Some examples of such mood disorders include delayed ejaculation, erectile disorder, female orgasmic disorder, female sexual interest/arousal disorder, genito-pelvic pain/penetration disorder, male hypoactive sexual desire disorder, premature (early) ejaculation, and substance/medication-induced sexual dysfunction. Any one or more compounds described in this disclosure may be administered to treat a sexual dysfunction disorder, including any of those specifically pointed out above.

In other embodiments, the mood disorder is gender dysphoria. Any one or more compounds described in this disclosure may be administered to treat the gender dysphoria.

In another aspect, the present disclosure is directed to methods for treating migraine, cluster headache, or other headache disorders. In the method, one or more compounds described herein, typically in the form of a pharmaceutical composition, is administered to a subject in need thereof in a therapeutically effective amount. The therapeutically effective amount is an amount that provides an improvement (typically, a lessening of or a shortening of the duration of) one or more symptoms of the headache disorder or that prevents a recurrence of one or more symptoms of the headache disorder. Any one or more compounds described in this disclosure may be administered to treat the migraine, cluster headache, or other headache disorder.

In another aspect, the present disclosure is directed to methods for treating inflammation or a condition associated with inflammation (e.g., arthritis). In the method, one or more compounds described herein, typically in the form of a pharmaceutical composition, is administered to a subject in need thereof in a therapeutically effective amount. The therapeutically effective amount is an amount that provides an improvement (typically, a lessening of or a shortening of the duration of) one or more symptoms of the inflammation or that prevents a recurrence of one or more symptoms of the inflammation. Any one or more compounds described in this disclosure may be administered to treat inflammation or a condition associated with inflammation.

In embodiments, methods include treating a mood disorder, *e.g.,* a depressive disorder, by administering to a patient in need thereof about 0.01 mg to about 400 mg of a compound disclosed herein. The exact dose and regimen of administration of the composition will necessarily be dependent upon the type and magnitude of the therapeutic or nutritional effect to be achieved and may vary depending on factors such as the particular compound, formula, route of administration, or age and condition of the individual subject to whom the composition is to be administered.

In embodiments, the dosage being administered may be in the range of about, for example, 0.01 to 400 mg, 0.01 to 300 mg, 0.01 to 250 mg, 0.01 to 200 mg, 0.01 to 150 mg, 0.01 to 100 mg, 0.01 to 75 mg, 0.01 to 50 mg, 0.01 to 25 mg, 0.01 to 20 mg, 0.01 to 15 mg, 0.01 to 10 mg, 0.01 to 5 mg, 0.01 to 1 mg, 0.01 to 0.5 mg, 0.01 to 0.1 mg, 0.1 to 400 mg, 0.1 to 300 mg, 0.1 to 250 mg, 0.1 to 200 mg, 0.1 to 150 mg, 0.1 to 100 mg, 0.1 to 75 mg, 0.1 to 50 mg, 0.1 to 25 mg, 0.1 to 20 mg, 0.1 to 15 mg, 0.1 to 10 mg, 0.1 to 5 mg, 0.1 to 1 mg, 0.5 to 400 mg, 0.5 to 300 mg, 0.5 to 250 mg, 0.5 to 200 mg, 0.5 to 150 mg, 0.5 to 100 mg, 0.5 to 75 mg, 0.5 to 50 mg, 0.5 to 25 mg, 0.5 to 20 mg, 0.5 to 15 mg, 0.5 to 10 mg, 0.5 to 5 mg, 0.5 to 1 mg, 1 to 400 mg, 1 to 300 mg, 1 to 250 mg, 1 to 200 mg, 1 to 150 mg, 1 to 100 mg, 1 to 75 mg, 1 to 50 mg, 1 to 25 mg, 1 to 20 mg, 1 to 15 mg, 1 to 10 mg, 1 to 5 mg, 5 to 400 mg, 5 to 300 mg, 5 to 250 mg, 5 to 200 mg, 5 to 150 mg, 5 to 100 mg, 5 to 75 mg, 5 to 50 mg, 5 to 25 mg, 5 to 20 mg, 5 to 15 mg, 5 to 10 mg, 10 to 400 mg, 10 to 300 mg, 10 to 250 mg, 10 to 200 mg, 10 to 150 mg, 10 to 100 mg, 10 to 50 mg, 10 to 25 mg, 10 to 20 mg, 10 to 15 mg, 20 to 400 mg, 20 to 300 mg, 20 to 250 mg, 20 to 200 mg, 20 to 150 mg, 20 to 100 mg, 20 to 50 mg, 20 to 40 mg, 50 to 300 mg, 50 to 250 mg, 50 to 200 mg, 50 to 150 mg, 50 to 100 mg, 100 to 300 mg, 100 to 250 mg, 100 to 200 mg. In some embodiments, the dosage is precisely or about 0.25 mg, 0.5 mg, 0.75 mg, 1 mg, 1.25 mg, 1.5 mg, 1.75 mg, 2.0 mg, 2.5 mg, 3.0 mg, 3.5 mg, 4.0 mg, 4.5 mg, 5 mg, 10 mg, 15 mg, 20 mg, 25 mg, 30, mg, 35 mg, 40 mg, 45 mg, 50 mg, 75 mg, 80 mg, 100 mg, 120 mg, 125 mg, 150 mg, 175 mg, 200 mg, 225 mg, 250 mg, 275 mg, 300 mg, or 400 mg, or an amount within a range between any two of these values (e.g., 2-5 mg, 40-80 mg, 80-100 mg, 80-120 mg, 100-120 mg, or 120-150 mg). In some embodiments, any of the above dosages is per 50, 60, or 70 kg of body weight. Any of the above dosages may be administered once, twice, or thrice per day or per every two days, or per every three days, or per every week or two weeks. Any one or more compounds described in this disclosure may be administered in any amount provided above, or a range between any two of these values, to treat any of the mood disorders described above.

In specific embodiments, dosages may include amounts of a compound disclosed herein in the range of about, *e.g.,* 1 mg to 200 mg, 1 mg to 100 mg, 1 mg to 50 mg, 1 mg to 40 mg, 1 mg to 30 mg, 1 mg to 20 mg, 1 mg to 15 mg, 0.01 mg to 10 mg, 0.1 mg to 15 mg, 0.15 mg to 12.5 mg, or 0.2 mg to 10 mg, with doses of 0.1 mg, 0.2 mg, 0.3 mg, 0.4 mg, 0.5 mg, 0.6 mg, 0.7 mg, 0.8 mg, 0.9 mg, 1.0 mg, 1.5 mg, 1.75 mg, 2 mg, 2.5 mg, 2.75 mg, 3 mg, 3.5 mg, 3.75 mg, 4 mg, 4.5 mg, 4.75 mg, 5 mg, 5.5 mg, 6 mg, 6.5 mg, 7 mg, 7.5 mg, 8 mg, 8.5 mg, 9 mg, 9.5 mg, 10 mg, 11 mg, 12 mg, 15 mg, 20 mg, 25 mg, 30 mg, 35 mg, 40 mg, 45 mg, 50 mg, 60 mg, 75 mg, 80 mg, 90 mg, 100 mg, 125 mg, 150 mg, and 200 mg being specific examples of doses. Any of the above dosages may be administered once, twice, or thrice per day or per every two days, or per every three days, or per every week or two weeks.

Typically, a compound disclosed herein is administered once, twice, three or four times daily, every other day, every three days, once weekly, twice monthly, once monthly, or 3-4 times yearly to a patient in need thereof. In some embodiments, the dosage is about, *e.g.,* 1-400 mg/day, or 1-300 mg/day, or 1-250 mg/day, or 1-200 mg/day, for example 300 mg/day, 250 mg/day, 200 mg/day, 150 mg/day, 100 mg/day, 75 mg/day, 50 mg/day, 40 mg/day, 30 mg/day, 25 mg/day, 20 mg/day, 15 mg/day, 10 mg/day, 5 mg/day, or 1 mg/day.

In some embodiments, pharmaceutical compositions for parenteral administration or inhalation, e.g., a spray or mist, of a compound disclosed herein include a concentration of about 0.005 mg/ml to about 500 mg/mL. In embodiments, the compositions include a compound disclosed herein at a concentration of, *e.g.,* about 0.05 mg/mL to about 50 mg/mL, about 0.05 mg/mL to about 100 mg/mL, about 0.005 mg/mL to about 500 mg/mL, about 0.1 mg/mL to about 50 mg/mL, about 0.1 mg/mL to about 10 mg/mL, about 0.05 mg/mL to about 25 mg/mL, about 0.05 mg/mL to about 10 mg/mL, about 0.05 mg/mL to about 5 mg/mL, or about 0.05 mg/mL to about 1 mg/mL.

In embodiments, the composition includes a compound disclosed herein at a concentration of, *e.g.,* about 0.05 mg/mL to about 15 mg/mL, about 0.5 mg/mL to about 10 mg/mL, about 0.25 mg/mL to about 5 mg/mL, about 0.5 mg/mL to about 7 mg/mL, about 1 mg/mL to about 10 mg/mL, about 5 mg/mL to about 10 mg/mL, about 5 mg/mL to about 15 mg/mL, about 5 mg/mL to 25 mg/mL, about 5 mg/mL to 50 mg/mL, or about 10 mg/mL to 100 mg/mL. In embodiments, the pharmaceutical compositions are formulated as a total volume of about, e.g., 10 mL, 20 mL, 25 mL, 50 mL, 100 mL, 200 mL, 250 mL, or 500 mL.

Typically, dosages may be administered to a subject once, twice, three or four times daily, every other day, every three days, once weekly, twice monthly, once monthly, or 3-4 times yearly. In embodiments, a compound disclosed herein is administered to a subject once in the morning or once in the evening. In embodiments, a compound disclosed herein is administered to a subject once in the morning, and once in the evening. In embodiments, a compound disclosed herein is administered to a subject three times a day *(e.g.,* at breakfast, lunch, and dinner), at a dose, *e.g.,* of 50 mg/administration *(e.g.,* 150 mg/day).

In embodiments, a compound disclosed herein is administered to a subject 12.5 mg/day in one or more doses. In embodiments, a compound disclosed herein is administered to a subject 25 mg/day in one or more doses. In embodiments, a compound disclosed herein is administered to a subject 35 mg/day in one or more doses. In embodiments, a compound disclosed herein is administered to a subject 50 mg/day in one or more doses. In embodiments, a compound disclosed herein is administered to a subject 75 mg/day in one or more doses. In embodiments, a compound disclosed herein is administered to a subject 100 mg/day in one or more doses. In embodiments, a compound disclosed herein is administered to a subject 150 mg/day in one or more doses. In embodiments, a compound disclosed herein is administered to a subject 200 mg/day in one or more doses. In embodiments, a compound disclosed herein is administered to a subject 250 mg/day in one or more doses.

In embodiments, the dosage of a compound disclosed herein is 0.0005-5 mg/kg, 0.001-1 mg/kg, 0.01-1 mg/kg or 0.1-5 mg/kg once, twice, three times or four times daily. For example, in embodiments, the dosage is 0.0005 mg/kg, 0.001 mg/kg, 0.005 mg/kg, 0.01 mg/kg, 0.025 mg/kg, 0.05 mg/kg, 0.1 mg/kg, 0.15 mg/kg, 0.2 mg/kg, 0.25 mg/kg, 0.3 mg/kg, 0.4 mg/kg, 0.5 mg/kg, 0.75 mg/kg, 1 mg/kg, 2.5 mg/kg, or 5 mg/kg, once, twice, three times or four times daily. In embodiments, a subject is administered a total daily dose of 0.01 mg to 500 mg of a compound disclosed herein once, twice, three times, or four times daily. In embodiments, the total amount administered to a subject in a 24-hour period is, *e.g.,* 0.01 mg, 0.025 mg, 0.05 mg, 0.075 mg, 0.1 mg, 0.125 mg, 0.15 mg, 0.175 mg, 0.2 mg, 0.25 mg, 0.3 mg, 0.4 mg, 0.5 mg, 0.75 mg, 1 mg, 1.5 mg, 2 mg, 2.5 mg, 3 mg, 4 mg, 5 mg, 7.5 mg, 10 mg, 12.5 mg, 15 mg, 17.5 mg, 20 mg, 25 mg, 30 mg, 35 mg, 40 mg, 45 mg, 50 mg, 60 mg, 75 mg, 80 mg, 90 mg, 100 mg, 125 mg, 150 mg, 175 mg, 200 mg, 250 mg, 300 mg, 400 mg, or 500 mg. In embodiments, the subject may be started at a low dose and the dosage is escalated. In embodiments, the subject may be started at a high dose and the dosage is decreased.

In embodiments, a compound disclosed herein may be administered, e.g., via inhalation or orally, at specified intervals. For example, during treatment a patient may be administered a compound disclosed herein at intervals of every, *e.g.,* 1 year, 6 months, 90 days, 60 days, 30 days, 14 days, 7 days, 3 days, 24 hours, 12 hours, 8 hours, 6 hours, 5 hours, 4 hours, 3 hours, 2.5 hours, 2.25 hours, 2 hours, 1.75 hours, 1.5 hours, 1.25 hours, 1 hour, 0.75 hour, 0.5 hour, or 0.25 hour.

In embodiments, a compound of the present disclosure or a pharmaceutically acceptable salt thereof is administered to a patient under the supervision of a healthcare provider. In embodiments, a compound of the present disclosure or a pharmaceutically acceptable salt thereof is administered to a patient under the supervision of a healthcare provider at a clinic specializing in the delivery of psychoactive treatments.

In embodiments, a compound of the present disclosure is administered to a patient under the supervision of a healthcare provider at a high dose intended to induce a psychedelic experience in the subject, e.g., 12.5 mg, 15 mg, 17.5 mg, 20 mg, 25 mg, 30 mg, 35 mg, 40 mg, 45 mg, 50 mg, 60 mg, 70 mg, 80 mg, 90 mg, 100 mg, 125 mg, or 150 mg. In some embodiments, the administration to a patient of a high dose under the supervision of a healthcare provider occurs periodically in order to maintain a therapeutic effect in the patient, e.g., every three days, twice weekly, once weekly, twice monthly, once monthly, thrice yearly, twice yearly, or once yearly.

In some embodiments, a compound of the present disclosure or a pharmaceutically acceptable salt thereof is administered by a patient on their own at home or otherwise away from the supervision of a healthcare provider. In some embodiments, a compound of the present disclosure or a pharmaceutically acceptable salt thereof is administered by a patient on their own at home or otherwise away from the supervision of a healthcare provider at a low dose intended to be sub-perceptual or to induce threshold psychoactive effects, *e.g.,* 0.1 mg, 0.25 mg, 0.5 mg, 0.75 mg, 1 mg, 1.5 mg, 2 mg, 2.5 mg, 3 mg, 4 mg, 5 mg, 7.5 mg, or 10 mg. In some embodiments, the administration by a patient of a low dose on their own occurs periodically in order to maintain a therapeutic effect in the patient, e.g., daily, every other day, every three days, twice weekly, once weekly, twice monthly, or once monthly.

In some embodiments, any compound (or pharmaceutical composition thereof) disclosed in this application may be administered one, two, three, or four times per day, wherein the administrations may be the same or different and are independently selected from any of the dosages provided in this application. In some embodiments, any compound (or pharmaceutical composition thereof) disclosed in this application may be administered one, two, three, four, five, six, seven, eight, nine, or ten times per week (possibly at equally spaced or alternating intervals), wherein the administrations may be the same or different and are independently selected from any of the dosages provided in this application. In some embodiments, any compound (or pharmaceutical composition thereof) disclosed in this application may be administered one, two, three, four, five, six, seven, eight, nine, ten, eleven, twelve, thirteen, fourteen, or fifteen times per month (possibly at equally spaced or alternating intervals, such as every two, three, four, or five days, or alternating between any of these), wherein the administrations may be the same or different and are independently selected from any of the dosages provided in this application. In some embodiments, any compound (or pharmaceutical composition thereof) disclosed in this application may be administered one, two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, or twenty-five times per year (possibly at equally spaced or alternating intervals, such as every two, three, or four weeks, or once or twice a month, or alternating between any of these), wherein the administrations may be the same or different and are independently selected from any of the dosages provided in this application.

In some embodiments, the treatment with the compound may be a component of a combination therapy or an adjunct therapy. For example, the subject or patient in need of the drug is treated or given another drug for the disease in conjunction with one or more of the presently described compounds. The combination therapy can be a sequential therapy wherein the patient is treated first with one drug and then another drug. Alternatively, the combination therapy can involve administering two drugs simultaneously. These can be administered independently by the same route or by two or more different routes of administration depending on the dosage forms employed.

In some embodiments, a compound disclosed herein may be administered in combination with one or more other antidepressant drugs, such as, tricyclic antidepressants, MAOIs, SSRIs, and double and triple uptake inhibitors and/or anxiolytic drugs for manufacturing a medicament for treating depression, anxiety, and/or other related diseases, including to provide relief from depression or anxiety and preventing recurrence of depression or anxiety. In some embodiments, therapeutics that may be used in combination with a compound of the present disclosure include, but are not limited to, Anafranil, Adapin, Aventyl, Elavil, Norpramin, Pamelor, Pertofrane, Sinequan, Surmontil, Tofranil, Vivactil, Parnate, Nardil, Marplan, Celexa, Lexapro, Luvox, Paxil, Prozac, Zoloft, Wellbutrin, Effexor, Remeron, Cymbalta, Desyrel (trazodone), and Ludiomill.

In the context of the present disclosure the term "5-HT2A receptor agonist" is intended to mean any compound or substance that activates the 5-HT2A receptor. The agonist may be a partial or full agonist.

As used herein, the term "pharmaceutically acceptable" refers to molecular entities and compositions that are "generally regarded as safe", *e.g.,* that are physiologically tolerable and do not typically produce an allergic or similar untoward reaction when administered to a human. In embodiments, this term refers to molecular entities and compositions approved by a regulatory agency of the federal or a state government, as the GRAS list under sections 204(s) and 409 of the Federal Food, Drug and Cosmetic Act, that is subject to premarket review and approval by the FDA or similar lists, the U.S. Pharmacopeia or another generally recognized pharmacopeia for use in animals, and more particularly in humans.

As used herein, the term "pharmaceutically acceptable salt" includes both acid and base addition salts, wherein the compound is modified by making acid or base salts thereof. Any compound disclosed in this application may be in the form of a pharmaceutically acceptable salt. Examples of pharmaceutically acceptable salts include but are not limited to mineral or organic acid salts of basic residues such as amines, and alkali or organic salts of acidic residues such as carboxylic acids. Pharmaceutically acceptable salts include conventional non-toxic salts or quaternary ammonium salts of the parent compound formed, for example, from non-toxic inorganic or organic acids. Such conventional non-toxic salts include those derived from inorganic acids such as hydrochloric, hydrobromic, sulfuric, sulfamic, phosphoric, and nitric acids; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, tolunesulfonic, naphthalenesulfonic, methanesulfonic, ethane disulfonic, and oxalic acids. The pharmaceutically acceptable salts of a compound disclosed herein can be synthesized from the parent compound, which contains a basic or acidic moiety, by conventional chemical methods.

The terms "about" or "approximately," as used herein, mean within an acceptable error range for the particular value as determined by one of ordinary skill in the art, which will depend in part on how the value is measured or determined, *i.e.,* the limitations of the measurement system. For example, "about" can mean within 3 or more than 3 standard deviations, per the practice in the art. Alternatively, "about" can mean a range of up to 20%, a range up to 10%, a range up to 5%, and/or a range up to 1% of a given value. Alternatively, particularly with respect to biological systems or processes, the term can mean within an order of magnitude, e.g., within 5-fold, or within 2-fold, of a value. "About" and "approximately" are used interchangeably herein.

In embodiments, the terms "effective amount" or "therapeutically effective amount" refer to an amount of a compound, material, composition, medicament, or other material that is effective to achieve a particular pharmacological and/or physiologic effect including but not limited to reducing the frequency or severity of sadness or lethargy, depressed mood, anxious or sad feelings, diminished interest in all or nearly all activities, significant increased or decreased appetite leading to weight gain or weight loss, insomnia, irritability, fatigue, feelings of worthlessness, feelings of helplessness, inability to concentrate, and recurrent thoughts of death or suicide, or to provide a desired pharmacologic and/or physiologic effect, for example, reducing, inhibiting, or reversing one or more of the underlying pathophysiological mechanisms underlying the neurological dysfunction, modulating dopamine levels or signaling, modulating serotonin levels or signaling, modulating norepinephrine levels or signaling, modulating glutamate or GABA levels or signaling, modulating synaptic connectivity or neurogenesis in certain brain regions, or a combination thereof. The precise dosage will vary according to a variety of factors such as subject-dependent variables (e.g., age, immune system health, clinical symptoms etc.), the disease or disorder being treated, as well as the route of administration and the pharmacokinetics of the agent being administered.

The subject disclosure is also intended to include all isotopes of atoms occurring in the compounds disclosed herein. Isotopes include those atoms having the same atomic number but different mass numbers. By way of general example and without limitation, isotopes of hydrogen include tritium and deuterium. Isotopes of carbon include ¹³C and ¹⁴C.

It will be noted that any notation of a carbon in structures throughout this application, when used without further notation, is intended to represent all isotopes of carbon, such as ¹²C, ¹³C, or ¹⁴C. Furthermore, any compounds containing ¹³C or ¹⁴C may specifically have the structure of any of the compounds disclosed herein.

It will also be noted that any notation of a hydrogen in structures throughout this application, when used without further notation, is intended to represent all isotopes of hydrogen, such as ¹H, ²H, or ³H. Furthermore, any compounds containing ²H or ³H may specifically have the structure of any of the compounds disclosed herein.

Isotopically-labeled compounds can generally be prepared by conventional techniques known to those skilled in the art using appropriate isotopically-labeled reagents in place of the non-labeled reagents employed.

In embodiments, deuterium-enriched variants of compounds disclosed herein and their use are contemplated and within the scope of the methods and compositions described herein. Deuterium can be incorporated at any position in place of hydrogen (protium) synthetically, selectively or non-selectively, according to synthetic procedures known in the art. For example, deuterium may be incorporated to various positions having an exchangeable proton, such as an amine N-H, via proton-deuterium equilibrium exchange. Alternatively, deuterium may be introduced during the synthesis of a compound through the use of deuterium-enriched reagents. For example, in Formula (1), deuterium may be incorporated in one or more groups attached to the 2-, 4-, 5-, 6-, and/or 7-position of the indole ring system, in the methylene linkers attached at the 3-position, or in the cyclopropyl ring. As a further example, in Formula (2), deuterium may be incorporated in one or more groups attached to the 2-, 4-, 5-, and/or 7-position of the indole ring system, in the methylene linkers attached at the 3-position, or in the R¹⁴ and/or R¹⁵ groups. As a further example, in Formula (3), deuterium may be incorporated in one or more groups attached to the 2-, 4-, 5-, and/or 7-position of the indole ring system, in the methylene linkers attached at the 3-position, or in the R¹⁶ group. As a further example, in Formula (4), deuterium may be incorporated in one or more groups attached to the 2-, 4-, 5-, and/or 7-position of the indole ring system, in the methylene linkers attached at the 3-position, or in the R¹⁷ and/or R¹⁸ groups. As a further example, in Formula (5), deuterium may be incorporated in one or more groups attached to the 2-, 4-, 6-, and/or 7-position of the indole ring system, in the methylene linkers attached at the 3-position, or in the R¹⁹ group. In some embodiments, the level of deuterium at each deuterium-enriched H site of the compound is 0.02% to 100%. In some embodiments, the level of deuterium at each deuterium-enriched H site of the compound is 50%-100%, 70%-100%, 90%-100%, 95%-100%, 96%-100%, 97%-100%, 98%-100%, or 99%-100%.

The compounds disclosed herein may be racemic and/or optically active isomers thereof. In this regard, some of the compounds can have asymmetric carbon atoms, and therefore, can exist either as racemic mixtures or as individual optical isomers (enantiomers). Compounds described herein that contain a chiral center include all possible stereoisomers of the compound, including compositions including the racemic mixture of the two enantiomers, as well as compositions including each enantiomer individually, substantially free of the other enantiomer. Thus, for example, contemplated herein is a composition including the S enantiomer of a compound substantially free of the R enantiomer, or the R enantiomer substantially free of the S enantiomer. If the named compound includes more than one chiral center, the scope of the present disclosure also includes compositions including mixtures of varying proportions between the diastereomers, as well as compositions including one or more diastereomers substantially free of one or more of the other diastereomers. By "substantially free" it is meant that the composition includes less than 25%, 15%, 10%, 8%, 5%, 3%, or less than 1% of the minor enantiomer or diastereomer(s).

### EXAMPLES

Methods for synthesizing, isolating, preparing, and administering various stereoisomers are known in the art. Separation of diastereomers or cis and trans isomers may be achieved by conventional techniques, such as, for example, by fractional crystallization, chromatography or high-performance liquid chromatography (HPLC) of a stereoisomeric mixture of the agent or a suitable salt or derivative thereof. An individual enantiomer of a compound disclosed herein may also be prepared from a corresponding optically pure intermediate or by resolution, such as by HPLC of the corresponding racemate using a suitable chiral support or by fractional crystallization of the diastereomeric salts formed by reaction of the corresponding racemate with a suitable optically active acid or base, as appropriate.

The compounds of the present disclosure may be prepared by techniques well known in organic synthesis and familiar to a practitioner ordinarily skilled in the art. For example, the compounds may be prepared by the synthetic transformations shown below under General Procedures, **Schemes 1-5,** and further described in the specific examples that follow.

### Abbreviations

DCM: Dichloromethane
DMAc: Dimethylacetamide
DMF: Dimethylformamide
DMSO: Dimethylsulfoxide
HLM: Human liver microsomes
HPLC: High-performance liquid chromatography
HRMS: High-resolution mass spectrometry
LC-MS: Liquid Chromatography-mass spectrometry
MAO: Monoamine oxidase
MTBE: Methyl tert-butyl ether
NADPH: Nicotinamide adenine dinucleotide phosphate hydride
NMR: Nuclear magnetic resonance
PBS: Phosphate buffered saline
Pd/C: Palladium on carbon
R.T.: Room temperature/ambient temperature
TEA: Triethylamine
THF: Tetrahydrofuran

### General Procedures

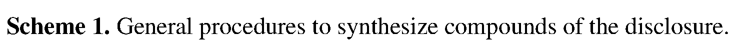

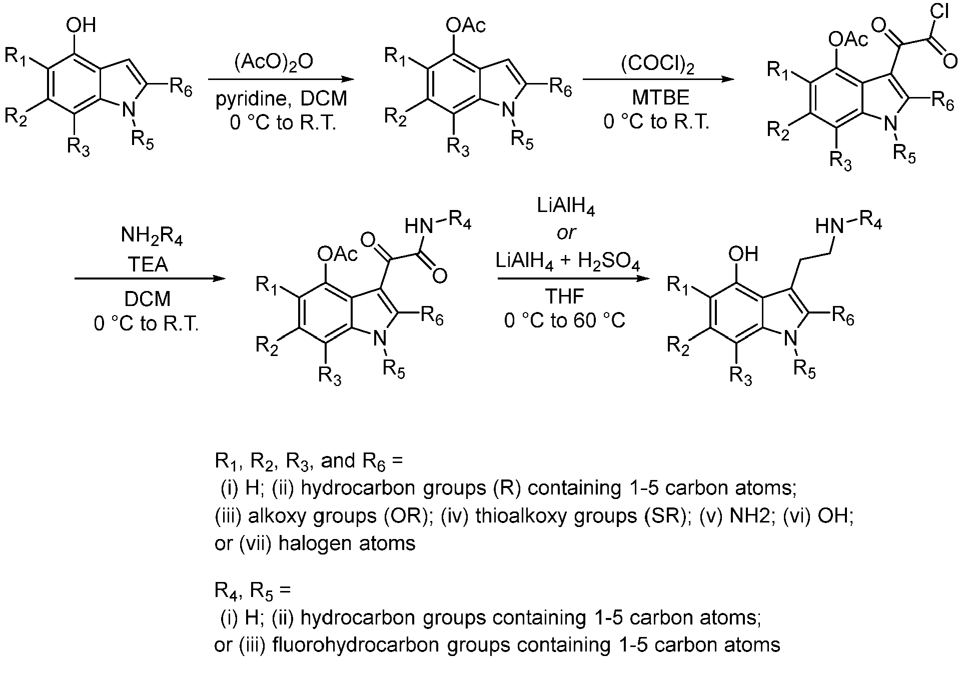

Some compounds may be prepared by alternative methods, such as those generally described in **Schemes 2-5.**

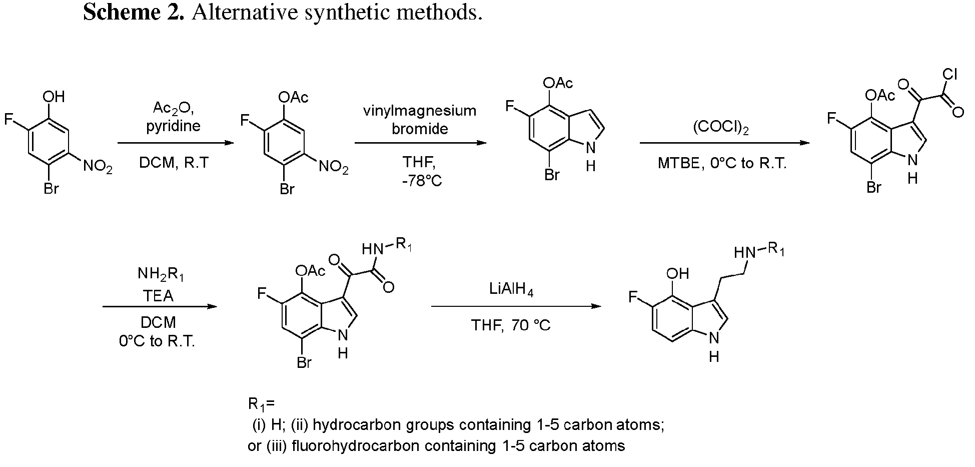

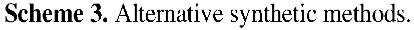

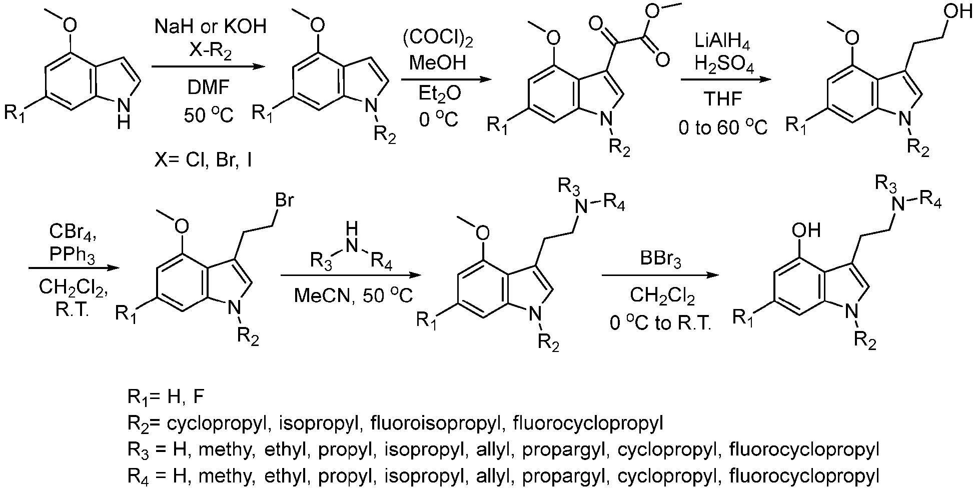

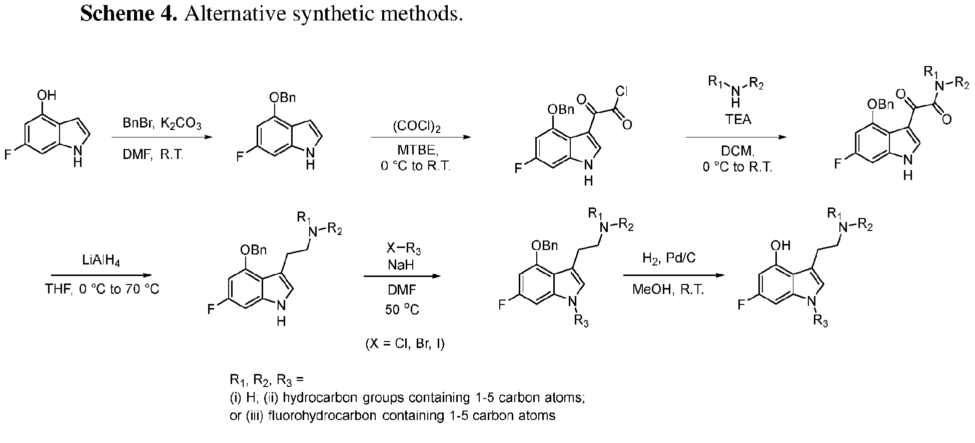

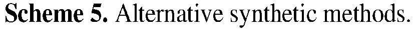

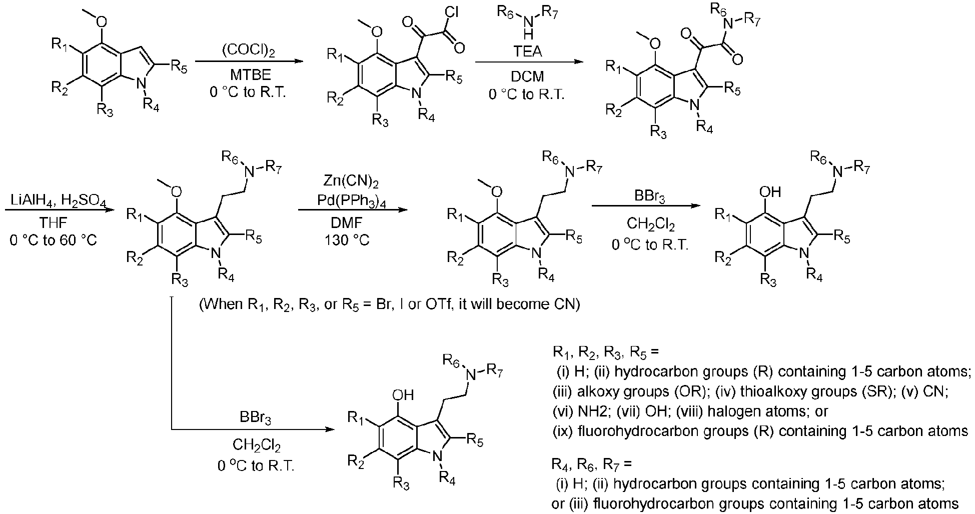

However, these may not be the only means by which to synthesize or obtain the desired compounds.

It should be understood that the examples and embodiments provided herein are exemplary. Those skilled in the art will envision various modifications of the examples and embodiments that are consistent with the scope of the disclosure herein. Such modifications are intended to be encompassed by the claims.

### Specific Examples

### Example 1. Preparation of Compound 1

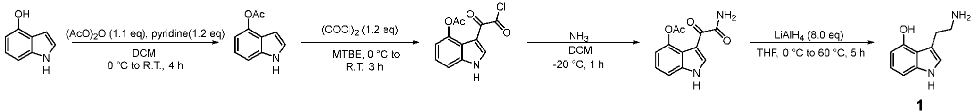

### Step 1: Preparation of 1H-indol-4-yl acetate.

To a stirred solution of 1H-indol-4-ol (100 g, 751.03 mmol, 1.0 eq.) in dry DCM (1000 mL) was added to a reaction vessel under nitrogen atmosphere. Then the reaction was cooled to 0 °C and pyridine was added (72.60 mL, 901.24 mmol, 1.2 eq.) dropwise. Acetic anhydride (102.57 mL, 826.14 mmol, 1.10 eq.) was then added dropwise at 0 °C, and stirred at 0 °C for 10 min. Then reaction was warmed to room temperature and stirred for 4 h. After completion of the reaction (monitored by TLC), the reaction mixture was diluted with water (1,000 mL) and extracted with DCM (2x500 mL). The combined organic layers were washed with brine and dried over anhydrous Na₂SO₄, and concentrated under reduced pressure by rotary evaporator at 45 °C to afford crude compound. The crude compound was stirred in 200 mL diethyl ether for 10 min, then solids were filtered and dried under vacuum to afford 1H-indol-4-yl acetate as an off-white solid (Yield: 100 g, 76%). **¹H NMR (400 MHz, DMSO-d₆):** δ = 11.28 (s, 1H), 7.34-7.28 (m, 2H), 7.08-7.04 (t, 1H), 6.73-6.71 (d, J = 8.0 Hz, 1H), 6.33-6.32 (t, 1H), 2.34 (s, 3H); **HRMS** m/z 176.25 [M+1]⁺.

### Step 2: Preparation of 3-(2-chloro-2-oxoacetyl)-1H-indol-4-yl acetate.

To a stirred solution of 1*H*-indol-4-yl acetate (15.0 g, 85.62 mmol, 1 eq.) in MTBE (300 mL) at 0 °C was added oxalyl chloride (8.81 mL, 102.75 mmol, 1.2 eq.) dropwise. Then the reaction mixture was stirred at 0 °C for 30 min and at room temperature for 3 h, (a yellow precipitate formed during this period). After completion of reaction (evidenced by TLC monitoring), the mixture was diluted with heptane (100 mL), filtered under nitrogen atmosphere, solids were washed with heptane (150 mL), and dried under vacuum, to afford 3-(2-chloro-2-oxoacetyl)-1H-indol-4-yl acetate as a light-yellow solid (Yield: 19 g, 83.53% crude). This crude material was directly used for the next step without further purification or characterization.

### Step 3: Preparation of 3-(2-amino-2-oxoacetyl)-1H-indol-4-yl acetate.

To a stirred solution of 3-(2-chloro-2-oxoacetyl)-1*H*-indol-4-yl acetate crude (6.5 g, 1 eq.) in DCM (130 mL) at -20 °C was sparged with ammonia gas for 10 min (solution color changed from yellow to light-brown), then stirring was continued further 1 h at 0 °C. After completion of the reaction (monitored by TLC), the reaction mass was concentrated under reduced pressure on rotary evaporator at 45 °C to afford crude compound. Crude material was diluted with 50 mL DCM, stirred for 5 min, filtered, solids were washed with 50 mL DCM and dried under vacuum to afford 3-(2-amino-2-oxoacetyl)-1*H*-indol-4-yl acetate as a light-yellow solid (Yield: 3.5 g, 60%). **HRMS** m/z 247.10 [M+1]⁺; **¹H NMR (400 MHz, DMSO-d₆):** δ = 8.56 (s, 1H), 8.01 (s, 1H), 7.66(s, 1H), 7.44-7.42 (d, J = 8.0, 1H), 7.28-7.24 (t, 1 H), 6.88-6.86 (d, J = 8 Hz, 1H),2.36 (s, 3H).

### Step 4: Preparation of 3-(2-aminoethyl)-1H-indol-4-ol (1).

To a stirred solution of 3-(2-amino-2-oxoacetyl)-1H-indol-4-yl acetate (1.5 g, 6.09 mmol, 1 eq.) in dry THF (60 mL) was added LAH solution (2.0M in THF; 24.36 mL, 48.74 mmol, 8 eq.) dropwise at 0 °C. The mixture was stirred at 0 °C for 30 min, then stirred at room temperature for 10 min, then heated at 60 °C for 5h. After completion of the reaction (monitored by TLC), the mixture was cooled to 0 °C, quenched dropwise with a THF/water mixure (1:1), until cessation of effervescence, then diluted with additional THF (100 mL), and stirred at room temperature for 30 min under nitrogen atmosphere. Then reaction mixture was filtered through Celite, washed with THF (100 mL), dried over anhydrous Na₂SO₄, and concentrated under reduced pressure on rotary evaporator at 45 °C in inert atmosphere to afford light-brown sticky solid. Crude compound was purified by preparative HPLC, to afford 3-(2-aminoethyl)-1*H*-indol-4-ol (1) as an off-white solid (Yield: 13 mg, 1.21%). **HRMS** m/z 177.10[M+1]⁺; **¹H NMR (400 MHz, CD₃OD):** *δ =* 6.91 (s, 1H), 6.89-6.81 (m, 2H), 6.35-6.33 (dd, *J =* 8.0, 0.8 Hz, 1H), 3.12-3.09 (m, 4H).

### Example 2. Preparation of Compound 2

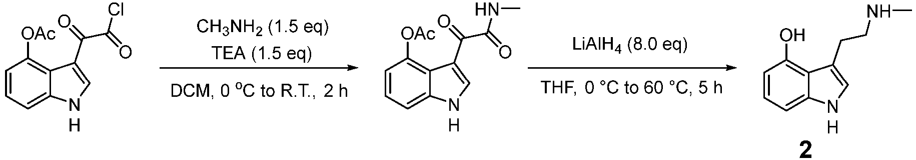

### Step 1: Preparation of 3-(2-(methylamino)-2-oxoacetyl)-1H-indol-4-yl acetate.

To a stirred solution of 3-(2-chloro-2-oxoacetyl)-1H-indol-4-yl acetate (6.5 g, 24.47 mmol, 1 eq.) in DCM (140 mL) at 0 °C, was added methylamine (2M in THF, 18.35 mL, 36.70 mmol, 1.5 eq.). Triethylamine (5.12 mL, 36.70 mmol, 1.5 eq.) was next added dropwise at 0 °C. Stirring was continued for 2 h at room temperature. After completion of the reaction (monitored by TLC), the reaction mixture was diluted with water (200 mL) and extracted with DCM (2x200 mL). The combined organic layers were separated, dried over anhydrous Na₂SO₄, and concentrated under reduced pressure by rotary evaporator at 45 °C. This crude material was suspended with 50 mL DCM, stirred for 5 min, filtered, solids were washed with 50 mL DCM, and dried under reduced pressure to afford 3-(2-(methylamino)-2-oxoacetyl)-1H-indol-4-yl acetate as a light-yellow solid (Yield: 4.50 g, 70.67%). **HRMS** m/z 261.05 [M+1]⁺; **¹H NMR (400 MHz, DMSO-d₆):** δ = 12.42 (s, 1 H), 8.71-8.66 (d, J = 4.4 Hz, 1H), 8.59-8.58 (d, J = 4 Hz, 1H), 7.46-7.44 (d, J= 8.0 Hz, 1 H), 7.29-7.25 (t, 1H), 6.88 (d, J = 8 Hz, 1H), 2.74 (d, J=4.8 Hz 3H), 2.35 (s, 3 H).

### Step 2: Preparation of 3-(2-(methylamino)ethyl)-1H-indol-4-ol (2).

To a stirred solution of crude 3-(2-(methylamino)-2-oxoacetyl)-1H-indol-4-yl acetate (1.5 g, 5.76 mmol, 1 eq.) in dry THF (60 mL) at 0 °C was added LAH solution (2.0 M in THF) (23.05 mL, 46.10 mmol, 8 eq.) dropwise at 0 °C. The reaction mixture was stirred at 0 °C for 30 min, then at room temperature for 10 min, then heated to 60 °C for 5 h. After completion of the reaction (monitored by TLC), the mixture was cooled to 0 °C and quenched with a THF/water mixture (1:1) dropwise until cessation of effervescence. The reaction mixture was then diluted with THF (100 mL) and stirred at room temperature for 30 min under nitrogen atmosphere. This mixture was filtered through Celite, washed with THF (200 mL), dried over anhydrous Na₂SO₄, and concentrated under reduced pressure by rotary evaporator at 45 °C in inert atmosphere to afford light-brown solid. Crude material was purified by trituration. Crude material was first dissolved in 5% methanol in DCM, then diethyl ether was added to produce solids that were filtered and dried to afford 3-(2-(methylamino)ethyl)-1H-indol-4-ol (2) as an off-white solid (Yield: 180.0 mg, 16.42 %). **HRMS** m/z 191.11 [M+1]⁺; **¹H NMR (400 MHz, DMSO-d₆):** δ = 10.55 (s, 1H), 6.897-6.892 (d, J = 2.0 Hz, 1H), 6.81-6.77 (t, 1H), 6.73-6.71 (d, J = 8 Hz, 1H), 6.23-6.21 (d, J = 8 Hz, 1 H), 2.86-2.83 (t, 2H), 2.73 (t, 2H), 2.27 (s, 3H).

### Example 3. Preparation of Compounds 3 and 4

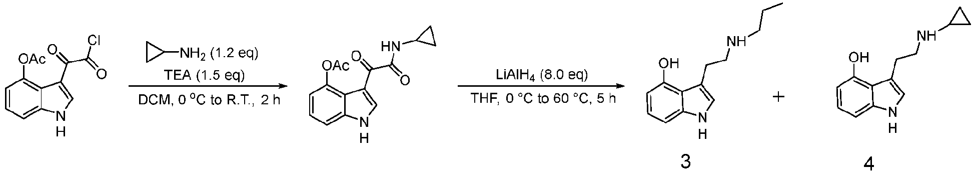

### Step 1: Preparation of 3-(2-(cyclopropylamino)-2-oxoacetyl)-1H-indol-4-yl acetate.

To a stirred solution of crude 3-(2-chloro-2-oxoacetyl)-1H-indol-4-yl acetate (6.0 g, 22.59 mmol, 1 eq.) in DCM (140 mL) at 0 °C, was added cyclopropanamine (1.55 g, 1.2 eq.). Then triethylamine (4.72 mL, 33.88 mmol, 1.5 eq.) was added to the reaction mixture dropwise at 0 °C. The reaction mixture was stirred for 2 h at room temperature. After completion of the reaction (monitored by TLC), the mixture was diluted with water (300 mL) and extracted with DCM (2x200 mL). The combined organic layers were separated, dried over anhydrous Na₂SO₄, and concentrated under reduced pressure by rotary evaporator at 45 °C, to give crude material. This was diluted with 30 mL DCM, stirred for 5 min, filtered, solids were washed with 30 mL DCM, and dried under vacuum to afford 3-(2-(cyclopropylamino)-2-oxoacetyl)-1H-indol-4-yl acetate as a light-yellow solid (Yield: 6.0 g, 85%). **¹H NMR (400 MHz, DMSO-d₆):** δ 12.42 (s, 1H), 8.66 (d, J = 4.8.0 Hz, 1H), 8.57 (s, 1H), 7.44 (d, J = 7.6 Hz ,1H), 7.27 (t, J = 7.6 Hz, 1H), 6.88 (d, J= 7.6 Hz, 1H), 2.85-2.79 (m, 1H), 2.35 (s, 3H), 0.71-0.66 (m, 2H), 0.65-0.62 (m, 2H).

### Step 2: Preparation of 3-(2-(propylamino)ethyl)-1H-indol-4-ol (3) and 3-(2-(cyclopropylamino) ethyl)-1H-indol-4-ol (4).

To a stirred solution of crude 3-(2-(cyclopropylamino)-2-oxoacetyl)-1H-indol-4-yl acetate (1.5 g, 5.24 mmol, 1 eq.) in dry THF (60 mL) at 0 °C, was added an LAH solution (2.0 M in THF; 20.9 mL, 41.91 mmol, 8 eq.) dropwise. This mixture was stirred at 0 °C for 30 min. The reaction mixture was then stirred at room temperature for 10 min, then heated to 60 °C for 5 h. After completion of reaction (monitored by TLC), the mixture was cooled at 0 °C and quenched with a THF/water mixture (1:1), until cessation of effervescence. This mixture was diluted with THF (100 mL) and stirred at room temperature for 30 min under nitrogen atmosphere. The resulting mixture was filtered through Celite, washed with THF (200 mL), dried over anhydrous Na₂SO₄, and concentrated under reduced pressure by rotary evaporator at 45 °C in inert atmosphere to afford a light-brown sticky solid. This material was purified by column chromatography using silica gel (100-200 mesh size) and the compound was eluted in a 7-9 % MeOH gradient in DCM, and desired fractions were concentrated under reduced pressure to afford 3-(2-(propylamino)ethyl)-1H-indol-4-ol **(3)** as an off-white solid (Yield: 200 mg, 17.49%) and 3-(2-(cyclopropylamino) ethyl)-1H-indol-4-ol **(4)** as an off-white solid (Yield: 105 mg, 9 %).

**Compound 3: HRMS** m/z 219.20 [M+1]⁺; **¹H NMR (400 MHz, DMSO-d₆):** δ = 10.55 (s, 1H), 6.89 (d, J = 2.0 Hz, 1H), 6.79 (t, J = 7.6 Hz, 1H), 6.72 (d, J = 8.0 Hz, 1H), 6.22 (d, J = 7.2 Hz, 1H), 2.85 (t, J = 5.2 Hz, 2H), 2.76 (t, J = 5.6 Hz, 2H), 2.50-2.45 (m, 2H), 1.48-1.39 (m, 2H), 0.85 (t, J = 7.6 Hz, 3H) ppm.

**Compound 4: HRMS** m/z 217.15 [M+1]⁺; **¹H NMR (400 MHz, DMSO-d₆):** δ 10.56 (s, 2H), 6.89-6.89 (d, J=2Hz, 1H), 6.80-6.72 (m, 2H), 6.25-6.23 (d, J=8Hz, 1H), 2.86 (s, 4H), 2.13-2.08 (m,1H), 0.38-0.33 (m, 2H), 0.31-0.24 (m, 2H).

### Example 4. Preparation of Compound 5

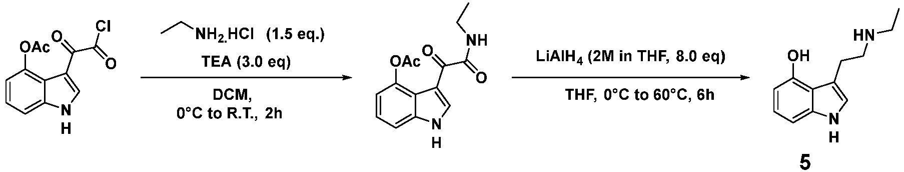

### Step 1: Preparation of 3-(2-(ethylamino)-2-oxoacetyl)-1H-indol-4-yl acetate.

To a stirred solution of crude 3-(2-chloro-2-oxoacetyl)-1H-indol-4-yl acetate (2.5 g, 9.41 mmol, 1 eq.) in DCM (40 mL) at 0 °C, was added ethylamine hydrochloride (1.15 g, 14.12 mmol, 1.5 eq.), followed by triethylamine (3.97 mL, 28.23 mmol, 3 eq.) added dropwise at 0 °C. The reaction mixture was stirred at room temperature for 2 h. After completion of the reaction (monitored by TLC), the reaction mixture was diluted with water (100 mL) and extracted with DCM (2x100 mL). The combined organic layers were pooled, dried over anhydrous Na₂SO₄, and concentrated under reduced pressure by rotary evaporator at 45 °C. Crude material was suspended in 15 mL DCM, stirred for 5 min, filtered, solids were washed with 10 mL DCM, and dried under reduced pressure to afford 3-(2-(ethylamino)-2-oxoacetyl)-1*H*-indol-4-yl acetate as a light-yellow solid (Yield: 1.5 g, 58.11%). **HRMS** m/z 273.0 [M+1]⁺; **¹H NMR (400 MHz, DMSO-d₆):** δ = 12.4 (s, 1 H), 8.63 (s, 2H), 7.43 (d, J = 8 Hz, 1H), 7.27 (t, J= 8.0 Hz, 1 H), 6.88 (d, J=8.0 Hz, 1H), 3.31-3.16 (m, 2H), 2.36 (s, 3H), 1.10 (t, J=8.0 Hz, 3 H).

### Step 2: Preparation of 3-(2-(ethylamino)ethyl)-1H-indol-4-ol (5).

To a stirred solution of 3-(2-(ethylamino)-2-oxoacetyl)-1*H*-indol-4-yl acetate (1.5 g, 5.47 mmol, 1 eq.) in dry THF (25 mL) at 0 °C, was added an LAH solution (2.0 M in THF, 21.9 mL, 43.8 mmol, 8 eq.) dropwise at 0 °C. The reaction mixture was stirred at 0 °C for 30 min, then stirred at room temperature for 10 min, then heated at 60 °C for 6 h. After completion of the reaction, it was cooled to 0 °C, quenched with a THF/water mixture (1:1) until cessation of effervescence, then diluted with additional THF (100 ml), and stirred at room temperature for 30 min under nitrogen atmosphere. The resulting mixture was filtered through Celite, washed with THF (200 mL), dried over anhydrous Na₂SO₄, and concentrated under reduced pressure by rotary evaporator at 45 °C in inert atmosphere to afford a light-brown solid. This crude material was purified by trituration. Crude material was first dissolved in 5% methanol in DCM, then diethyl ether was added to produce solids that were filtered and dried to afford 3-(2-(ethylamino)ethyl)-1*H*-indol-4-ol (5) as an off-white solid (Yield: 114.0 mg, 10.20 %). **HRMS** m/z 205.15 [M+1]⁺; **¹H NMR (400 MHz, DMSO-d₆):** δ = 10.56 (s, 1H), 6.897 (d, J = 2.4 Hz, 1H), 6.79 (t, J=7.6 Hz,1H), 6.72 (d, J = 8 Hz, 1H), 6.23 (d, J = 7.2 Hz, 1 H), 2.85 (t, J=5.8 Hz, 2H), 2.77 (t, J=5.6 Hz, 2H), 2.56 (t, J=7.2 Hz, 2H), 1.02 (t, J=7.2 Hz, 3H).

### Example 5. Preparation of Compound 6

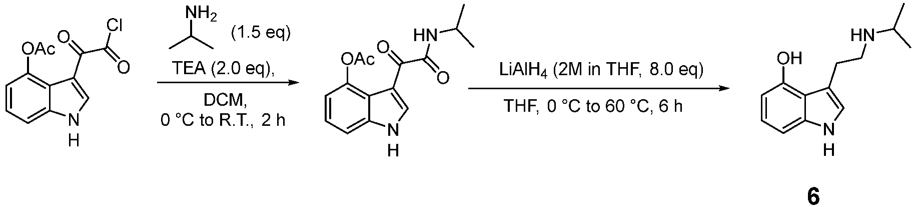

### Step 1: Preparation of 3-(2-(isopropylamino)-2-oxoacetyl)-1H-indol-4-yl acetate.

To a stirred solution of crude 3-(2-chloro-2-oxoacetyl)-1H-indol-4-yl acetate (2.5 g, 9.41 mmol, 1 eq.) in DCM (40 mL) at 0 °C, was added propan-2-amine (1.21 mL,14.1 mmol, 1.5 eq.) followed by triethylamine (2.65 mL, 18.8 mmol, 2 eq.) added dropwise at 0 °C. The reaction mixture was stirred at room temperature for 2 h. After completion of the reaction (monitored by TLC), the reaction mixture was diluted with water (100 mL) and extracted with DCM (2x100 mL). The combined organic layers were pooled, dried over anhydrous Na₂SO₄, and concentrated under reduced pressure by rotary evaporator at 45 °C, affording a crude compound that was suspended in 15 mL DCM, stirred for 5 min, filtered, and the solids were washed with 10 mL DCM and dried under reduce pressure to afford 3-(2-(isopropylamino)-2-oxoacetyl)-1H-indol-4-yl acetate as light-yellow solid (Yield: 2 g, 73.79%). **HRMS** m/z 289.10 [M+1] **⁺; ¹H NMR (400 MHz, DMSO-d₆):** δ = 12.25 (s, 1 H), 8.56 (s, 1H), 8.45 (d, J = 8.4 Hz, 1H), 7.47 (d, J= 12.0 Hz, 1H), 7.25 (t, J= 6.6 Hz, 1H), 6.88 (d, J = 8 Hz, 1H), 4.05-3.97 (m, 1H), 2.53-2.50 (m, 2H), 2.50-2.42 (m, 3H), 1.22-1.13 (m, 6H), 0.95-0.92(m, 3H).

### Step 2: Preparation of 3-(2-(isopropylamino)ethyl)-1H-indol-4-ol (6).

To a stirred solution of 3-(2-(isopropylamino)-2-oxoacetyl)-1H-indol-4-yl acetate (2 g, 6.94 mmol, 1 eq.) in dry THF (30 mL) at 0 °C, was added LAH solution (2 M in THF, 27.7 mL, 55.5 mmol, 8.0 eq.) dropwise at 0 °C. The reaction mixture was stirred at 0 °C for 30 min, then stirred at room temperature for 10 min, then heated at 60 °C for 6 h. After completion of the reaction, it was cooled to 0 °C, quenched with THF/water mixture (1:1) until cessation of effervescence, then diluted with additional THF (100 ml), and stirred at room temperature for 30 min under nitrogen atmosphere. The resulting mixture was filtered through Celite, washed with THF (200 mL), dried over anhydrous Na₂SO₄, and concentrated under reduced pressure by rotary evaporator at 45 °C in inert atmosphere to afford a light-brown solid. This crude material was purified by trituration. Crude material was first dissolved in 5% methanol in DCM, then diethyl ether was added to produce solids that were filtered and dried to afford 3-(2-(isopropylamino)ethyl)-1H-indol-4-ol (6) as a light-grey solid (Yield: 183.0 mg, 12.09 %). **HRMS** m/z 219.20 [M+1] ⁺; **¹H NMR (400 MHz, DMSO-d₆):** δ = 10.55 (s, 1H), 6.89 (d, J = 2.0 Hz, 1H), 6.79 (t, J=7.8 Hz 1H), 6.72 (d, J = 4 Hz, 1H), 6.23 (d, J = 8 Hz, 1 H), 2.84 (t, J=5.6Hz, 2H), 2.73 (t, J=6.8 Hz, 2H), 2.72-2.66 (m, 1H), 0.997-0.982 (d, 6H).

### Example 6. Preparation of Compound 26

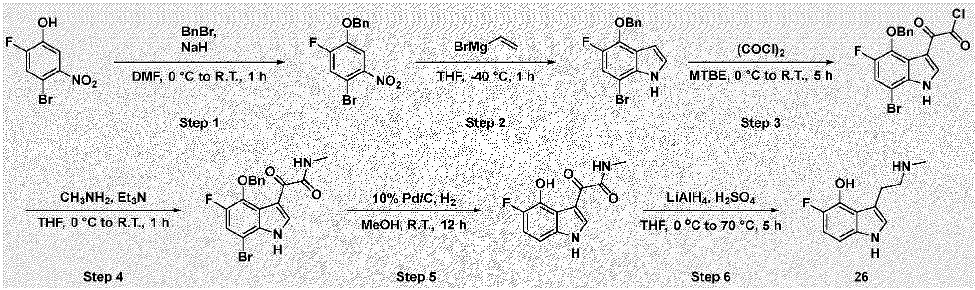

### Step 1: Preparation of 1-(benzyloxy)-4-bromo-2-fluoro-5-nitrobenzene.

To a stirred solution of 4-bromo-2-fluoro-5-nitrophenol (5 g, 21.19 mmol) in anhydrous DMF (50 mL) was added NaH (1.02 g, 25.42 mmol) portionwise at 0 °C under a N₂ atmosphere and the mixture was stirred for 15 min. Benzyl bromide (3.7 mL, 31.78 mmol) was added dropwise via an addition funnel over 10 min to the reaction mixture at 0 °C, and the reaction mixture was stirred for an additional 45 min at the same temperature. Reaction progress was monitored by TLC and LC-MS. After completion, the reaction mixture was quenched with ice-cold water (15 mL) and extracted with EtOAc (2 x 75 mL). The combined organic layers were washed with brine (50 mL), dried over anhydrous Na₂SO₄, and concentrated under reduced pressure to afford crude product. The residue was purified by silica gel chromatography, eluting with 0-5% EtOAc in hexanes, to afford 1-(benzyloxy)-4-bromo-2-fluoro-5-nitrobenzene as a white solid (Yield: 5.5 g, 80 %). **¹H NMR (400 MHz, DMSO-d₆):** *δ* = 8.07-8.05 (d, *J* = 7.6 Hz 1H), 7.98-7.95 (d, *J =*10.4 Hz 1H), 7.50-7.46 (d, *J* = 8.4 Hz 2H), 7.44-7.35 (m, 3H), 5.27 (s, 2H) ppm.

### Step 2: Preparation of 4-(benzyloxy)-7-bromo-5-fluoro-1H-indole.

To a stirred solution of 1-(benzyloxy)-4-bromo-2-fluoro-5-nitrobenzene (5.5 g, 16.86 mmol) in anhydrous THF (50 mL), was added vinyl magnesium bromide (2.0 M in THF; 25.30 mL, 50.59 mmol) at -40 °C and the resulting mixture was stirred for an additional 60 min at the same temperature. Reaction progress was monitored by TLC and LC-MS. After completion of the reaction, the mixture was quenched by dropwise addition of saturated ammonium chloride solution (15 mL), then the reaction mixture was diluted with water (25 ml) and extracted with ethyl acetate (2 x 50 mL). The combined organic layers were washed with brine (50 mL), dried over anhydrous Na₂SO₄, and concentrated under reduced pressure to afford crude product. Purification by silica gel chromatography, eluting with 0-20% EtOAc in hexanes, afforded 4-(benzyloxy)-7-bromo-5-fluoro-1H-indole as an orange liquid (Yield: 2.2 g, 40.74%). **HRMS** m/z 320.10 [M+1]⁺; **¹H NMR (400 MHz, DMSO-d₆): *δ*** = 11.41 (s, 1H), 7.47-7.45 (d, *J =* 7.6 Hz, 2H), 7.40-7.28 (m, 5H), 6.67 (s, 1H), 5.27 (s, 2H) ppm.

### Step 3: Preparation of 2-(4-(benzyloxy)-7-bromo-5-fluoro-1H-indol-3-yl)-2-oxoacetyl chloride.

To a stirred solution of 4-(benzyloxy)-7-bromo-5-fluoro-1*H*-indole (1.0 g, 3.12 mmol) in MTBE (20 mL) at 0 °C was added (COCl)₂ (1.34 mL, 15.62 mmol) dropwise over a period of 5 min. The reaction mixture was stirred at 0 °C for 30 min and then allowed to warm to room temperature and stirred for 5 h. The progress of the reaction was monitored by TLC. After completion, the reaction mixture was concentrated and placed under N₂ atmosphere to afford 2-(4-(benzyloxy)-7-bromo-5-fluoro-1*H*-indol-3-yl)-2-oxoacetyl chloride as a light-yellow liquid (Yield: 1.2 g, 93.56%). *Note:* The crude compound was directly used for the next step without further purification.

### Step 4: Preparation of 2-(4-(benzyloxy)-7-bromo-5-fluoro-1H-indol-3-yl)-N-methyl-2-oxoacetamide.

To a stirred solution of 2-(4-(benzyloxy)-7-bromo-5-fluoro-1H-indol-3-yl)-2-oxoacetyl chloride (1.2 g, 2.92 mmol) in anhydrous THF (20 mL) at 0 °C, was added CH₃NH₂ (2M in THF; 2.19 mL, 4.38 mmol). Triethylamine (1.22 mL, 8.76 mmol) was added to the reaction mixture dropwise at 0 °C. The reaction mixture was then stirred for 1 h at room temperature. After completion of the reaction (monitored by TLC), the mixture was diluted with water (20 mL) and extracted with DCM (2 x 20 mL). The combined organic layers were dried over anhydrous Na₂SO₄ and concentrated under reduced pressure to afford the crude compound, which was purified by silica gel chromatography eluting with 0-5% MeOH in DCM to afford 2-(4-(benzyloxy)-7-bromo-5-fluoro-1H-indol-3-yl)-N-methyl-2-oxoacetamide as a yellow solid (Yield: 0.650 g, 54.89%). **HRMS** m/z 406.95 [M+1]⁺; **¹H NMR (400 MHz, DMSO-d₆):** δ = 12.52 (s, 1H), 8.69 (br s, 1H), 8.60 (s, 1H), 7.59-7.56 (d, *J =* 10.8 Hz 1H), 7.54-7.52 (d, *J =* 7.2 Hz 1H), 7.39-7.30 (m, 3H), 5.05 (s, 2H), 2.73-2.72 (d, *J =* 4.83 H) ppm.

### Step 5: Preparation of 2-(5-fluoro-4-hydroxy-1H-indol-3-yl)-N-methyl-2-oxoacetamide.

To a stirred solution of 2-(4-(benzyloxy)-7-bromo-5-fluoro-1H-indol-3-yl)-N-methyl-2-oxoacetamide (1.3 g, 3.21 mmol) in methanol (25 mL) was added 10% Pd/C (0.759 g, 0.642 mmol) at room temperature under N₂ atmosphere. The reaction mixture was degassed with N₂ for 2-3 min and then stirred at room temperature under an H₂ atmosphere (balloon) for 12 h. After completion of reaction (monitored by TLC and LC-MS), the reaction mixture was passed through a Celite pad to remove the catalyst and the pad was washed with 10% MeOH in DCM (2 x 30 mL). The combined organic solvents were concentrated under reduced pressure to afford the crude product, which was triturated using n-pentane (10 mL) and diethyl ether (3 mL) to afford 2-(5-fluoro-4-hydroxy-1H-indol-3-yl)-N-methyl-2-oxoacetamide as a brown sticky solid (Yield: 0.700 g, 92.3%). **HRMS** m/z 237.10 [M+1]⁺.

### Step 6: Preparation of 5-fluoro-3-(2-(methylamino)ethyl)-1H-indol-4-ol (26).

To a stirred solution of LiAlH₄ (2.0M in THF; 11.8 mL, 23.7 mmol) in anhydrous THF (25 mL) was added H₂SO₄ (0.63 mL, 11.85 mmol) dropwise at 0 °C. The reaction mixture was stirred at 0 °C for 30 min, followed by the addition of 2-(5-fluoro-4-hydroxy-1H-indol-3-yl)-N-methyl-2-oxoacetamide (0.70 g, 2.96 mmol) in THF (50 mL) at 0 °C over a period of 10 min. The reaction mixture was then heated to 70 °C for 5 h. Reaction progress was monitored by TLC and LC-MS. Once complete, the reaction mixture was allowed to cool to room temperature and was quenched with THF:water (1:1) at 0 °C, until effervescence stopped. The mixture was then diluted with THF (100 mL), stirred at room temperature for 30 min under N₂ atmosphere, and filtered through Celite. The Celite was washed with THF (200 mL) and the combined organic solvents were dried over anhydrous Na₂SO₄ and concentrated under reduced pressure to afford the crude product, which was purified by silica gel column chromatography using 0-10% MeOH in DCM, to afford 5-fluoro-3-(2-(methylamino)ethyl)-1*H*-indol-4-ol **(26)** as a grey solid (Yield: 0.10 g, 16.2%). **HRMS** m/z 209.20 [M+1]⁺; **¹H NMR (400 MHz, DMSO-d₆):** *δ* = 10.60 (s, 1H), 9.09 (br s, 2H), 6.96 (d, *J =* 2Hz 1H), 6.81-6.76 (m,1H), 6.61-6.58 (dd, *J* = 3.6 Hz, *J =* 8.4 Hz 1H), 2.85-2.81 (m, 2H), 2.77-2.74 (m, 2H), 2.29 (m, 3H) ppm.

### Example 7. Preparation of Compound 7 Hemifumarate

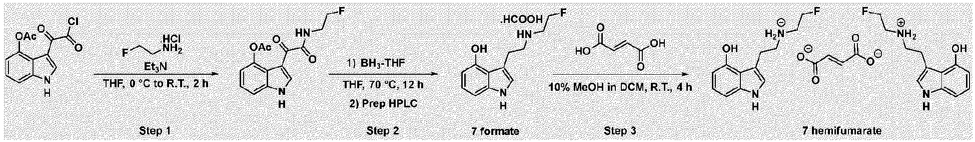

### Step 1: Preparation of 3-(2-((2-fluoroethyl)amino)-2-oxoacetyl)-1H-indol-4-yl acetate.

To a stirred solution of 3-(2-chloro-2-oxoacetyl)-1H-indol-4-yl acetate (0.50 g, 1.8 mmol) in anhydrous THF (10 mL) at 0 °C, was added 2-fluoroethan-1-amine hydrochloride (0.281 mg, 2.82 mmol). Triethylamine (0.79 mL, 5.65 mmol) was added dropwise at 0 °C and the reaction mixture was stirred for 2 h at room temperature. After completion of the reaction (monitored by TLC), the reaction mixture was diluted with water (10 mL) and the aqueous phase was extracted with DCM (2 x 20 mL). The combined organic layers were dried over anhydrous Na₂SO₄ and concentrated under reduced pressure to afford the crude compound, which was purified by silica gel chromatography using 0-5% MeOH in DCM to afford 3-(2-((2-fluoroethyl)amino)-2-oxoacetyl)-1H-indol-4-yl acetate as a light-yellow solid (Yield: 0.50 g, 90.89%). **HRMS** m/z 243.05 [M+1]⁺; **¹H NMR (400 MHz, DMSO-d₆):** δ = 12.46 (s, 1H), 8.85 (t, *J =* 6 Hz, 1H), 8.63 (s, 1H), 7.44 (d, *J =* 8 Hz, 1H), 7.27 (t, *J =* 8 Hz, 1H), 6.89 (d, J = 7.6 Hz, 1H), 4.60 (t, *J = 5.2* Hz, 1H), 4.48 (t, *J =* 5.2 Hz, 1H), 3.56-3.52 (m, 1H), 3.50-3.33 (m, 1H), 2.37 (s, 3H) ppm.

### Step 2: Preparation of 3-(2-((2-fluoroethyl)amino)ethyl)-1H-indol-4-ol (7) formate.

To a stirred solution of 3-(2-((2-fluoroethyl)amino)-2-oxoacetyl)-1H-indol-4-yl acetate (0.300 g, 1.03 mmol) in anhydrous THF (15 mL) at 0 °C was added BH₃-THF solution (2.0M in THF; 7.70 mL, 15.40 mmol) over a period of 10 min followed by heating to 70 °C for 12 h. The reaction progress was monitored by TLC and LC-MS. Once complete, the reaction mixture was allowed to cool to room temperature and then to 0 °C. The reaction was quenched with MeOH (10 mL) until effervescence stopped. The reaction mixture was then diluted with THF (100 mL), stirred at room temperature for 30 min under N₂ atmosphere, and then concentrated under reduced pressure to afford the crude product. The residue was purified by prep HPLC (0.1 % aqueous formic acid in acetonitrile; 2 % to 98 % gradient over 17 min. Flow rate: 18 ml/min. Column: Waters XBridge C8 130Å, 5 µm, 19 mm X 250 mm) to afford 3-(2-((2-fluoroethyl)amino)ethyl)-1H-indol-4-ol (7) formate as a grey solid (Yield: 0.065 g, 28.49 %). **HRMS** m/z 223.20 [M+1]⁺; **¹H NMR (400MHz, DMSO-d₆):** δ = 10.62 (s, 1H), 8.22 (s, 1H), 6.92 (d, *J =* 2Hz, 1H), 6.80 (t, *J =* 8 Hz, 1H), 6.75 (d, *J* = 8 Hz, 1H), 6.28 (d, *J* = 7.2 Hz, 1H), 4.59 (t, J = 5.2 Hz,1H), 4.47 (t, *J = 4.8* Hz, 1H), 4.67 (t, *J = 4.4* Hz, 1H), 4.56 (t, *J =* 4 Hz, 2H), 2.98-2.89 (m, 6H) ppm.

### Step 3: Preparation of 3-(2-((2-fluoroethyl)amino)ethyl)-1H-indol-4-ol (7) hemifumarate.

To a stirred solution of 3-(2-((2-fluoroethyl)amino)ethyl)-1*H*-indol-4-ol (7) formate (0.042 g, 0.188 mmol) in 10% MeOH in DCM (2 mL) was added fumaric acid (0.011 g, 0.094 mmol) at 0 °C over a period of 5 min. The reaction mixture was stirred at room temperature for 4 h. After completion, the reaction mixture was concentrated under reduced pressure to afford a crude compound. The crude residue was triturated by using *n*-pentane (2 mL) and diethyl ether (1 mL) to afford 3-(2-((2-fluoroethyl)amino)ethyl)-1*H*-indol-4-ol **(7)** hemifumarate as a grey solid (Yield: 0.033 g). **HRMS** m/z 223.25 [M+1]⁺; **¹H NMR (400MHz, DMSO-d₆):** *δ* = 10.63 (s, 2H), 9.09 (s, 2H), 6.92 (d, *J =* 2.4 Hz, 2H), 6.82-6.74 (m, 4H), 6.49 (s, 2H), 6.29-6.27 (m, 2H), 4.60 (t, *J =* 4.8Hz, 2H), 4.48 (t, *J =* 4.8 Hz, 2H) 3.01-2.92 (m, 12H) ppm.

### Example 8. Preparation of Compound 27 Hemifumarate

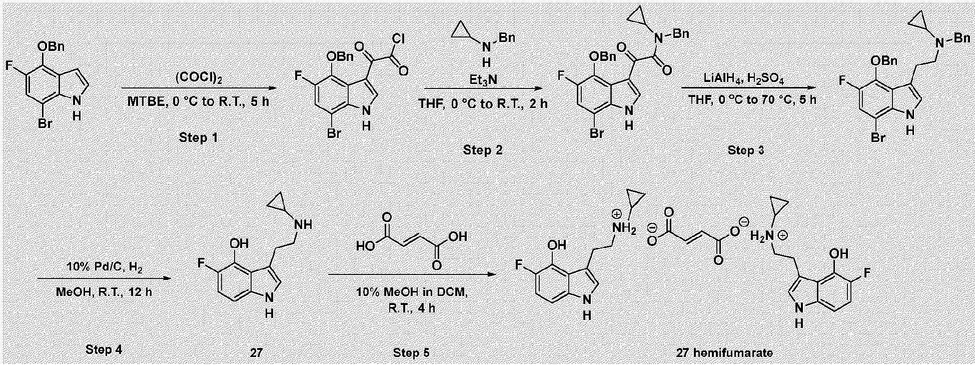

### Step 1: Preparation of 2-(4-(benzyloxy)-7-bromo-5-fluoro-1H-indol-3-yl)-2-oxoacetyl chloride.

To a stirred solution of 4-(benzyloxy)-7-bromo-5-fluoro-1H-indole (1.5 g, 4.69 mmol) in MTBE (15 mL) at 0 °C was added (COCl)₂ (0.6 mL, 7.03 mmol) dropwise over a period of 5 min. The reaction mixture was stirred at 0 °C for 30 min and later allowed to stir 5 h at room temperature. The progress of the reaction was monitored by TLC. Once complete, the solvent was removed under reduced pressure (and backfilled with N₂ atmosphere) to afford 2-(4-(benzyloxy)-7-bromo-5-fluoro-1H-indol-3-yl)-2-oxoacetyl chloride as a light-yellow liquid (Yield: 1.5 g, *77.97%). Note:* This crude compound was directly used for the next step without further purification.

### Step 2: Preparation of N-benzyl-2-(4-(benzyloxy)-7-bromo-5-fluoro-1H-indol-3-yl)-N-cyclopropyl-2-oxoacetamide.

To a stirred solution of 2-(4-(benzyloxy)-7-bromo-5-fluoro-1H-indol-3-yl)-2-oxoacetyl chloride (1.5 g, 3.65 mmol) in anhydrous THF (20 mL) at 0 °C, was added *N-*benzylcyclopropanamine (0.783 mL, 4.38 mmol). Triethylamine (1.53 mL, 10.96 mmol) was then added to the reaction mixture dropwise at 0 °C and the resulting mixture was stirred for 2 h at room temperature. After completion (monitored by TLC), the reaction mixture was diluted with water (20 mL) and extracted with DCM (2 x 20 mL). The combined organic layers were dried over anhydrous Na₂SO₄ and concentrated under reduced pressure to afford the crude product. Purification by silica gel chromatography using 0-5% MeOH in DCM afforded N-benzyl-2-(4-(benzyloxy)-7-bromo-5-fluoro-1H-indol-3-yl)-N-cyclopropyl-2-oxoacetamide as a yellow solid (Yield: 0.900 g, 47.25%). **HRMS** m/z 522.85 [M+2]⁺; **¹H NMR (400MHz, DMSO-d₆):** *δ* = 12.67 (s, 1H), 8.03 (s, 1H), 7.59 (d, *J =* 10.8 Hz, 1H), 7.51 (d, *J =* 7.2 Hz, 2H), 7.41-7.29 (m, 8H), 5.09 (s, 2H), 4.54 (s, 2H), 2.66-2.56 (m, 1H), 0.63 (d, *J =* 4 Hz, 2H), 0.56 (d, *J =* 6.8 Hz, 2H) ppm.

### Step 3: Preparation of N-benzyl-N-(2-(4-(benzyloxy)-7-bromo-5-fluoro-1H-indol-3-yl)ethyl)cyclopropanamine.

To a stirred solution of LiAlH₄ (2.0M in THF; 3.45 mL, 6.90 mmol) in anhydrous THF (4.5 mL) was added H₂SO₄ (0.18 mL, 3.45 mmol) dropwise at 0 °C and the reaction mixture was stirred at 0 °C for 30 min. To the solution was then added N-benzyl-2-(4-(benzyloxy)-7-bromo-5-fluoro-1*H*-indol-3-yl)-N-cyclopropyl-2-oxoacetamide (0.900 g, 1.73 mmol) in THF (9.0 mL) at 0 °C over a period of 10 min and the resulting mixture was heated to 60 °C for 5 h. Reaction progress was monitored by TLC and LC-MS. Once complete, the reaction mixture was allowed to cool to room temperature and then quenched with THF/water (1:1) at 0 °C until effervescence stopped. The quenched reaction mixture was then diluted with THF (100 mL), stirred at room temperature for 30 min under N₂ atmosphere, and then filtered through Celite. The Celite pad was washed with THF (200 mL), and the combined organic solvents were dried over anhydrous Na₂SO₄. The solvent was concentrated under reduced pressure to afford crude product, which was purified by prep HPLC (0.1 % aqueous formic acid in acetonitrile; 2 % to 99 % gradient over 17 min. Flow rate: 18 ml/min. Column: Waters XSelect CSH C18 130Å, 5 µm, 19 mm X 250 mm), to afford N-benzyl-N-(2-(4-(benzyloxy)-7-bromo-5-fluoro-1H-indol-3-yl)ethyl)cyclopropanamine as a colorless sticky solid (Yield: 0.440 g, 51.66%). **HRMS** m/z 494.95 [M+2]⁺; **¹H NMR (400MHz, DMSO-d₆):** *δ* = 11.08 (s, 1H), 7.45 (d, *J =* 7.6 Hz, 2H), 7.38-7.32 (m, 3H), 7.31-7.16 (m, 6H), 7.07 (s, 1H), 5.11 (s, 2H), 3.62 (s, 2H), 2.94-2.88 (m, 2H), 2.72-2.66 (m, 2H), 1.67-1.66 (m, 1H), 0.29 (d, *J =* 6.4 Hz, 2H), 0.18 (s, 2H) ppm.

### Step 4: Preparation of 3-(2-(cyclopropylamino)ethyl)-5-fluoro-1H-indol-4-ol (27).

To a stirred solution of N-benzyl-N-(2-(4-(benzyloxy)-7-bromo-5-fluoro-1*H*-indol-3-yl)ethyl)cyclopropanamine (0.300 g, 0.608 mmol) in methanol (25 mL) was added 10% Pd/C (0.144 g, 0.121 mmol) at room temperature under N₂ atmosphere. The reaction mixture was degassed with N₂ for 2-3 min and then placed under H₂ atmosphere (balloon) and stirred at room temperature for 7 h. After completion (monitored by TLC and LC-MS), the reaction mixture was passed through a Celite pad to remove the catalyst and the pad was washed with 10% MeOH in DCM (2 x 30 mL). The combined organic solvents were removed under reduced pressure to afford a crude product, which was triturated using n-pentane (10 mL) and diethyl ether (3 mL). This afforded 3-(2-(cyclopropylamino)ethyl)-5-fluoro-1H-indol-4-ol **(27)** as a colourless sticky solid (Yield: 0.030 g, 21.06%). **HRMS** m/z 235.17 [M+1]⁺; **¹H NMR (400MHz, DMSO-d₆):** δ = 10.68 (s, 1H), 7.00 (d, *J* = 1.6 Hz, 1H), 6.82 (dd, *J =* 8.8 Hz, 11.2 Hz, 1H), 6.67 (dd, *J =* 3.6 Hz, 8.8 Hz, 1H), 2.96-2.90 (m, 4H), 2.27 (s, 1H), 0.480 (d, *J =* 6 Hz, 2H), 0.42 (br s, 2H) ppm.

### Step 5: Preparation of 3-(2-(cyclopropylamino)ethyl)-5-fluoro-1H-indol-4-ol (27) hemifumarate.

To a stirred solution of 3-(2-(cyclopropylamino)ethyl)-5-fluoro-1*H*-indol-4-ol **(27;** 0.030 g, 0.128 mmol) in 10% MeOH in DCM (2 mL) was added fumaric acid (0.007 g, 0.064 mmol) at 0 °C over a period of 5 min. The reaction mixture was stirred at room temperature for 4 h. The reaction mixture was concentrated under reduced pressure to afford a crude material which was triturated using n-pentane (2 mL) and diethyl ether (1 mL) to afford 3-(2-(cyclopropylamino)ethyl)-5-fluoro-1H-indol-4-ol **(27)** hemifumarate as a grey solid (Yield: 0.020 g). **HRMS** m/z 235 [M+1]⁺; **¹H NMR (400MHz, DMSO-d₆):** δ = 10.70 (s, 2H), 10.00 (br. 2H), 7.01 (s, 2H), 6.85-6.80 (m, 2H), 6.68 (dd, *J* = 2.8 Hz, *J* = 8.4 Hz, 2H), 6.54 (s, 2H), 2.98-2.96 (m, 4H), 2.93-2.92 (m, 4H), 2.30-2.29 (m, 2H), 0.49-0.45 (m, 8H) ppm.

### Example 9. Preparation of Compound 23

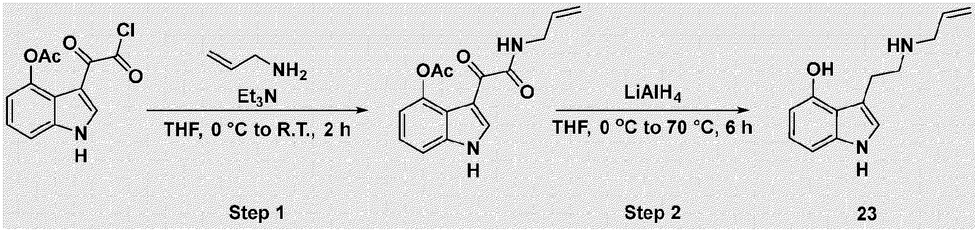

### Step 1: Preparation of 3-(2-(allylamino)-2-oxoacetyl)-1H-indol-4-yl acetate.

To a stirred solution of 3-(2-chloro-2-oxoacetyl)-1*H*-indol-4-yl acetate (see Example 1; 1.2 g, 4.52 mmol) in anhydrous THF (20 mL) at 0 °C, was added prop-2-en-1-amine (0.51 mL, 6.78 mmol). Triethylamine (0.63 mL, 4.52 mmol) was added to the reaction mixture dropwise at 0 °C, followed by stirring for 2 h at room temperature. After completion of the reaction (monitored by TLC), the mixture was diluted with water (20 mL) and extracted with DCM (2 x 20 mL). The combined organic layers were dried over anhydrous Na₂SO₄ and concentrated under reduced pressure to afford a crude residue. Purification by silica gel chromatography using 0-5% MeOH in DCM afforded 3-(2-(allylamino)-2-oxoacetyl)-1H-indol-4-yl acetate as a yellow solid (Yield: 0.900 g, 69.59%). **HRMS** m/z 287.00[M+1]⁺; **¹H NMR (400MHz, DMSO-d₆):** δ = 12.43 (s, 1H), 8.83 (t, *J = 5.6* Hz, 1H), 8.60 (d, *J =* 2.4 Hz, 1H), 7.43 (d, *J = 7.6* Hz, 1H), 7.27 (t, *J =* 8 Hz, 1H), 6.89 (d, J = 7.2 Hz, 1H), 5.90-5.83 (m, 1H) 5.19-5.08 (m, 2H), 3.82 (br, 2H), 2.36 (s, 3H) ppm.

### Step 2: Preparation of 3-(2-(allylamino)ethyl)-1H-indol-4-ol (23).

To a stirred solution of LiAlH₄ (2.0 M in THF; 12.57 mL, 25.15 mmol) in anhydrous THF (25 mL) at 0 °C was added 3-(2-(allylamino)-2-oxoacetyl)-1*H*-indol-4-yl acetate (0.90 g, 3.14 mmol) in THF (50 mL) over a period of 10 min. The mixture was then heated to 70 °C for 6 h. Reaction progress was monitored by TLC and LC-MS. Once complete, the reaction mixture was allowed to cool to room temperature and then cooled to 0 °C. The reaction was quenched with THF/water (1:1) until all effervescence stopped. The reaction mixture was then diluted with THF (100 mL), stirred at room temperature for 30 min under N₂ atmosphere, and then filtered through Celite. The filter pad was washed with THF (200 mL) and the combined organic filtrates were dried over anhydrous Na₂SO₄ and concentrated under reduced pressure to afford a crude product, which was purified by silica gel column chromatography using 0-10% MeOH in DCM, to afford 3-(2-*(allylamino)ethyl)-1H-indol-4-ol* **(23)** as a grey solid (Yield: 0.190 g, 27.94 %). **HRMS** m/z 217.25 [M+1]⁺; **¹H NMR (400MHz, DMSO-d₆):** *δ* = 10.57 (s, 1H), 6.89 (d, *J =* 2Hz, 1H), 6.81-6.77 (m, 1H), 6.73 (d, *J =* 7 Hz, 1H), 6.24 (d, *J =* 6.8 Hz, 1H), 5.87-5.77 (m, 1H), 5.16 (d, *J =* 4Hz, 1H), 5.04 (d, *J =* 8Hz, 1H), 3.15 (d, *J =* 6 Hz, 2H), 2.86 (t, *J =* 6 Hz, 2H), 2.76 (t, *J =* 6 Hz, 2H) ppm.

### Example 10. Preparation of Compound 24

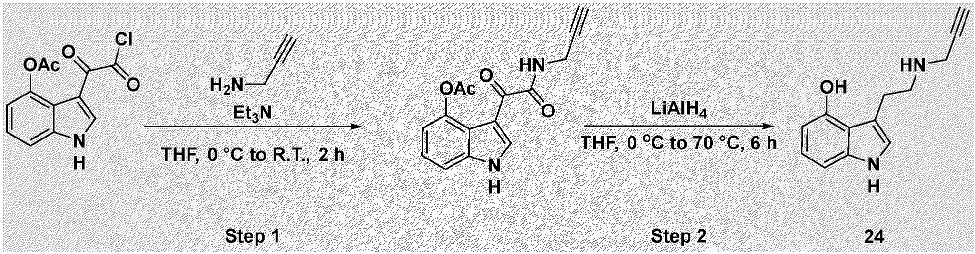

### Step 1: Preparation of 3-(2-oxo-2-(prop-2-yn-1-ylamino)acetyl)-1H-indol-4-yl acetate.

To a stirred solution of 3-(2-chloro-2-oxoacetyl)-1H-indol-4-yl acetate (see Example 1; 2.3 g, 8.66 mmol) in anhydrous THF (20 mL) at 0 °C, was added prop-2-yn-1-amine (0.83 mL, 12.9 mmol). Triethylamine (3.62 mL, 25.97 mmol) was then added to the reaction mixture dropwise at 0 °C and the resulting mixture was stirred for 2 h at room temperature. After completion (monitored by TLC), the mixture was diluted with water (20 mL) and extracted with DCM (2 x 20 mL). The combined organic layers were dried over anhydrous Na₂SO₄ and concentrated under reduced pressure to afford a crude material, which was purified by silica gel chromatography using 0-5% MeOH in DCM to afford 3-(2-oxo-2-(prop-2-yn-1-ylamino)acetyl)-1H-indol-4-yl acetate as a yellow solid (Yield: 1.6 g, 65.01%). **HRMS** m/z 243.05 [M+1]⁺.

### Step 2: Preparation of 3-(2-(prop-2-yn-1-ylamino)ethyl)-1H-indol-4-ol (24).

To a stirred solution of LiAlH₄ (2 M in THF; 22.5 mL, 45.03 mmol) in anhydrous THF (50 mL) was added a solution of 3-(2-oxo-2-(prop-2-yn-1-ylamino)acetyl)-1H-indol-4-yl acetate (1.6 g, 5.63 mmol) in THF (50 mL) dropwise at 0 °C over a period of 10 min. The reaction mixture was then heated to 70 °C for 6 h. Reaction progress was monitored by TLC and LC-MS. Once judged as complete, the reaction mixture was allowed to reach room temperature and then was quenched with THF/water (1:1) at 0 °C, until effervescence stopped. The reaction mixture was diluted with THF (100 mL), stirred at room temperature for 30 min under N₂ atmosphere, filtered through Celite, and the filter cake was washed with THF (200 mL). The combined organic filtrates were dried over anhydrous Na₂SO₄ and concentrated under reduced pressure to afford the crude product, which was purified by silica gel column chromatography using 0-10% MeOH in DCM, to afford 3-(2-(prop-2-yn-1-ylamino)ethyl)-1*H*-indol-4-ol **(24)** as a grey solid (Yield: 0.445 g, 36.90 %). **HRMS** m/z 215.20 [M+1]⁺; **¹H NMR (400MHz, DMSO-d₆):** δ =10.58 (s, 1H), 10.16 (br s, 1H), 6.89 (d, J= 2 Hz, 1H), 6.80-6.72 (m, 2H), 6.25 (d, *J =* 7.2 Hz, 1H), 3.32-3.31 (m, 2H), 3.04 (t, *J =* 2.4 Hz, 1H), 2.89-2.87 (m, 2H), 2.84-2.83 (m, 2H) ppm.

### Example 11. Preparation of Compound 28 Hemifumarate

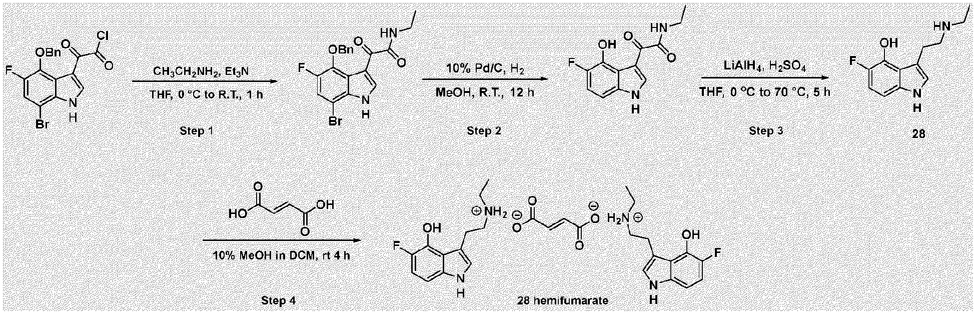

### Step 1: Preparation of 2-(4-(benzyloxy)-7-bromo-5-fluoro-1H-indol-3-yl)-N-ethyl-2-oxoacetamide.

To a stirred solution of 2-(4-(benzyloxy)-7-bromo-5-fluoro-1H-indol-3-yl)-2-oxoacetyl chloride (see Example 6; 1.2 g, 2.92 mmol) in anhydrous THF (20 mL) at 0 °C, was added ethylamine (2M in THF; 2.19 mL, 4.38 mmol). Triethylamine (1.22 mL, 8.77 mmol) was then added to the solution dropwise at 0 °C and the reaction mixture was stirred for 1 h at room temperature. After completion (monitored by TLC), the reaction mixture was diluted with water (20 mL) and extracted with DCM (2 x 20 mL). The combined organic layers were dried over anhydrous Na₂SO₄ and concentrated under reduced pressure to afford a crude residue, which was purified by silica gel chromatography (0-5% MeOH in DCM) to afford 2-(4-(benzyloxy)-7-bromo-5-fluoro-1*H*-indol-3-yl)-N-ethyl-2-oxoacetamide as a yellow liquid (Yield: 0.500 g, 51.98%). **HRMS** m/z 418.8 [M+1]⁺; **¹H NMR (400 MHz, DMSO-d₆):** δ = 12.53 (s, 1H), 8.75 (t, *J =* 5.6 Hz 1H), 8.57 (s, 1H), 7.58 (d, J = 10.4 Hz, 1 H), 7.54-7.52 (m, 2H), 7.47-7.13 (m, 3H), 5.05 (s, 2H), 3.21 (t, *J =* 6.4 Hz, 2H), 3.26-3.19 (m 2H), 1.10 (t, *J =* 8 Hz, 3H) ppm.

### Step 2: Preparation of N-ethyl-2-(5-fluoro-4-hydroxy-1H-indol-3-yl)-2-oxoacetamide.

To a stirred solution of 2-(4-(benzyloxy)-7-bromo-5-fluoro-1H-indol-3-yl)-N-ethyl-2-oxoacetamide (1.8 g, 5.47 mmol) in methanol (25 mL) was added 10% Pd/C (1.3 g, 1.09 mmol) at room temperature under N₂ atmosphere. The reaction mixture was purged with N₂ for 2-3 min and then evacuated and stirred at room temperature under H₂ atmosphere (balloon) for 12 h. After completion (monitored by TLC and LC-MS), the reaction mixture was passed through a Celite pad to remove the catalyst and the filter was washed with 10% MeOH in DCM (2 x 30 mL). The combined organic filtrates were concentrated under reduced pressure to afford a crude residue, which was triturated using n-pentane (10 mL) and diethyl ether (3 mL) to afford N-ethyl-2-(5-fluoro-4-hydroxy-1H-indol-3-yl)-2-oxoacetamide as a brown, sticky solid (Yield: 0.550 g, 51.19%). **HRMS** m/z 251.05 [M+1]⁺; **¹H NMR (400 MHz, DMSO-d₆):** *δ* 12.62 (s, 1H), 11.32 (s,1H), 8.95-8.82 (m, 2H), 7.16-7.11 (m, 1H), 6.94-6.91 (m, 1H) 3.28-3.12 (m, 2H), 1.11 (t, *J =* 7.2 Hz, 3H) ppm.

### Step 3: Preparation of 3-(2-(ethylamino)ethyl)-5-fluoro-1H-indol-4-ol (28).

To a stirred solution of LiAlH₄ (2M in THF; 8.79 mL, 17.58 mmol) in anhydrous THF (20 mL) was added H₂SO₄ (0.47 mL, 8.79 mmol) dropwise at 0 °C. The reaction mixture was stirred at 0 °C for 30 min, and then a solution of N-ethyl-2-(5-fluoro-4-hydroxy-1H-indol-3-yl)-2-oxoacetamide (0.550 g, 2.20 mmol) in THF (20 mL) was added dropwise over a period of 10 min at 0 °C. The reaction mixture was then heated to 70 °C for 5 h. Reaction progress was monitored by TLC and LC-MS. Once complete, the reaction mixture was allowed to cool to room temperature and then cooled to 0 °C and quenched with THF/water (1:1) until effervescence stopped. The quenched reaction mixture was diluted with THF (100 mL), stirred at room temperature for 30 min under N₂ atmosphere, filtered through Celite, and the filter cake was washed with THF (200 mL). The combined organic filtrates were dried over anhydrous Na₂SO₄ and concentrated under reduced pressure to afford a crude product, which was purified by silica gel column chromatography using 0-10% MeOH in DCM to afford 3-(2-(ethylamino)ethyl)-5-fluoro-1*H*-indol-4-ol **(28)** as a grey solid (Yield: 0.057 g, 11.67%). **HRMS** m/z 223.20 [M+1]⁺; **¹H NMR (400 MHz, DMSO-d₆):** δ = 10.59 (s, 1H), 9.07 (br s, 2H), 6.96 (d, *J =* 2 Hz, 1H), 6.81-6.76 (m, 1H), 6.61-6.58 (dd, *J =* 3.6 Hz, *J =* 8.4 Hz 1H), 2.85-2.82 (m, 2H), 2.78-2.76 (m, 2H), 2.58-2.49 (m, 2H), 2.29 (t, *J =* 8 Hz, 3H) ppm.

### Step 4: Preparation of 3-(2-(ethylamino)ethyl)-5-fluoro-1H-indol-4-ol (28) hemifumarate.

To a stirred solution of 3-(2-(ethylamino)ethyl)-5-fluoro-1H-indol-4-ol **(28;** 0.300 g, 1.35 mmol) in 10% MeOH in DCM (5 mL) was added fumaric acid (0.078 g, 0.674 mmol) at 0 °C over a period of 5 min. The reaction mixture was stirred at room temperature for 4 h and then concentrated under reduced pressure to afford a crude residue. This material was triturated using n-pentane (2 mL) and diethyl ether (1 mL) to afford 3-(2-(ethylamino)ethyl)-5-fluoro-1H-indol-4-ol **(28)** hemifumarate as a grey solid (Yield: 0.222 g). **HRMS** m/z 223.20 [M+1]⁺; **¹H NMR (400 MHz, DMSO-d₆):** δ = 10.74 (s, 2H), 9.33 (brss, 2H), 7.03-7.02 (s, 2H), 6.86-6.81 (m, 2H), 6.69-6.54 (m, 2H), 6.43 (s, 2H), 2.99 (s, 8H), 2.85-2.71 (m, 4H), 1.10 (t, *J =* 7.2 Hz, 6H) ppm.

### Example 12. Preparation of Compound 29

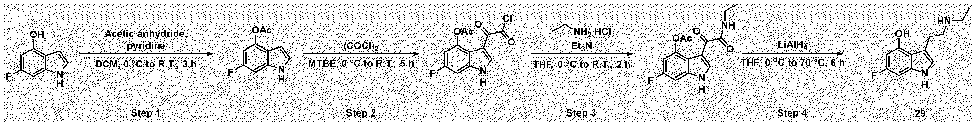

### Step 1: Preparation of 6-fluoro-1H-indol-4-yl acetate.

To a stirred solution of 6-fluoro-1*H*-indol-4-ol (1 g, 6.62 mmol) in anhydrous DCM (15 mL) was added pyridine (0.79 mL, 9.92 mmol) portion-wise at 0 °C under an N₂ atmosphere and the resulting solution was stirred for 15 min. Acetic anhydride (0.98 mL, 7.94 mmol) was then added dropwise over 5 min at 0 °C and the reaction mixture was stirred for an additional 3 h at 0 °C. Reaction progress was monitored by TLC and LC-MS. After completion, the mixture was quenched with ice-cold water (15 mL) and extracted with DCM (2 x 75 mL). The combined organic layers were washed with brine (50 mL), dried over anhydrous Na₂SO₄, filtered, and concentrated under reduced pressure to afford a crude residue. This material was purified by silica gel chromatography, eluting with 0-5% EtOAc in hexane, to afford 6-fluoro-1*H*-indol-4-yl acetate as a white solid **(Yield: 1.1 g, 86.06%). HRMS** m/z 192.05 [M-1]⁻; **¹H NMR (400MHz, DMSO-d₆):** δ = 11.33 (s, 1H), 7.34-33 (m, 1H), 7.10 (d, *J =* 9.6 Hz, 1H), 6.72 (dd, *J =* 2 Hz, *J =* 10.4 Hz, 1H), 6.34 (s, 1H), 2.35 (s, 3H) ppm.

### Step 2: Preparation of 3-(2-chloro-2-oxoacetyl)-6-fluoro-1H-indol-4-yl acetate.

To a stirred solution of 6-fluoro-1*H*-indol-4-yl acetate (1.1 g, 5.69 mmol) in MTBE (15 mL) at 0 °C was added (COCl)₂ (0.58 mL, 6.83 mmol) dropwise over a period of 5 minutes and the reaction mixture was stirred at 0 °C for 30 min. The reaction mixture was then stirred for 5 h at room temperature. The progress of the reaction was monitored by TLC. Once complete, the solvent was removed under reduced pressure (and backfilled with N₂)to afford 3-(2-chloro-2-oxoacetyl)-6-fluoro-1*H*-indol-4-yl acetate as a light-yellow liquid (Yield: 1.2 g, 74.30%) crude. *Note:* The crude compound was directly used in the next step without further purification.

### Step 3: Preparation of 3-(2-(ethylamino)-2-oxoacetyl)-6-fluoro-1H-indol-4-yl acetate.

To a stirred solution of 3-(2-chloro-2-oxoacetyl)-6-fluoro-1*H*-indol-4-yl acetate (1.2 g, 4.23 mmol) in anhydrous THF (20 mL) at 0 °C, was added ethylamine hydrochloride (0.51 g, 6.35 mmol). Triethylamine (1.77 mL, 12.69 mmol) was then added dropwise at 0 °C, and the reaction mixture was stirred for 2 h at room temperature. After completion (monitored by TLC), the reaction mixture was diluted with water (20 mL) and extracted with DCM (2 x 20 mL). The combined organic layers were dried over anhydrous Na₂SO₄ and concentrated under reduced pressure to afford 3-(2-(ethylamino)-2-oxoacetyl)-6-fluoro-1*H*-indol-4-yl acetate (crude) as a yellow solid (Yield: 0.550 g, 44.48%). **HRMS** m/z 291.00[M-1]⁻. *Note:* The crude material was directly used for the next step without further purification.

### Step 4: Preparation of 3-(2-(ethylamino)ethyl)-6-fluoro-1H-indol-4-ol (29).

To a stirred solution of LiAlH₄ (2.0M in THF; 7.53 mL, 15.05 mmol) in anhydrous THF (15 mL) at 0 °C was added a solution of 3-(2-(ethylamino)-2-oxoacetyl)-6-fluoro-1H-indol-4-yl acetate (0.550 g, 1.88 mmol) in THF (50 mL) dropwise over a period of 10 min. The resulting solution was heated to 70°C for 6 h. Reaction progress was monitored by TLC and LC-MS. Once complete, the reaction mixture was allowed to cool to room temperature and then further cooled to 0 °C. The mixture was quenched with THF/water (1:1) until effervescence stopped. The mixture was diluted with THF (100 mL), stirred at room temperature for 30 min under N₂ atmosphere, filtered through Celite, and the Celite pad was washed with THF (200 mL). The combined organic filtrates were dried over anhydrous Na₂SO₄ and concentrated under reduced pressure to afford a crude residue, which was purified by silica gel column chromatography using 0-10% MeOH in DCM, to afford 3-(2-(ethylamino)ethyl)-6-fluoro-1*H*-indol-4-ol **(29)** as a grey solid. (Yield: 0.238 g, 56.90%). **HRMS** m/z 223.20 [M+1]⁺; **¹H NMR (400MHz, DMSO-d₆):** δ = 10.62 (s, 1H), 8.51 (br, 1H), 6.88 (s, 1H), 6.43 (dd, *J =* 2 Hz, *J =* 9.6 Hz, 1H), 6.02 (dd, *J* = 2 Hz, *J =* 12 Hz, 1H), 2.81- 2.79 (m, 2H), 2.76-2.75 (m, 2H), 2.61-2.55 (m, 2H), 1.02 (t, *J =* 7.2 Hz, 3H) ppm.

### Example 13. Preparation of Compound 30 Hemifumarate

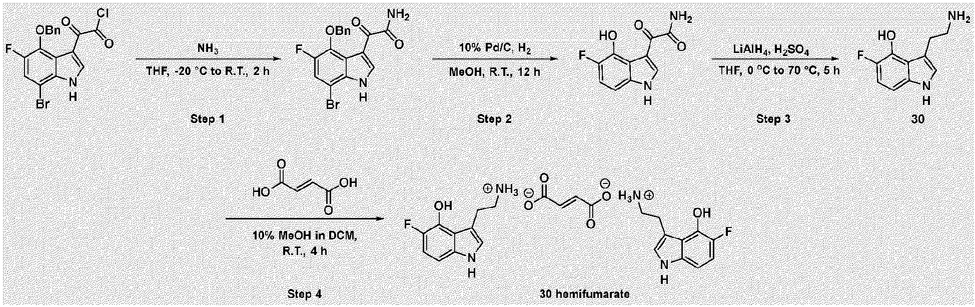

### Step 1: Preparation of 2-(4-(benzyloxy)-7-bromo-5-fluoro-1H-indol-3-yl)-2-oxoacetamide.

To a stirred solution of 2-(4-(benzyloxy)-7-bromo-5-fluoro-1H-indol-3-yl)-2-oxoacetyl chloride (see Example 6; 1.2 g, 2.92 mmol) in THF (20 mL) at -20 °C was added ammonia gas via bubbling for 10 min (excess; color changed from yellow to light-brown solution) and stirring was then continued for 1.5 h at room temperature. After completion (monitored by TLC), the reaction mixture was concentrated under reduced pressure to afford a crude residue, which was purified by silica gel column chromatography using 0-5% MeOH in DCM, to afford 2-(4-(benzyloxy)-7-bromo-5-fluoro-1H-indol-3-yl)-2-oxoacetamide as light brown solid (Yield: 0.770 g, 67.35%). **HRMS** m/z 393.05 [M+1]⁺; **¹H NMR (400 MHz, DMSO-d₆):** δ = 12.53 (s, 1H), 8.49 (s, 1H), 8.14 (s, 1H), 7.74 (s, 1H), 7.59-7.53 (m, 3H), 7.39-7.30 (m, 3H), 5.05 (s, 2H) ppm.

### Step 2: Preparation of 2-(5-fluoro-4-hydroxy-1H-indol-3-yl)-2-oxoacetamide.

To a stirred solution of 2-(4-(benzyloxy)-7-bromo-5-fluoro-1*H`*-indol-3-yl)-2-oxoacetamide (0.770 g, 1.97 mmol) in methanol (25 mL) was added 10% Pd/C (0.466 g, 0.393 mmol) at room temperature under N₂ atmosphere. The reaction mixture was purged with N₂ for 2-3 min and then stirred at room temperature under H₂ atmosphere (balloon) for 12 h. After completion (monitored by TLC and LC-MS), the reaction mixture was passed through a Celite pad to remove the catalyst and the filter cake was washed with 10% MeOH in DCM (2 x 30 mL). The combined organic filtrates were concentrated under reduced pressure to afford the crude product, which was triturated using n-pentane (10 mL) and diethyl ether (3 mL) to afford 2-(5-fluoro-4-hydroxy-1*H*-indol-3-yl)-2-oxoacetamide as a brown, sticky solid (Yield: 0.400 g, 91.47%). **HRMS** m/z 221.00 [M-1]⁻.

### Step 3: Preparation of 3-(2-aminoethyl)-5-fluoro-1H-indol-4-ol (30).

To a stirred solution of LiAlH₄ (2M in THF; 7.20 mL, 14.40 mmol) in anhydrous THF (15 mL) was added H₂SO₄ (0.38 mL, 7.20 mmol) dropwise at 0 °C. The reaction mixture was stirred at 0 °C for 30 min, and then a solution of 2-(5-fluoro-4-hydroxy-1H-indol-3-yl)-2-oxoacetamide (0.400 g, 1.80 mmol) in THF (50 mL) was added over a period of 10 min and the reaction mixture was heated to 70 °C for 5 h. Reaction progress was monitored by TLC and LC-MS. Once complete, the reaction mixture was allowed to cool to room temperature and then quenched with THF/water (1:1) at 0 °C until effervescence stopped. The quenched reaction mixture was diluted with THF (100 mL), stirred at room temperature for 30 min under N₂ atmosphere,filtered through Celite, and the filter cake was washed with THF (200 mL). The combined organic filtrates were dried over anhydrous Na₂SO₄ and concentrated under reduced pressure to afford the crude product, which was purified by silica gel column chromatography using 0-10% MeOH in DCM, to afford 3-(2-aminoethyl)-5-fluoro-1*H*-indol-4-ol **(30)** as a grey solid (Yield: 0.050 g, 14.30 %). **HRMS** m/z 195.09 [M+1]⁺.

### Step 4: Preparation of 3-(2-aminoethyl)-5-fluoro-1H-indol-4-ol (30) hemifumarate.

To a stirred solution of 3-(2-aminoethyl)-5-fluoro-1*H*-indol-4-ol **(30;** 0.050 g, 0.257 mmol) in 10% MeOH in DCM (2 mL) at 0 °C was added fumaric acid (0.015 g, 0.128 mmol) over a period of 5 min. The reaction mixture was stirred at room temperature for 4 h. The mixture was concentrated under reduced pressure to afford a crude residue, which was triturated using n-pentane (2 mL) and diethyl ether (1 mL) to afford 3-(2-aminoethyl)-5-fluoro-1*H*-indol-4-ol **(30)** hemifumarate as a grey solid (Yield: 0.026 g). **HRMS** m/z 195.25 [M+1]⁺; **¹H NMR (400 MHz, DMSO-d₆):** δ = 10.72 (s, 1H), 8.38 (br s, 4H), 7.00 (d, *J =* 2Hz, 2H), 6.85-6.80 (m, 2H), 6.68-6.65 (dd, *J = 3.2* Hz *J* = 8.4 Hz, 2H), 6.36 (s, 2H), 2.93 (s, 8H) ppm.

### Example 14. Preparation of Compound 31 Hemifumarate

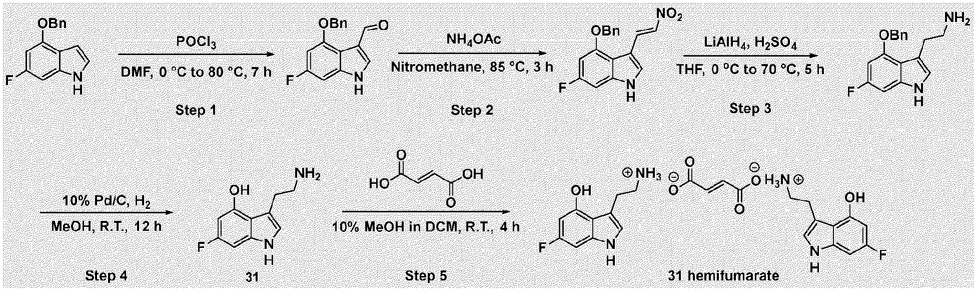

### Step 1: Preparation of 4-(benzyloxy)-6-fluoro-1H-indole-3-carbaldehyde.

Phosphorus oxychloride (2.5 mL, 27.36 mmol) was added dropwise to anhydrous dimethylformamide (20 mL) at 0 °C. The mixture was stirred for 30 minutes and then 4-(benzyloxy)-6-fluoro-1H-indole (2.2 g, 9.12 mmol) was added as a dimethylformamide solution (10 mL per 1 g of indole). The mixture was then allowed to warm to room temperature and stirred for 7 h. The reaction became a heavy suspension that required vigorous stirring. To this suspension, 3.8 M aqueous potassium hydroxide (5.12 g, 91.19 mmol) was added via a dropping funnel and the mixture was heated to reflux for 7 h. It was cooled to room temperature before adding saturated aqueous NaHCO₃ and ethyl acetate (50 mL) until the mixture became clear and the organic layer separated. The aqueous layer was extracted with ethyl acetate (3 x 50 mL) and the combined organic layers were dried over anhydrous Na₂SO₄ and concentrated under reduced pressure to afford crude 4-(benzyloxy)-6-fluoro-1H-indole-3-carbaldehyde as a yellow solid (Yield: 0.900 g, 36.65%). **HRMS** m/z 270.00 [M+1]⁺; **¹H NMR (400MHz, DMSO-d₆):** δ = 12.26 (s, 1H), 10.25 (s, 1H), 8.05 (d, *J =* 2.8 Hz, 1H), 7.53 (d, *J =* 7.2 Hz, 2H), 7.43 (t, *J =* 7.4 Hz, 2H), 7.35 (t, *J =* 7.2 Hz, 1H), 6.92 (dd, *J* = 2 Hz, 9.2 Hz, 1H), 6.84 (dd, *J =* 1.6 Hz, 12 Hz, 1H), 5.29 (s, 2H) ppm.

### Step 2: Preparation of (E)-4-(benzyloxy)-6-fluoro-3-(2-nitrovinyl)-1H-indole.

To a stirred solution of 4-(benzyloxy)-6-fluoro-1H-indole-3-carbaldehyde (0.800 g, 2.97 mmol) in nitromethane (10 mL) was added NH₄OAc (0.630 g, 8.17 mmol) at room temperature under N₂ atmosphere. The reaction mixture was then stirred for 3 h at 85 °C. Reaction progress was monitored by TLC and LC-MS. After completion, the reaction mixture was quenched with ice-cold water (15 mL) and extracted with EtOAc (2 x 75 mL). The combined organic layers were washed with brine (50 mL), dried over anhydrous Na₂SO₄, and concentrated under reduced pressure to afford the crude product. The crude residue was triturated using *n*-pentane (10 mL) and diethyl ether (2 mL) to afford (E)-4-(benzyloxy)-6-fluoro-3-(2-nitrovinyl)-1*H*-indole as a yellow solid (Yield: 0.750 g, 80.83%). **HRMS** m/z 313.00 [M+1]⁺; **¹H NMR (400MHz, DMSO-d₆):** *δ* 12.26 (s, 1H), 8.58 (d, *J* = 13.2 Hz, 1H), 8.25 (s, 1H), 8.03 (d, *J =* 16 Hz, 1H), 7.64-7.55 (m, 2H), 7.51-7.36 (m, 3H), 6.92 (dd, *J =* 2 Hz, 9.2 Hz, 1H), 6.83 (dd, *J =* 1.6 Hz, 11.6 Hz, 1H), 5.27 (s, 2H) ppm.

### Step 3: Preparation of 2-(4-(benzyloxy)-6-fluoro-1H-indol-3-yl)ethan-1-amine.

To a stirred solution of LiAlH₄ (2.0M in THF; 2.56 mL, 5.12 mmol) in anhydrous THF (10 mL) at 0 °C was added H₂SO₄ (0.14 mL, 2.56 mmol) dropwise. The solution was stirred for 30 min and then a solution of (*E*)-4-(benzyloxy)-6-fluoro-3-(2-nitrovinyl)-1*H*-indole (0.400 g, 1.28 mmol) in THF (50 mL) added at 0 °C over a period of 10 min. The reaction mixture was then heated to 70 °C for 5 h. Reaction progress was monitored by TLC and LC-MS. Once complete, the reaction mixture was allowed to cool to room temperature and quenched with THF/water (1:1) while at 0 °C until effervescence stopped. The quenched reaction mixture was diluted with THF (100 mL), stirred at room temperature for 30 min under N₂ atmosphere, filtered through Celite, and the filter cake was washed with THF (200 mL). The combined organic filtrates were dried over anhydrous Na₂SO₄ and concentrated under reduced pressure to afford 2-(4-(benzyloxy)-6-fluoro-1*H*-indol-3-yl)ethan-1-amine (crude) as a brown sticky solid (Yield: 0.240 g, 65.90%). **HRMS** m/z 285.05 [M+1]⁺. *Note:* The crude compound was directly used for the next step without further purification.

### Step 4: Preparation of 3-(2-aminoethyl)-6-fluoro-1H-indol-4-ol (31).

To a stirred solution of 2-(4-(benzyloxy)-6-fluoro-1*H*-indol-3-yl)ethan-1-amine (0.070 g, 0.246 mmol) in methanol (25 mL) was added 10% Pd/C (0.058 g, 0.058 mmol) at room temperature under N₂ atmosphere. The reaction mixture was degassed with N₂ for 2-3 min and then placed under H₂ atmosphere (balloon) and stirred at room temperature for 12 h. After completion of the reaction (monitored by TLC and LC-MS), the mixture was passed through Celite to remove the catalyst and the filter cake was washed with 10% MeOH in DCM (2 x 30 mL). The combined organic filtrates were concentrated under reduced pressure to afford the crude product, which was triturated using n-pentane (10 mL) and diethyl ether (3 mL) to afford 3-(2-aminoethyl)-6-fluoro-1*H*-indol-4-ol (**31**) as a brown solid (Yield: 0.040 g, 83.66%). **HRMS** m/z 195.10 [M+1]⁺; **¹H NMR (400MHz, DMSO-d₆):** *δ* = 10.69 (s, 1H), 8.05 (br. 1H), 6.91 (s, 1H), 6.48 (dd, *J =* 2 Hz, 9.6 Hz, 1H), 6.07 (dd, *J= 1.6* Hz, 12 Hz, 1H), 2.89-2.86 (m, 4H), 1.89 (s, 2H) ppm.

### Step 5: Preparation of 3-(2-aminoethyl)-6-fluoro-1H-indol-4-ol (31) hemifumarate.

To a stirred solution of 3-(2-aminoethyl)-6-fluoro-1H-indol-4-ol (**31;** 0.040 g, 0.205 mmol) in 10% MeOH in DCM (2 mL) at 0 °C was added fumaric acid (0.012 g, 0.103 mmol) over a period of 5 min. The reaction mixture was stirred at room temperature for 4 h. The mixture was concentrated under reduced pressure to afford a crude residue, which was triturated using n-pentane (2 mL) and diethyl ether (1 mL) to afford 3-(2-aminoethyl)-6-fluoro-1*H*-indol-4-ol (**31**) hemifumarate as a grey solid (Yield: 0.030 g). **HRMS** m/z 195.20 [M+1]⁺; **¹H NMR (400MHz, DMSO-d₆):** *δ* = 10.82 (s, 1H), 9.02 (br. 3H), 6.96 (d, *J* = 1.6 Hz, 1H), 6.53 (dd, *J =* 2 Hz, 9.6 Hz, 1H), 6.43 (s, 1H), 6.21 (dd, *J* = 2 Hz, 11.6 Hz, 1H), 3.06-3.01 (m, 4H) ppm.

### Example 15. Preparation of Compound 32 Hemifumarate

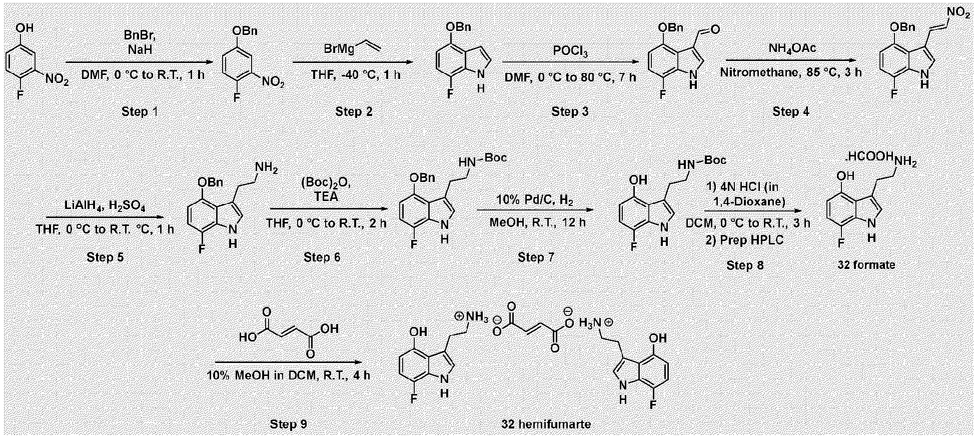

### Step 1: Preparation of 4-(benzyloxy)-1-fluoro-2-nitrobenzene.

To a stirred solution of 4-fluoro-3-nitrophenol (10.00 g, 63.65 mmol) in anhydrous DMF (80 mL) at 0 °C was added NaH (3.06 g, 76.38 mmol) portionwise under an N₂ atmosphere and the mixture was stirred for 15 min. was then added dropwise via an addition funnel over 15 min at 0 °C and the mixture was stirred for an additional 45 min at the same temperature. Reaction progress was monitored by TLC and LC-MS. After completion, the reaction mixture was quenched with ice-cold water (15 mL) and extracted with EtOAc (2 x 75 mL). The combined organic layers were washed with brine (50 mL), dried over anhydrous Na₂SO₄, and concentrated under reduced pressure to afford the crude product. This material was purified via silica gel chromatography, eluting with 0-5% EtOAc in hexane, to afford 4-(benzyloxy)-1-fluoro-2-nitrobenzene as a white solid (Yield: 10 g, 63.55 %). **¹H NMR (400MHz, DMSO-d₆):** *δ* = 7.74-7.72 (m, 1H), 7.56-7.33 (m, 7H), 5.20 (s, 2H) ppm.

### Step 2: Preparation of 4-(benzyloxy)-7-fluoro-1H-indole.

To a stirred solution of 4-(benzyloxy)-1-fluoro-2-nitrobenzene (4.0 g, 16.18 mmol) in anhydrous THF (50 mL) at -40 °C, was added vinyl magnesium bromide (1.0 M in THF; 48.54 mL, 48.54 mmol) and the reaction mixture was stirred for an additional 15 min at the same temperature. Reaction progress was monitored by TLC and LC-MS. After completion, the reaction mixture was quenched by dropwise addition of saturated ammonium chloride solution (15 mL) and the mixture was diluted with water (25 mL) and extracted with ethyl acetate (2 x 50 mL). The combined organic layers were washed with brine (50 mL), dried over anhydrous Na₂SO₄, and concentrated under reduced pressure to afford the crude product. Purification was carried out by silica gel chromatography, eluting with 0-20% EtOAc in hexane, to afford 4-(benzyloxy)-7-fluoro-1*H*-indole as an orange liquid (Yield: 0.650 g, 16.65%). **¹H NMR (400MHz, DMSO-d₆):** *δ* = 11.55 (s, 1H), 7.47 (d, *J =* 7.2 Hz, 2H), 7.38 (t, *J* = 7.2 Hz, 2H), 7.31 (d, *J* = 7.6 Hz, 1H), 7.27 (t, *J =* 2.8 Hz, 1H), 6.76 (dd, *J* = 8.4 Hz, 11.2 Hz, 1H), 6.52-6.51 (m, 1H), 6.44 (dd, *J =* 3.2 Hz, 8.4Hz, 1H), 5.16 (s, 2H) ppm.

### Step 3: Preparation of 4-(benzyloxy)-7-fluoro-1H-indole-3-carbaldehyde.

Phosphorus oxychloride (0.968 mL, 10.36 mmol) was added dropwise to dry dimethylformamide (20 mL) at 0 °C. The mixture was stirred for 30 minutes and then 4-(benzyloxy)-7-fluoro-1*H*-indole (1.0 g, 4.14 mmol) was added as a dimethylformamide solution (10 mL). The mixture was then allowed to warm to room temperature and stirred for 7 h. The reaction became a heavy suspension that required vigorous stirring. At this time, a 3.8 M aqueous potassium hydroxide solution (2.3 g, 41.45 mmol) was added via a dropping funnel and the mixture was heated to reflux for 7 h. The reaction was cooled to room temperature before adding a mixture of saturated aqueous NaHCO₃ and ethyl acetate (30 mL)until the mixture became clear and the organic layer separated. The aqueous layer was extracted with ethyl acetate (3 x 30 mL) and the combined organic layers were dried over anhydrous Na₂SO₄ and concentrated under reduced pressure to afford crude 4-(benzyloxy)-7-fluoro-1*H*-indole-3-carbaldehyde as a yellow solid (Yield: 0.900 g, 80.64%). **HRMS** m/z 270.00 [M+1]⁺; **¹H NMR (400MHz, DMSO-d₆):** *δ* = 12.80 (s, 1H), 10.32 (s, 1H), 8.10 (d, *J =* 2.4 Hz, 1H), 7.52 (d, *J = 7.2* Hz, 2H), 7.42 (t, *J =* 7.2 Hz, 2H), 7.34 (t, *J =* 7.2 Hz, 1H), 6.99 (dd, *J* = 8.4 Hz, 10.4 Hz, 1H), 6.77 (dd, *J =* 3.2 Hz, 8.8 Hz, 1H), 5.26 (s, 2H) ppm.

### Step 4: Preparation of (E)-4-(benzyloxy)-7-fluoro-3-(2-nitrovinyl)-1H-indole.

To a stirred solution of 4-(benzyloxy)-7-fluoro-1*H*-indole-3-carbaldehyde (0.600 g, 2.23 mmol) in nitromethane (10 mL) was added NH₄OAc (0.472 g, 6.13 mmol) at room temperature under N₂ atmosphere. The reaction mixture was then heated and stirred for 3 h at 85 °C. Reaction progress was monitored by TLC and LC-MS. After completion, the reaction mixture was quenched with ice-cold water (15 mL) and extracted with EtOAc (2 x 75 mL). The combined organic layers were washed with brine (50 mL), dried over anhydrous Na₂SO₄, and concentrated under reduced pressure to afford the crude product. This crude residue was triturated using *n*-pentane (10 mL) and diethyl ether (2 mL) to afford (E)-4-(benzyloxy)-7-fluoro-3-(2-nitrovinyl)-1*H*-indole as a yellow solid (Yield: 0.550 g, 79.04%). **HRMS** m/z 312.90 [M+1]⁺. *Note:* The crude compound was directly used for the next step without further purification.

### Step 5: Preparation of 2-(4-(benzyloxy)-7-fluoro-1H-indol-3-yl)ethan-1-amine.

To a stirred solution of LiAlH₄ (2.0 M in THF) (3.52 mL, 7.04 mmol) in anhydrous THF (15 mL) at 0 °C was added H₂SO₄ (0.18 mL, 3.52 mmol) dropwise and the resulting mixture was stirred at 0 °C for 30 min. At this point, (*E*)-4-(benzyloxy)-7-fluoro-3-(2-nitrovinyl)-1*H*-indole (0.550 g, 1.76 mmol) in THF (50 mL) was added to the solution at 0 °C over a period of 10 min and then the reaction was stirred at R.T. for 1 h. Reaction progress was monitored by TLC and LC-MS. Once complete, the reaction mixture was cooled to 0 °C and then quenched with THF/water (1:1) at until the effervescence stopped. The reaction mixture was then diluted with THF (100 mL), stirred at room temperature for 30 min under N₂ atmosphere, filtered through Celite, and the filter cake was washed with THF (200 mL). The combined organic filtrates were dried over anhydrous Na₂SO₄ and concentrated under reduced pressure to afford 2-(4-(benzyloxy)-7-fluoro-1*H*-indol-3-yl)ethan-1-amine (crude) as a brown, sticky solid (Yield: 0.500 g, 99.85 %). **HRMS** m/z 285.05 [M+1]⁺. *Note:* The crude compound was directly used for the next step without further purification.

### Step 6: Preparation of tert-butyl (2-(4-(benzyloxy)-7-fluoro-1H-indol-3-yl)ethyl)carbamate.

To a stirred solution of 2-(4-(benzyloxy)-7-fluoro-1*H*-indol-3-yl)ethan-1-amine (0.500 g, 1.76 mmol) in THF at 0 °C (10 mL) was added Et₃N (0.49 mL, 3.52 mmol) and di-*tert*-butyl dicarbonate (0.60 mL, 2.64 mmol) under inert atmosphere and the mixture was stirred at R.T. for 2 h. Reaction progress was monitored by TLC and LC-MS. After completion, the reaction mixture was quenched by ice-cold water (15 mL) and extracted with EtOAc (2 x 75 mL). The combined organic layers were washed with brine (50 mL) solution, dried over anhydrous Na₂SO₄ and concentrated under reduced pressure to afford crude product, which was purified by silica gel chromatography, eluting with 0-5% EtOAc in hexane, to afford *tert*-butyl (2-(4-(benzyloxy)-7-fluoro-1*H*-indol-3-yl)ethyl)carbamate as a pale yellow solid (Yield: 0.400 g, 59.17 %). **HRMS** m/z 383.10 [M-1]⁻; **¹H NMR (400MHz, DMSO-d₆):** *δ* = 11.26 (s, 1H), 7.49 (d, *J* =7.2 Hz, 2H), 7.39 (t, *J =* 7.2Hz, 2H), 7.33-7.29 (m, 1H), 7.03 (s, 1H), 6.76-6.69 (m, 2H), 6.40 (dd, *J =* 3.2 Hz, 8.4 Hz, 1H), 5.17 (s, 2H), 3.22-3.17 (m, 2H), 2.93 (t, *J* = 7.2 Hz, 2H), 1.35 (s, 9H) ppm.

### Step 7: Preparation of tert-butyl (2-(7-fluoro-4-hydroxy-1H-indol-3-yl)ethyl)carbamate.

To a stirred solution of *tert*-butyl (2-(4-(benzyloxy)-7-fluoro-1*H*-indol-3-yl)ethyl)carbamate (0.400 g, 1.04 mmol) in methanol (25 mL) was added 10% Pd/C (0.246 g, 0.208 mmol) at room temperature under N₂ atmosphere. The reaction mixture was degassed with N₂ for 2-3 min and then placed under H₂ atmosphere (balloon) and stirred at room temperature for 12 h. After completion (monitored by TLC and LC-MS), the reaction mixture was passed through Celite to remove the catalyst and the filter cake was washed with 10% MeOH in DCM (2 x 30 mL). The combined organic filtrates were concentrated under reduced pressure to afford the crude product, which was triturated with *n*-pentane (10 mL) and diethyl ether (3 mL) to afford *tert*-butyl (2-(7-fluoro-4-hydroxy-1*H*-indol-3-yl)ethyl)carbamate as a white solid (Yield: 0.250 g, 81.64%). **HRMS** m/z 293.05 [M-1]⁻.

### Step 8: Preparation of 3-(2-aminoethyl)-7-fluoro-1H-indol-4-ol (32) formate.

To a stirred solution of *tert*-butyl (2-(7-fluoro-4-hydroxy-1*H*-indol-3-yl)ethyl)carbamate (0.250 g, 0.849 mmol) in dichloromethane (2 mL) at 0 °C was added 4 N hydrogen chloride in 1,4-dioxane (3 mL) over a period of 5 min. The reaction mixture was stirred at room temperature for 3 h. After completion, the reaction mixture was concentrated under reduced pressure to afford a crude residue, which was purified by prep HPLC (0.1 % aqueous formic acid in acetonitrile; 2 % to 99 % gradient over 19 min. Flow rate: 18 ml/min. Column: Waters XSelect CSH C18 130Å, 5 µm, 19 mm X 250 mm), to afford 3-(2-aminoethyl)-7-fluoro-1H-indol-4-ol (**32**) formate as an off-white solid (Yield: 0.150 g, 73.51%). **HRMS** m/z 195.12 [M+1]⁺. **¹H NMR (400MHz, DMSO-d₆):** *δ* = 11.17 (s, 1H), 8.38 (s, 1H), 8.01 (br. 1H), 7.04 (d, *J =* 2 Hz, 1H), 6.64 (dd, *J* = 8.4 Hz, 10.8 Hz, 1H), 6.20 (dd, *J* = 3.2 Hz, 8.4 Hz, 1H), 3.04-2.98 (m, 4H) ppm.

### Step 9: Preparation of 3-(2-aminoethyl)-7-fluoro-1H-indol-4-ol (32) hemifumarate.

To a stirred solution of 3-(2-aminoethyl)-7-fluoro-1*H*-indol-4-ol (**32**) formate (0.045 g, 0.231 mmol) in 10% MeOH in DCM (2 mL) at 0 °C was added fumaric acid (0.013 g, 0.116 mmol) over a period of 5 min. The reaction mixture was stirred at room temperature for 4 h. The reaction mixture was concentrated under reduced pressure to afford the crude compound, which was triturated with n-pentane (2 mL) and diethyl ether (1 mL) to afford 3-(2-aminoethyl)-7-fluoro-1*H-*indol-4-ol (32) hemifumarate as a grey solid (Yield: 0.035 g). **HRMS** m/z 195.20 [M+1]⁺; **¹H NMR (400MHz, DMSO-d₆):** *δ* = 11.17 (s, 1H), 8.50 (br. 3H), 7.04 (d, *J =* 1.6 Hz, 1H), 6.66-6.61 (m, 1H), 6.39 (s, 1H), 6.20 (dd, *J* = 3.2 Hz, 8 Hz, 1H), 3.06-3.00 (m, 4H) ppm.

### Example 16. Preparation of Compound 33 Fumarate

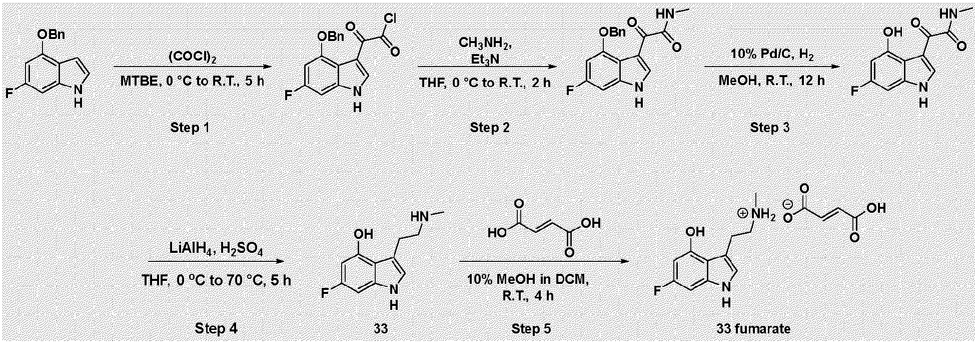

### Step 1: Preparation of 2-(4-(benzyloxy)-6-fluoro-1H-indol-3-yl)-2-oxoacetyl chloride.

To a stirred solution of 4-(benzyloxy)-6-fluoro-1*H*-indole (2 g, 8.29 mmol) in MTBE (15 mL) at 0 °C was added (COCl)₂ (1.07 mL, 12.43 mmol) in a dropwise fashion over a period of 5 min. The reaction mixture was stirred at 0 °C for 30 min and later allowed to stir for 5 h at room temperature. The progress of the reaction was monitored by TLC. Once complete, the solvents were removed under reduced pressure and then backfilled with N₂ atmosphere to afford crude 2-(4-(benzyloxy)-6-fluoro-1*H*-indol-3-yl)-2-oxoacetyl chloride as a pale-yellow liquid (Yield: 1.8 g, 65.46%). *Note:* The crude compound was directly used for the next step without further purification.

### Step 2: Preparation of 2-(4-(benzyloxy)-6-fluoro-1H-indol-3-yl)-N-methyl-2-oxoacetamide.

To a stirred solution of 2-(4-(benzyloxy)-6-fluoro-1*H*-indol-3-yl)-2-oxoacetyl chloride (2 g, 6.03 mmol) in anhydrous THF (20 mL) at 0 °C, was added methylamine (2.0 M in THF; 6.03 mL, 12.06 mmol). Triethylamine (2.52 mL, 18.09 mmol) was then added to the reaction mixture dropwise at 0 °C and the solution was stirred for 2 h at room temperature. After completion (monitored by TLC), the mixture was diluted with water (20 mL) and extracted with DCM (2 x 20 mL). The combined organic layers were dried over anhydrous Na₂SO₄ and concentrated under reduced pressure to afford a crude residue, which was purified by silica gel chromatography using 0-5% MeOH in DCM to afford 2-(4-(benzyloxy)-6-fluoro-1*H*-indol-3-yl)-*N*-methyl-2-oxoacetamide as a yellow solid (Yield: 0.650 g, 33.04%). **HRMS** m/z 327.05 [M+1]⁺; **¹H NMR (400MHz, DMSO-d₆):** *δ* = 12.17 (s, 1H), 8.60 (d, *J =* 4.8 Hz, 1H), 8.34 (d, *J* = 2.8 Hz, 1H), 7.66 (d, *J* = 7.6 Hz, 2H), 7.39 (t, *J =* 7.6 Hz, 2H), 7.30 (t, *J =* 7.2 Hz, 1H) 6.89 (dd, *J =* 2 & 8.8 Hz, 1H), 6.70 (dd, *J* = 1.6 & 12.4 Hz, 1H), 5.25 (s, 2H), 2.67 (d, *J* = 7.6 Hz, 3H) ppm.

### Step 3: Preparation of 2-(6-fluoro-4-hydroxy-1H-indol-3-yl)-N-methyl-2-oxoacetamide.

To a stirred solution of 2-(4-(benzyloxy)-6-fluoro-1*H*-indol-3-yl)-*N*-methyl-2-oxoacetamide (1.2 g, 3.68 mmol) in methanol (25 mL) was added 10% Pd/C (0.871 g, 0.735 mmol) at room temperature under N₂ atmosphere. The reaction mixture was purged with N₂ for 2-3 min and then stirred at room temperature under H₂ atmosphere (balloon) for 12 h. After completion (monitored by TLC and LC-MS), the reaction mixture was passed through a Celite pad to remove the catalyst and the filter cake was washed with 10% MeOH in DCM (2 x 30 mL). The combined organic filtrates were concentrated under reduced pressure to afford a crude residue, which was triturated using n-pentane (10 mL) and diethyl ether (3 mL) to afford 2-(6-fluoro-4-hydroxy-1*H-*indol-3-yl)-*N*-methyl-2-oxoacetamide as a brown, sticky solid (Yield: 0.800 g, 92.10%). **HRMS** m/z 237.05 [M+1]⁺; **¹H NMR (400MHz, DMSO-d₆);** *δ* = 11.41 (s, 1H), 8.88 (d, *J =* 4.8 Hz, 1H), 8.81 (s, 1H), 6.78 (d, *J =* 4.4 Hz, 1H), 6.17 (d, *J =* 4.4 Hz, 1H), 2.75 (d, *J* = 4.8 Hz, 1H) ppm.

### Step 4: Preparation of 6-fluoro-3-(2-(methylamino)ethyl)-1H-indol-4-ol (33).

To a stirred solution of LiAlH₄ (2.0M in THF; 6.77 mL, 13.55 mmol) in anhydrous THF (15 mL) at 0 °C was added H₂SO₄ (0.36 mL, 6.77 mmol) dropwise. The solution was stirred at 0 °C for 30 min, and then 2-(6-fluoro-4-hydroxy-1*H*-indol-3-yl)-*N*-methyl-2-oxoacetamide (0.400 g, 1.69 mmol) in THF (20 mL) was added at 0 °C over a period of 10 min. The reaction mixture was then heated to 70 °C for 5 h. The progress of the reaction was monitored by TLC and LC-MS. Once complete, the reaction mixture was allowed to cool to room temperature and then quenched with THF/water (1:1) at 0 °C, until effervescence stopped. The reaction mixture was then diluted with THF (100 mL), stirred at room temperature for 30 min under N₂ atmosphere, filtered through Celite, and the filter cake was washed with THF (200 mL). The combined organic filtrates were dried over anhydrous Na₂SO₄ and concentrated under reduced pressure to afford a crude product, which was purified by prep HPLC (0.1 % aqueous formic acid in acetonitrile; 3 % to 97 % gradient over 17 min. Flow rate: 18 ml/min. Column: Waters SunFire C18 100Å, 10 µm, 19 mm X 250 mm), to afford 6-fluoro-3-(2-(methylamino)ethyl)-1H-indol-4-ol (33) as a yellow, sticky solid (Yield: 0.028 g, 7.94%). **HRMS** m/z 209.15 [M+1]⁺; **¹H NMR (400MHz, DMSO-d₆):** *δ* = 10.63 (s, 1H), 6.88 (d, *J* = 2 Hz, 1H), 6.44 (dd, *J* = 2.4 & 10 Hz, 1H), 6.02 (dd, *J =* 2 & 12 Hz, 1H), 2.83-2.73 (m, 4H), 2.28 (s, 3H) ppm.

### Step 5: Preparation of 6-fluoro-3-(2-(methylamino)ethyl)-1H-indol-4-ol (33) fumarate.

To a stirred solution of 6-fluoro-3-(2-(methylamino)ethyl)-1*H*-indol-4-ol (**33**; 0.028 g, 0.134 mmol) in 10% MeOH in DCM (2 mL) was added fumaric acid (0.015 g, 0.134 mmol) at 0 °C over a period of 5 min. The mixture was stirred at room temperature for 4 h. The reaction mixture was concentrated under reduced pressure to afford a crude residue, which was triturated with n-pentane (2 mL) and diethyl ether (1 mL) to afford 6-fluoro-3-(2-(methylamino)ethyl)-1*H*-indol-4-ol (**33**) fumarate as a yellow solid. (Yield: 0.036 g). **HRMS** m/z 209.25 [M+1]⁺.**¹H NMR (400MHz, DMSO-d₆):** *δ* = 10.79 (s, 1H), 9.67 (br., 3H), 6.95 (d, *J =* 1.6 Hz, 1H), 6.51 (dd, *J* = 2 & 10 Hz, 1H), 6.42 (s, 2H), 6.16 (dd, *J* = 1.6 & 11.6 Hz, 1H), 3.04-3.00 (m, 4H), 2.47 (s, 3H) ppm.

### Example 17. Preparation of Compound 34 Hemifumarate

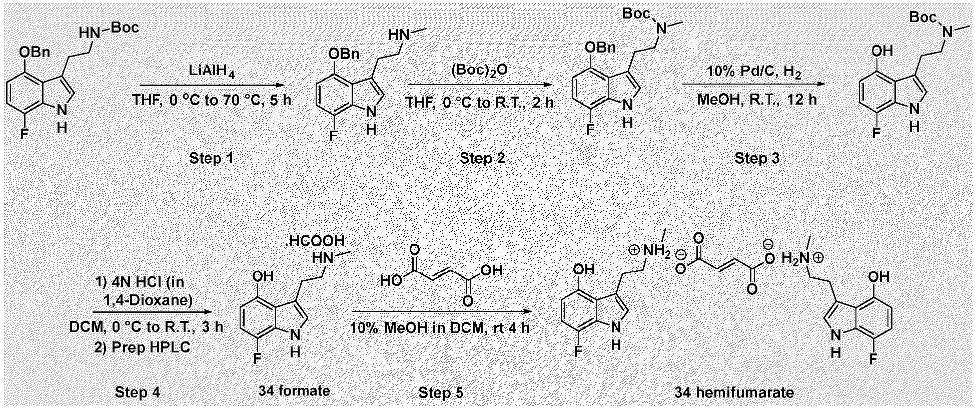

### Step 1: Preparation of 2-(4-(benzyloxy)-7-fluoro-1H-indol-3-yl)-N-methylethan-1-amine.

To a stirred solution of LiAlH₄ (2.0M in THF) (1.17 mL, 2.34 mmol) in anhydrous THF (5 mL) was added *tert*-butyl (2-(4-(benzyloxy)-7-fluoro-1H-indol-3-yl)ethyl)carbamate (see Example 15; 0.300 g, 0.780 mmol) in THF (30 mL) at 0 °C over a period of 5 min and the resulting mixture was stirred at 70 °C for 5 h. Reaction progress was monitored by TLC and LC-MS. Once complete, the reaction mixture was allowed to cool to room temperature, and then quenched with THF/water (1:1) at 0 °C, until effervescence stopped. The quenched reaction mixture was diluted with THF (50 mL), stirred at room temperature for 30 min under N₂ atmosphere, filtered through Celite, and the filter cake was washed with THF (100 mL). The combined organic filtrates were dried over anhydrous Na₂SO₄ and concentrated under reduced pressure to afford 2-(4-(benzyloxy)-7-fluoro-1*H-*indol-3-yl)-*N*-methylethan-1-amine (crude) as a brown, sticky solid (Yield: 0.310 g). **HRMS** m/z 299.00 [M+1]⁺. *Note:* The crude compound was directly used for the next step without further purification.

### Step 2: Preparation of tert-butyl (2-(4-(benzyloxy)-7-fluoro-1H-indol-3-yl)ethyl)(methyl) carbamate.

To a stirred solution of 2-(4-(benzyloxy)-7-fluoro-1*H*-indol-3-yl)-*N*-methylethan-1-amine (0.310 g, 1.04 mmol) in THF (10 mL) at 0 °C was added Boc anhydride (0.35 mL, 1.56 mmol) under inert atmosphere and the resulting mixture was stirred at R.T. for 2 h. Reaction progress was monitored by TLC and LC-MS. After completion, the reaction mixture was quenched with ice cold water (10 mL) and extracted with EtOAc (2 x 15 mL). The combined organic layers were washed with brine (5 mL), dried over anhydrous Na₂SO₄, and concentrated under reduced pressure to afford the crude product, which was purified by silica gel chromatography. Elution with 0-5% EtOAc in hexane afforded *tert*-butyl (2-(4-(benzyloxy)-7-fluoro-1*H*-indol-3-yl)ethyl)(methyl)carbamate as a pale-yellow solid (Yield: 0.160 g, 38.65 %). **HRMS** m/z 397.05 [M-1]⁻; **¹H NMR (400MHz, DMSO-d₆):** *δ* = 11.25 (s, 1H), 7.51 (d, *J* =7.6 Hz, 2H), 7.41 (t, *J =* 7.2 Hz, 2H), 7.34 (t, *J =* 7.6 Hz, 1H), 7.05-6.98 (m, 1H), 6.77 (dd, *J =* 8.4 Hz, 10.8 Hz, 1H), 6.45 (dd, *J =* 3.2 Hz, 8.8 Hz, 1H), 5.15 (s, 2H), 3.38-3.36 (m, 2H), 2.90 (t, *J =* 6.8 Hz, 2H), 2.55-2.45 (m, 3H), 1.36-1.04 (m, 9H) ppm.

### Step 3: Preparation of tert-butyl (2-(7-fluoro-4-hydroxy-1H-indol-3-yl)ethyl)(methyl) carbamate.

To a stirred solution of *tert*-butyl (2-(4-(benzyloxy)-7-fluoro-1*H*-indol-3-yl)ethyl)(methyl)carbamate (0.160 g, 0.401 mmol) in methanol (15 mL) was added 10% Pd/C (0.095 g, 0.08 mmol) at room temperature under N₂ atmosphere. The reaction mixture was degassed with N₂ for 2-3 min and then placed under H₂ atmosphere (balloon) and stirred at room temperature for 24 h. After completion (monitored by TLC and LC-MS), the reaction mixture was passed through a Celite pad to remove the catalyst and the filter cake was washed with 10% MeOH in DCM (2 x 30 mL). The combined organic filtrates were concentrated under reduced pressure to afford the crude product, which was triturated using n-pentane (10 mL) and diethyl ether (3 mL). This afforded *tert-*butyl (2-(7-fluoro-4-hydroxy-1*H*-indol-3-yl)ethyl)(methyl)carbamate as a brown solid (Yield: 0.100 g, 80.77%). **HRMS** m/z 307.05 [M-1]⁻; **¹H NMR (400MHz, DMSO-d₆):** *δ* = 11.02 (s, 1H), 9.33-9.27 (m, 1H), 6.96-6.90 (m, 1H), 6.60 (dd, *J =* 8.4 Hz, 11.2 Hz, 1H), 6.17 (dd, *J* = 3.2 Hz, 8 Hz, 1H), 3.46-3.35 (m, 3H), 2.92 (t, *J*= 6.45 Hz, 2H), 2.74 (s, 2H), 1.38-1.12 (m, 9H) ppm.

### Step 4: Preparation of 7-fluoro-3-(2-(methylamino)ethyl)-1H-indol-4-ol (34) formate.

To a stirred solution of *tert*-butyl (2-(7-fluoro-4-hydroxy-1*H*-indol-3-yl)ethyl)(methyl)carbamate (0.100 g, 0.324 mmol) in dichloromethane (2 mL) was added 4 N hydrogen chloride in 1,4-dioxane (3 mL) at 0 °C over a period of 5 min. The reaction mixture was stirred at room temperature for 3 h. At this time, the reaction mixture was concentrated under reduced pressure and the resulting crude material was purified by prep HPLC (0.1 % aqueous formic acid in acetonitrile; 2 % to 99 % gradient over 17 min. Flow rate: 18 ml/min. Column: Waters XSelect C18 130Å, 5 µm, 19 mm X 250 mm) to afford 7-fluoro-3-(2-(methylamino)ethyl)-1*H*-indol-4-ol (**34**) formate as a brown, sticky solid (Yield: 0.024 g, 35.54%). **¹H NMR (400MHz, DMSO-d₆):** *δ* = 11.15 (s, 1H), 8.56 (s, 1H), 7.03 (s, 1H), 6.63 (dd, *J =* 8.4 Hz, 10.8 Hz, 1H), 6.17 (dd, *J* = 3.2 Hz, 8 Hz, 1H), 2.99 (s, 4H), 2.43 (s, 4H) ppm.

### Step 5: Preparation of 7-fluoro-3-(2-(methylamino)ethyl)-1H-indol-4-ol (34) hemifumarate.

To a stirred solution of 7-fluoro-3-(2-(methylamino)ethyl)-1*H*-indol-4-ol (**34**) formate (0.024 g, 0.115 mmol) in 10% MeOH in DCM (2 mL) was added fumaric acid (0.007 g, 0.057 mmol) at 0 °C over a period of 5 min. The reaction mixture was stirred at room temperature for 4 h. At this time, the reaction mixture was concentrated under reduced pressure to afford a crude material, which was triturated using n-pentane (2 mL) and diethyl ether (1 mL) to afford 7-fluoro-3-(2-(methylamino)ethyl)-1*H*-indol-4-ol (*34*) hemifumarate salt as a grey solid (Yield: 0.023 g). **HRMS** m/z 209.15 [M+1]⁺; **¹H NMR (400MHz, DMSO-d₆):** *δ* = 11.17 (s, 2H), 9.37 (br, 4H), 7.04 (d, *J* = 1.6 Hz, 2H), 6.66-6.61 (m, 2H), 6.39 (s, 2H), 6.20 (dd, *J* = 3.2 Hz, 8 Hz, 2H), 3.00 (s, 8H), 2.44 (s, 6H) ppm.

### Example 18. Preparation of Compound 35 Formate

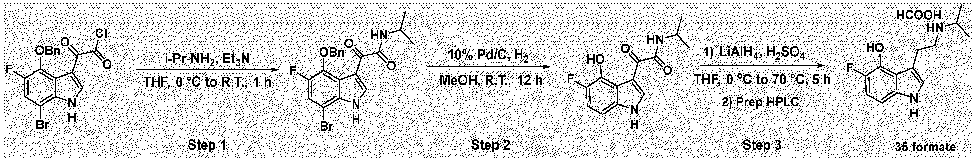

### Step 1: Preparation of 2-(4-(benzyloxy)-7-bromo-5-fluoro-1H-indol-3-yl)-N-isopropyl-2-oxoacetamide.

To a stirred solution of 2-(4-(benzyloxy)-7-bromo-5-fluoro-1*H*-indol-3-yl)-2-oxoacetyl chloride (see Example 6; 1.2 g, 2.92 mmol) in anhydrous THF (20 mL) at 0 °C, was added i-Pr-NH₂ (0.37 mL, 4.38 mmol). Triethylamine (1.22 mL, 8.76 mmol) was then added to the reaction mixture dropwise at 0 °C and it was stirred for 1 h at room temperature. After completion (monitored by TLC), the mixture was diluted with water (20 mL) and extracted with DCM (2 x 20 mL). The combined organic layers were dried over anhydrous Na₂SO₄ and concentrated under reduced pressure to afford a crude residue, which was purified by silica gel chromatography using 0-5% MeOH in DCM to afford 2-(4-(benzyloxy)-7-bromo-5-fluoro-1*H*-indol-3-yl)-*N*-isopropyl-2-oxoacetamide as a yellow liquid (Yield: 0.80 g, 63.18%). **HRMS** m/z 434.95 [M+1]⁺; **¹H NMR (400 MHz, DMSO-d₆):** *δ* = 12.53 (s, 1H), 8.58-8.56 (d, *J =* 8 Hz, 1H), 8.49 (s, 1H), 7.58 (d, *J =* 10.5 Hz, 2H), 7.52 (d, *J =* 6.8 Hz, 2H), 7.39-7.30 (m, 3H), 5.06 (s, 2H), 4.06-3.97 (m, 1H), 1.22-1.00 (s, 6H) ppm.

### Step 2: Preparation of 2-(5-fluoro-4-hydroxy-1H-indol-3-yl)-N-isopropyl-2-oxoacetamide.

To a stirred solution of 2-(4-(benzyloxy)-7-bromo-5-fluoro-1*H*-indol-3-yl)-N-isopropyl-2-oxoacetamide (0.800 g, 1.85 mmol) in methanol (25 mL) was added 10% Pd/C (0.437 g, 0.369 mmol) at room temperature under N₂ atmosphere. The reaction mixture was purged with N₂ for 2-3 min and then stirred at room temperature under H₂ atmosphere (balloon) for 12 h. After completion (monitored by TLC and LC-MS), the reaction mixture was passed through a Celite pad to remove the catalyst and the pad was washed with 10% MeOH in DCM (2 x 30 mL). The combined organic filtrates were concentrated under reduced pressure to afford the crude product, which was triturated using *n*-pentane (10 mL) and diethyl ether (3 mL) to afford 2-(5-fluoro-4-hydroxy-1*H*-indol-3-yl)-*N-*isopropyl-2-oxoacetamide as a brown, sticky solid (Yield: 0.400 g, 81.9%). **HRMS** m/z 265.10 [M+1]⁺.

### Step 3: Preparation of 5-fluoro-3-(2-(isopropylamino)ethyl)-1H-indol-4-ol (35) formate.

To a stirred solution of LiAlH₄ (2 M in THF; 6.05 mL, 12.11 mmol) in anhydrous THF (15 mL) was added H₂SO₄ (0.32 mL, 6.05 mmol) dropwise at 0 °C. The reaction mixture was stirred at 0 °C for 30 min followed by the addition of 2-(5-fluoro-4-hydroxy-1*H*-indol-3-yl)-*N*-isopropyl-2-oxoacetamide (0.400 g, 1.51 mmol) in THF (50 mL) at 0 °C over a period of 10 min. Once the addition was complete, the reaction mixture was heated to 70 °C for 5 h. Reaction progress was monitored by TLC and LC-MS. Once complete, the reaction mixture was allowed to cool to room temperature and then quenched with THF/water (1:1) at 0 °C until effervescence stopped. The quenched reaction mixture was diluted with THF (100 mL), stirred at room temperature for 30 min under N₂ atmosphere, filtered through Celite, and the filter cake was washed with THF (200 mL). The combined organic filtrates were dried over anhydrous Na₂SO₄ and concentrated under reduced pressure to afford the crude product, which was purified by prep HPLC (0.1 % aqueous formic acid in acetonitrile; 2 % to 90 % gradient over 18 min. Flow rate: 18 ml/min. Column: Phenomenex Kinetex Biphneyl, 5 µm, 19 mm X 250 mm), to afford 5-fluoro-3-(2-(isopropylamino)ethyl)-1*H-*indol-4-ol (**35**) formate as a grey solid (Yield: 0.016 g, 4%). **HRMS** m/z 237.25 [M+1]⁺; **¹H NMR** (400 MHz, **DMSO-d₆):** *δ* = 10.69 (s, 1H), 8.29 (s, 1H), 7.01-7.00 (d, *J* =2 Hz, 1H), 6.84-6.80 (m, 1H), 6.66-6.64 (dd, *J* = 3.2 Hz, *J =* 8.4 Hz, 1H), 2.92 (brs, 5H), 1.09 (d, *J =* 6.4 Hz, 6H) ppm.

### Example 19. Preparation of Compound 36 Hemifumarate

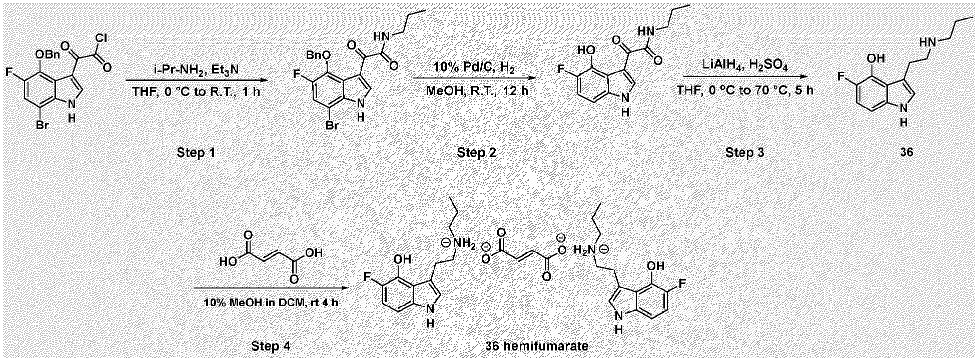

### Step 1: Preparation of 2-(4-(benzyloxy)-7-bromo-5-fluoro-1H-indol-3-yl)-2-oxo-N-propylacetamide.

To a stirred solution of 2-(4-(benzyloxy)-7-bromo-5-fluoro-1*H*-indol-3-yl)-2-oxoacetyl chloride (see Example 6; 1.2 g, 2.92 mmol) in anhydrous THF (20 mL) at 0 °C, was added *n*-Pr-NH₂ (0.36 mL, 4.38 mmol). Triethylamine (1.22 mL, 8.77 mmol) was added to the reaction mixture dropwise while at 0 °C and the reaction mixture was then stirred for 1 h at room temperature. After completion (monitored by TLC), the mixture was diluted with water (20 mL) and the aqueous phase was extracted with DCM (2 x 20 mL). The combined organic layers were dried over anhydrous Na₂SO₄ and concentrated under reduced pressure to afford the crude compound, which was purified by silica gel chromatography using 0-5% MeOH in DCM to afford 2-(4-(benzyloxy)-7-bromo-5-fluoro-1H-indol-3-yl)-2-oxo-*N*-propylacetamide as a yellow solid (Yield: 0.800 g, 63.18%). **HRMS** m/z 433.9 [M+1]⁺; **¹H NMR** (**400 MHz, DMSO-d₆):** *δ* = 12.5 (s, 1H) 8.76 (t, *J =* 5.6 Hz, 1H) 8.55 (s,1H) 7.60-7.54 (m, 3H) 7.40-7.33 (m, 3H) 5.07 (s, 2H) 3.19-3.17 (m, 2H) 1.55-1.53 (m, 2H) 0.77 (t, *J* = 7.6 Hz, 3H) ppm.

### Step 2: Preparation of 2-(5-fluoro-4-hydroxy-1H-indol-3-yl)-2-oxo-N-propylacetamide.

To a stirred solution of 2-(4-(benzyloxy)-7-bromo-5-fluoro-1*H*-indol-3-yl)-2-oxo-*N-*propylacetamide (0.800 g, 2.33 mmol) in methanol (25 mL) was added 10% Pd/C (0.552 g, 0.466 mmol) at room temperature under N₂ atmosphere. The reaction mixture was purged with N₂ for 2-3 min and then stirred at room temperature under H₂ atmosphere (balloon) for 12 h. After completion (monitored by TLC and LC-MS), the reaction mixture was passed through a Celite pad to remove the catalyst and the pad was washed with 10% MeOH in DCM (2 x 30 mL). The combined organic filtrates were concentrated under reduced pressure to afford the crude product, which was triturated using n-pentane (10 mL) and diethyl ether (3 mL) to afford 2-(5-fluoro-4-hydroxy-1*H*-indol-3-yl)-2-oxo-*N*-propylacetamide as a brown, sticky solid (Yield: 0.450 g, 92.23%). **HRMS** m/z 265.10 [M+1]⁺; **¹H NMR** (**400 MHz, DMSO-d₆):** *δ* = 12.59 (s, 1H), 11.29 (s, 1H), 8.90 (s, 1H), 8.80 (d, *J*= 3.6 Hz, 1H), 7.16-7.13 (m, 1H), 7.10-6.95 (m, 1H), 3.21-3.16 (m, 2H), 1.58-1.52 (m, 2H), 0.88 (t, *J* = 7.2 Hz, 3H) ppm.

### Step 3: Preparation of 5-fluoro-3-(2-(propylamino)ethyl)-1H-indol-4-ol (36).

To a stirred solution of LiAlH₄ (2M in THF; 6.81 mL, 13.62 mmol) in anhydrous THF (15 mL) was added H₂SO₄ (0.66 mL, 6.81 mmol) dropwise while at 0 °C and the solution was stirred at 0 °C for 30 min. To this mixture was then added 2-(5-fluoro-4-hydroxy-1*H*-indol-3-yl)-2-oxo-*N-*propylacetamide (0.450 g, 1.70 mmol) in THF (50 mL) at 0 °C over a period of 10 min. The reaction mixture was then heated to 70 °C for 5 h. Reaction progress was monitored by TLC and LC-MS. Once complete, the reaction mixture was allowed to cool to room temperature and then quenched with THF/water (1:1) at 0 °C, until all effervescence stopped. The reaction mixture was diluted with THF (100 mL), stirred at room temperature for 30 min under N₂ atmosphere, filtered through Celite, and the filter cake was washed with THF (200 mL). The combined organic filtrates were dried over anhydrous Na₂SO₄ and concentrated under reduced pressure to afford a crude product, which was purified by silica gel column chromatography using 0-10% MeOH in DCM, to afford 5-fluoro-3-(2-(propylamino)ethyl)-1*H*-indol-4-ol (**36**) as a grey solid (Yield: 0.16 g, 39.76%). **HRMS** m/z 237.20 [M+1]⁺; **¹H NMR** (**400 MHz, DMSO-d₆):** *δ =* 10.59 (s, 1H), 8.88 (br s, 1H), 6.96 (s, 1H), 6.95-6.76 (m,1H) , 6.62-6.59 (m, 1H) , 2.85-2.83 (m, 2H), 2.78-2.76 (m, 2H), 2.47-2.33 (m, 2H), 0.86 (t, *J =* 7.2 Hz, 3H) ppm.

### Step 4: Preparation of 5-fluoro-3-(2-(propylamino)ethyl)-1H-indol-4-ol (36) hemifumarate.

To a stirred solution of 5-fluoro-3-(2-(propylamino)ethyl)-1*H*-indol-4-ol (**36;** 0.100 g, 0.423 mmol) in 10% MeOH and DCM (2 mL) was added fumaric acid (0.024 g, 0.211 mmol) while at 0 °C over a period of 5 min. The reaction mixture was then stirred at room temperature for 4 h. At this time, the mixture was concentrated under reduced pressure to afford a crude residue, which was triturated by using n-pentane (2 mL) and diethyl ether (1 mL) to afford 5-fluoro-3-(2-(propylamino)ethyl)-1H-indol-4-ol (36) hemifumarate salt as a grey solid (Yield: 0.096 g). **HRMS** m/z 237.25 [M+1]⁺; **¹H NMR** (**400 MHz, DMSO-d₆):** *δ* = 10.73 (s, 1H), 9.53 (br s, 4H), 7.03-7.02 (s, 2H), 6.86-6.81 (m, 2H), 6.69-6.66 (m, 2H), 6.43 (s, 2H), 2.98 (s, 8H), 2.70-2.66 (m, 4H), 1.58-1.52 (m, 4H), 0.88 (t, *J =* 7.6 Hz, 6H) ppm.

### Example 20. Preparation of Compound 37 Hemifumarate

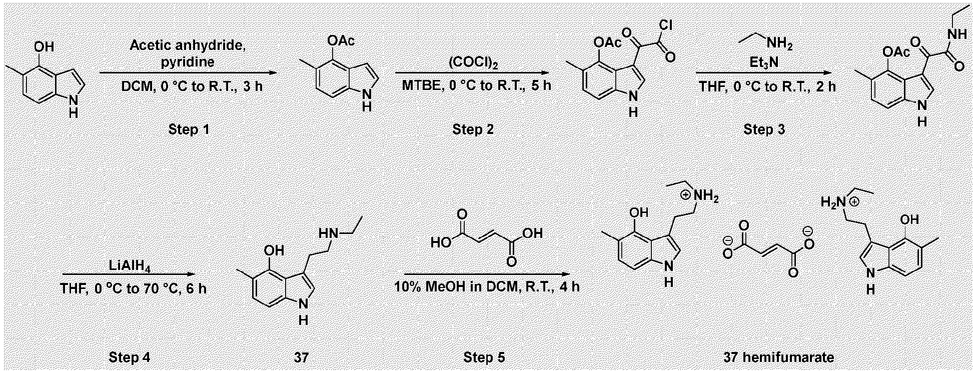

### Step 1: Preparation of 5-methyl-1H-indol-4-yl acetate.

To a stirred solution of 5-methyl-1*H*-indol-4-ol (1.1 g, 7.47 mmol) in anhydrous DCM (15 mL) was added pyridine (0.90 mL, 11.21 mmol) portion-wise at 0 °C under an N₂ atmosphere and the mixture was stirred for 15 min. Acetic anhydride (1.1 mL, 8.97 mmol) was then added dropwise via an additional funnel over 5 min to the above reaction mixture at 0 °C and the resulting mixture was stirred for an additional 3 h at 0 °C. Reaction progress was monitored by TLC and LC-MS. After completion, the reaction mixture was quenched with ice-cold water (15 mL) and extracted with DCM (2 x 75 mL). The combined organic layers were washed with brine (50 mL), dried over anhydrous Na₂SO₄, and concentrated under reduced pressure to afford the crude product, which was purified by silica gel chromatography, eluting with 0-5% EtOAc in hexane, to provide 5-methyl-1*H-*indol-4-yl acetate as a white solid (Yield: 1.1 g, 77.78%). **HRMS** m/z 190.05 [M+1]⁺; **¹H NMR (400MHz, DMSO-d₆):** *δ* = 11.14 (s, 1H), 7.28 (t, *J =* 2.8 Hz, 1H), 7.19 (d, *J*= 8.4 Hz, 1H), 6.94 (d, *J =* 8 Hz, 1H), 6.25 (t, *J =* 2 Hz, 1H), 2.36 (s, 3H), 2.15 (s, 3H) ppm.

### Step 2: Preparation of 3-(2-chloro-2-oxoacetyl)-5-methyl-1H-indol-4-yl acetate.

To a stirred solution of 5-methyl-1*H*-indol-4-yl acetate (0.500 g, 2.64 mmol) in MTBE (15 mL) at 0 °C was added (COCl)₂ (0.33 mL, 3.96 mmol) dropwise over a period of 5 min and the reaction mixture was stirred at 0 °C for 30 min and then at room temperature for 5 h. The progress of the reaction was monitored by TLC and upon completion, the mixture was concentrated under reduced pressure to afford 3-(2-chloro-2-oxoacetyl)-5-methyl-1*H*-indol-4-yl acetate as a pale-yellow liquid (Yield: 0.650 g, 87.95 *%). Note:* The crude compound was directly used for the next step without further purification.

### Step 3: Preparation of 3-(2-(ethylamino)-2-oxoacetyl)-5-methyl-1H-indol-4-yl acetate.

To a stirred solution of 3-(2-chloro-2-oxoacetyl)-5-methyl-1*H*-indol-4-yl acetate (0.650 g, 2.32 mmol) in anhydrous THF (20 mL) at 0 °C was added ethanamine (2 M in THF; 1.74 mL, 3.49 mmol). Et₃N (0.97 mL, 6.97 mmol) was then added to the reaction mixture dropwise at 0 °C and the resulting mixture was stirred for 2 h at room temperature. After completion of the reaction (monitored by TLC), the reaction mass was diluted with water (10 mL) and extracted with DCM (2 x 10 mL). The combined organic layers were dried over anhydrous Na₂SO₄ and concentrated under reduced pressure to afford the crude compound, which was triturated using *n*-pentane (5 mL) and diethyl ether (1 mL) to afford 3-(2-(ethylamino)-2-oxoacetyl)-5-methyl-1*H*-indol-4-yl acetate as a yellow solid (Yield: 0.500 g, 74.62%). **HRMS** m/z 289.00 [M+1]⁺; **¹H NMR (400MHz, DMSO-d₆):** *δ* = 12.30 (s, 1H), 8.63 (t, *J =* 5.6 Hz, 1H), 8.55 (s, 1H), 7.32 (d, *J =* 8 Hz, 1H), 7.16 (d, *J =* 8.4 Hz, 1H), 3.25-3.16 (m, 2H), 2.39 (s, 3H), 2.19 (s, 3H), 1.10 (t, *J =* 7.2 Hz, 3H) ppm.

### Step 4: Preparation of 3-(2-(ethylamino)ethyl)-5-methyl-1H-indol-4-ol (37).

To a stirred solution of LiAlH₄ solution (2.0 M in THF; 13.01 mL, 26.01 mmol) in anhydrous (25 mL) THF was added 3-(2-(ethylamino)-2-oxoacetyl)-5-methyl-1*H*-indol-4-yl acetate (0.500 g, 1.73 mmol) in THF (50 mL) at 0 °C over a period of 10 min and the resulting mixture was then heated to 70 °C for 9 h. Reaction progress was monitored by TLC and LC-MS, and upon completion, the reaction was cooled to room temperature and then to 0 °C and quenched with THF/water (1:1), until effervescence stopped. The quenched reaction mixture was diluted with THF (100 mL), stirred at room temperature for 30 min under N₂ atmosphere, filtered through Celite, and the filter cake was washed with THF (200 mL). The combined organic filtrates were dried over anhydrous Na₂SO₄ and concentrated under reduced pressure to provide the crude product, which was purified by silica gel column chromatography using 0-10% MeOH in DCM, to afford 3-(2-(ethylamino)ethyl)-5-methyl-1*H*-indol-4-ol (**37**) as a grey solid (Yield: 0.140 g, 36.98 %). **HRMS** m/z 219.22 [M+1]⁺; **¹H NMR (400MHz, DMSO-d₆):** *δ* = 10.42 (s, 1H), 6.86 (d, *J =* 2 Hz, 1H), 6.73 (d, *J* = 8 Hz, 1H), 6.64 (d, *J* = 8 Hz, 1H), 3.41-3.36 (m, 3H), 2.82-2.76 (m, 4H), 2.56-2.51 (m, 2H), 2.15 (s, 3H), 1.04 (t, *J =* 7.2 Hz, 3H) ppm.

### Step 5: Preparation of 3-(2-(ethylamino)ethyl)-5-methyl-1H-indol-4-ol (37) hemifumarate.

To a stirred solution of 3-(2-(ethylamino)ethyl)-5-methyl-1*H*-indol-4-ol (**37;** 0.140 g, 0.641 mmol) in 10% MeOH in DCM (2 mL) was added fumaric acid (0.037 g, 0.320 mmol) at 0 °C over a period of 5 min and the reaction mixture was stirred at room temperature for 4 h. At this time, the reaction mixture was concentrated under reduced pressure to afford the crude product, which was triturated using n-pentane (2 mL) and diethyl ether (1 mL) to afford 3-(2-(ethylamino)ethyl)-5-methyl-1*H*-indol-4-ol (37) hemifumarate as a grey solid (Yield: 0.111 g, 59.82%). **HRMS** m/z 219.30 [M+1]⁺; **¹H NMR** (**400 MHz, DMSO-d₆):** δ = 10.53 (s, 2H), 9.36 (br s, 2H), 6.91 (d, *J =* 2 Hz, 2H), 6.74 (d, *J =* 8 Hz, 2H), 6.69 (d, *J =* 8 Hz, 2H), 6.43 (s, 2H), 2.98 (s, 8H), 2.78-2.72 (m, 4H), 2.18 (s, 6H), 1.11 (t, *J =* 7.2 Hz, 6H) ppm.

### Example 21. Preparation of Compound 38 Hemifumarate

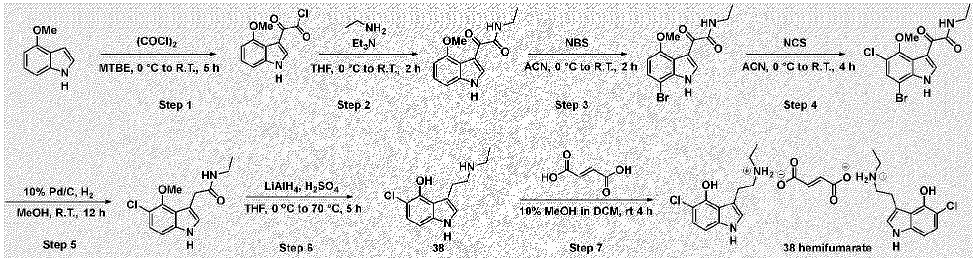

### Step 1: Preparation of 2-(4-methoxy-1H-indol-3-yl)-2-oxoacetyl chloride.

To a stirred solution of 4-methoxy-1*H*-indole (5.0 g, 33.97 mmol) in MTBE (50 mL) at 0 °C was added (COCl)₂ (4.37 mL, 50.96 mmol) in dropwise over a period of 5 min and the reaction mixture was stirred at 0 °C for 30 min. Then, the reaction mixture was warmed to room temperature and stirred for 5 h. After completion of the reaction (monitored by TLC), the reaction mixture was concentrated under N₂ atmosphere to afford 2-(4-methoxy-1*H*-indol-3-yl)-2-oxoacetyl chloride as a dark-yellow liquid (Yield: 5.7 g, 70.60 *%). Note:* Crude compound was directly used for the next step without further purification.

### Step 2: Preparation of N-ethyl-2-(4-methoxy-1H-indol-3-yl)-2-oxoacetamide.

To a stirred solution of 2-(4-methoxy-1*H*-indol-3-yl)-2-oxoacetyl chloride (5.7 g, 23.99 mmol) in anhydrous THF (50 mL) at 0 °C, was added ethylamine (2M in THF; 17.99 mL, 35.98 mmol) dropwise followed by dropwise addition of Et₃N (10 mL, 71.96 mmol) at 0 °C. The reaction mixture was warmed to room temperature and stirred for 2 h. After completion of the reaction (monitored by TLC), the reaction mixture was diluted with water (25 mL), extracted with DCM (2 x 20 mL). The combined organic solvent was dried over anhydrous Na₂SO₄ and concentrated under reduced pressure to afford crude compound which was triturated with n-pentane (5 mL) and diethyl ether (1 mL) to afford *N*-ethyl-2-(4-methoxy-1*H*-indol-3-yl)-2-oxoacetamide (crude) as a yellow solid (Yield: 3.5 g, 59.25%). **HRMS** m/z 246.90 [M+1]⁺; **¹H NMR** (**400MHz, DMSO-d₆):** *δ =* 12.10 (s, 1H), 8.60 (t, *J =* 4.8 Hz, 1H), 8.32 (d, *J* = 2.8 Hz, 1H), 7.17 (t, *J =* 8 Hz, 1H), 7.09 (d, *J =* 8 Hz, 1H), 6.70 (d, *J =* 7.6 Hz, 1H), 3.81 (s, 3H), 3.26-3.19 (m, 2H), 1.11 (t, *J =* 7.2 Hz, 3H) ppm.

### Step 3: Preparation of 2-(7-bromo-4-methoxy-1H-indol-3-yl)-N-ethyl-2-oxoacetamide.

To a stirred solution of *N*-ethyl-2-(4-methoxy-1*H*-indol-3-yl)-2-oxoacetamide (3.5 g, 14.21 mmol) in anhydrous ACN (20 mL) was added NBS (0.970 g, 17.05 mmol) portionwise at 0 °C under an N₂ atmosphere and the resulting mixture was then stirred for 2 h at room temperature. Reaction progress was monitored by TLC and LC-MS. After completion, the reaction mixture was quenched with ice-cold water (15 mL) and extracted with EtOAc (2 x 75 mL). The combined organic layers were washed with brine (50 mL), dried over anhydrous Na₂SO₄, and concentrated under reduced pressure to afford the crude product, which was purified by silica gel chromatography, eluting with 0-15% EtOAc in hexane, to afford 2-(7-bromo-4-methoxy-1*H*-indol-3-yl)-*N*-ethyl-2-oxoacetamide as a yellow solid (Yield: 2.2 g, 47.61%). **HRMS** m/z 326.85 [M+2]⁺; **¹H NMR (400MHz, DMSO-d₆):** *δ* = 12.27 (s, 1H), 8.65 (s, 1H), 8.29 (d, *J =* 2.8 Hz, 1H), 7.38 (d, *J =* 8.4 Hz, 1H), 6.68 (d, *J* = 8.4 Hz, 1H), 3.81 (s, 3H), 3.24-3.20 (m, 2H), 1.10 (t, *J =* 7.2 Hz, 3H) ppm.

### Step 4: Preparation of 2-(7-bromo-5-chloro-4-methoxy-1H-indol-3-yl)-N-ethyl-2-oxoacetamide.

To a stirred solution of 2-(7-bromo-4-methoxy-1*H*-indol-3-yl)-N-ethyl-2-oxoacetamide (2.2 g, 6.77 mmol) in anhydrous ACN (20 mL) was added NCS (0.471 g, 8.12 mmol) portionwise at 0 °C under an N₂ atmosphere and the resulting mixture was then stirred for 4 h at room temperature. Reaction progress was monitored by TLC and LC-MS. After completion, the reaction mixture was quenched with ice-cold water (15 mL) and extracted with EtOAc (2 x 75 mL). The combined organic layers were washed with brine (50 mL), dried over anhydrous Na₂SO₄, and concentrated under reduced pressure to afford the crude product, which was purified by silica gel chromatography, eluting with 10-25% EtOAc in hexane, to afford 2-(7-bromo-5-chloro-4-methoxy-1*H*-indol-3-yl)-*N*-ethyl-2-oxoacetamide as a yellow solid (Yield: 1.3 g, 53.43 %). **HRMS** m/z 360.85 [M+2]⁺; **¹H NMR (400MHz, DMSO-d₆):** *δ* = 12.62 (s, 1H), 8.80 (s, 1H), 8.59 (d, *J =* 2.8 Hz, 1H), 7.64 (s, 1H), 3.79 (s, 3H), 3.27-3.20 (m, 2H), 1.17-1.09 (m, 3H) ppm.

### Step 5: Preparation of 2-(5-chloro-4-methoxy-1H-indol-3-yl)-N-ethylacetamide.

To a stirred solution of 2-(7-bromo-5-chloro-4-methoxy-1*H*-indol-3-yl)-N-ethyl-2-oxoacetamide (1.3 g, 3.62 mmol) in methanol (25 mL) was added 10% Pd/C (0.856 g, 0.723 mmol) at room temperature under N₂ atmosphere. The reaction mixture was degassed with N₂ for 2-3 min and stirred at room temperature under H₂ atmosphere (balloon) for 12 h. After completion of the reaction (monitored by TLC and LC-MS), the reaction mixture was passed through a Celite pad to remove the catalyst and the filter cake was washed with 10% MeOH in DCM (2 x 30 mL). The combined organic filtrates were concentrated under reduced pressure to afford the crude product, which was purified by silica gel chromatography, eluting with 0-5% MeOH in DCM, to afford 2-(5-chloro-4-methoxy-1*H*-indol-3-yl)-*N*-ethylacetamide as a brown, sticky solid (Yield: 0.520 g, 53.93 %). **HRMS** m/z 267.05 [M+1]⁺; **¹H NMR (400MHz, DMSO-d₆):** *δ* = 12.26 (s, 1H), 8.65 (t, *J* = 5.6 Hz, 1H), 8.29 (d, *J* = 3.2 Hz, 1H), 7.38 (d, *J =* 8.4 Hz, 1H), 6.67 (d, *J =* 8.4 Hz, 1H), 3.81 (s, 3H), 3.25-3.19 (m, 2H), 2.56 (s, 1H), 1.10 (t, *J =* 7.2 Hz, 3H) ppm.

### Step 6: Synthesis of 5-chloro-3-(2-(ethylamino)ethyl)-1H-indol-4-ol (38).

To a stirred solution of LiAlH₄ (2.0 M in THF; 3.90 mL, 7.80 mmol) in anhydrous THF (15 mL) was added H₂SO₄ (0.20 mL, 3.90 mmol) dropwise at 0 °C and the mixture was stirred at 0 °C for 30 min. At solution of 2-(5-chloro-4-methoxy-1*H*-indol-3-yl)-*N*-ethylacetamide (0.520 g, 1.95 mmol) in THF (50 mL) was then added at 0 °C over a period of 10 min and the resulting mixture was heated to 70 °C for 5 h. Reaction progress was monitored by TLC and LC-MS and upon completion, the reaction mixture was cooled to room temperature and then to 0 °C, and then quenched with THF/water (**1**:1) at 0 °C until effervesce stopped. The reaction mixture was then diluted with THF (100 mL), stirred at room temperature for 30 min under N₂ atmosphere, filtered through Celite, and the filter cake was washed with THF (100 mL). The combined organic filtrates were dried over anhydrous Na₂SO₄ and concentrated under reduced pressure to afford the crude product, which was purified by silica gel column chromatography using 0-10% MeOH in DCM, to afford 5-chloro-3-(2-(ethylamino)ethyl)-1*H*-indol-4-ol (38) as a grey solid (Yield: 0.17 g, 36.53%). **HRMS** m/z 239.05[M+1]⁺; **¹H NMR** (**400MHz, DMSO-d₆):** *δ* = 10.69 (s, 1H), 9.89-9.40 (m, 1H), 6.95 (d, *J* = 2.4 Hz, 1H), 6.89 (d, *J =* 8.4 Hz, 1H), 6.65 (d, *J =* 8.4 Hz, 1H), 3.56-3.36 (m, 1H), 2.96-2.79 (m, 4H), 2.59 (q, *J* = 7.2 Hz, 2H), 1.07 (t, *J =* 7.2 Hz, 3H) ppm.

### Step 7: Synthesis of 5-chloro-3-(2-(ethylamino)ethyl)-1H-indol-4-ol (38) hemifumarate.

To a stirred solution of 5-chloro-3-(2-(ethylamino)ethyl)-1*H*-indol-4-ol (**38;** 0.170 g, 0.712 mmol) in 10% MeOH in DCM (2 mL) was added fumaric acid (0.041 g, 0.356 mmol) at 0 °C over a period of 5 min and the reaction mixture was stirred at room temperature for 4 h. At this time, the reaction mixture was concentrated under reduced pressure to afford the crude product, which was triturated using n-pentane (4 mL) and diethyl ether (2 mL) to afford 5-chloro-3-(2-(ethylamino)ethyl)-1H-indol-4-ol (38) hemifumarate as an off-white solid (Yield: 0.180 g, 71.65%). **HRMS** m/z 239.20 [M+1]⁺; **¹H NMR (400MHz, DMSO-d₆):** *δ* = 10.79 (s, 2H), 10.06 (br. s, 3H), 6.99 (d, *J* = 2.4 Hz, 2H), 6.91 (d, *J* = 8.4 Hz, 2H), 6.72 (d, *J =* 8.4 Hz, 2H), 6.46 (s, 2H), 2.93 (s, 8H), 2.73-2.66 (m, 4H), 1.10 (t, *J =* 7.2 Hz, 6H) ppm.

### Example 22. Preparation of Compound 39 Hemifumarate

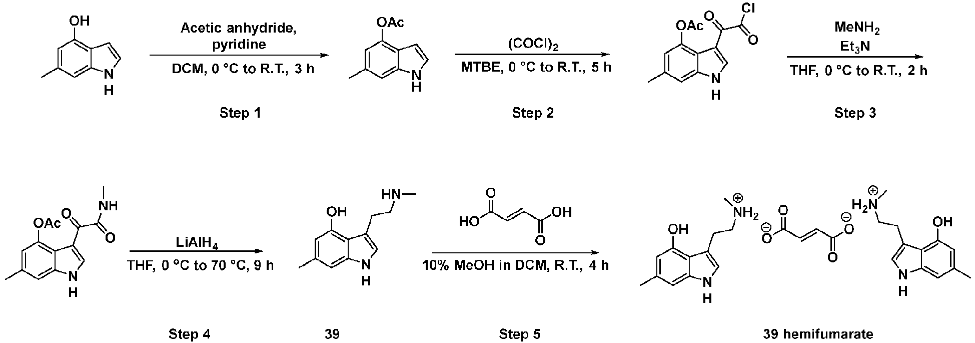

### Step 1: Preparation of 6-methyl-1H-indol-4-yl acetate.

To a stirred solution of 6-methyl-1*H*-indol-4-ol (0.50 g, 3.40 mmol) in anhydrous DCM (10 mL) was added pyridine (0.41 mL, 5.10 mmol) dropwise at 0 °C under an N₂ atmosphere and the mixture was stirred for 15 min. Acetic anhydride (0.50 mL, 4.08 mmol) was then added dropwise over a period of 5 min at 0 °C and the reaction mixture was warmed to room temperature and stirred for an additional 3 h at room temperature. Reaction progress was monitored by TLC and LC-MS. After completion, the reaction mixture was quenched by slow addition of ice-cold water (10 mL) and extracted with DCM (2 x 15 mL). The combined organic extracts were washed with brine (25 mL), dried over anhydrous Na₂SO₄, and concentrated under reduced pressure to afford the crude product. This crude was triturated with diethyl ether (2 mL) and n-pentane (10 mL) to provide 6-methyl-1*H-*indol-4-yl acetate as a brown solid **(Yield: 0.500 g, 77.78 %).** HR**MS** m/z 189.9 [M+1]⁺; **¹H NMR (400MHz, DMSO-d₆):** *δ* = 11.11 (s, 1H), 7.24 (t, *J =* 2.8 Hz, 1H), 7.07 (s, 1H), 6.56 (s, 1H), 6.24 (s, 1H), 2.37 (s, 3H), 2.32 (s, 3H) ppm.

### Step 2: Preparation of 3-(2-chloro-2-oxoacetyl)-6-methyl-1H-indol-4-yl acetate.

To a stirred solution of 6-methyl-1*H*-indol-4-yl acetate (0.500 g, 2.64 mmol) in MTBE (10 mL) at 0 °C was added (COCl)₂ (0.33 mL, 3.96 mmol) dropwise over a period of 5 min and the resulting mixture was stirred at 0 °C for 30 min. The reaction mixture was then allowed to warm to room temperature and stirred for 5 h. The progress of the reaction was monitored by TLC. After completion, the reaction mixture was concentrated under reduced pressure (and backfilled with N₂ atmosphere) to afford 3-(2-chloro-2-oxoacetyl)-6-methyl-1*H*-indol-4-yl acetate as a dark-yellow liquid (Yield: 0.600 g, 81.19 *%). Note:* The crude compound was directly used for the next step without further purification.

### Step 3: Preparation of 6-methyl-3-(2-(methylamino)-2-oxoacetyl)-1H-indol-4-yl acetate.

To a stirred solution of 3-(2-chloro-2-oxoacetyl)-6-methyl-1*H*-indol-4-yl acetate (0.600 g, 2.15 mmol) in anhydrous THF (10 mL) at 0 °C, was added methylamine (2M in THF; 1.61 mL, 3.22 mmol) dropwise followed by Et₃N (0.89 mL, 6.44 mmol). After complete addition, the reaction mixture was warmed to room temperature and stirred for 2 h. After completion of the reaction (monitored by TLC), the reaction mass was diluted with water (10 mL) and extracted with DCM (2 x 20 mL). The combined organic extracts were dried over anhydrous Na₂SO₄ and concentrated under reduced pressure to afford the crude compound, which was triturated with *n*-pentane (5 mL) and diethyl ether (1 mL) to afford 6-methyl-3-(2-(methylamino)-2-oxoacetyl)-1*H*-indol-4-yl acetate as a yellow solid (Yield: 0.500 g, 85%). **HRMS** m/z 274.95 [M+1]⁺ ; **¹H NMR (400MHz, DMSO-d₆):** *δ* = 12.29 (s, 1H), 8.60 (s, 1H), 8.56 (d, *J =* 4.4 Hz, 1H), 7.22 (s, 1H), 6.73 (s, 1H), 2.73 (d, *J =* 4.8 Hz, 3H), 2.40 (s, 3H), 2.34 (s, 3H) ppm.

### Step 4: Preparation of 6-methyl-3-(2-(methylamino)ethyl)-1H-indol-4-ol (39).

To a stirred solution of LiAlH₄ (2.0M in THF; 7.29 mL, 14.58 mmol) in anhydrous THF (30 mL) was added H₂SO₄ (0.39 mL, 7.29 mmol) dropwise at 0 °C and the reaction mixture was stirred at 0 °C for 30 min. A solution of 6-methyl-3-(2-(methylamino)-2-oxoacetyl)-1*H*-indol-4-yl acetate (0.50 g, 1.82 mmol) in THF (10 mL) was then added at 0 °C over a period of 10 min. After complete addition, the reaction mixture was heated to 70 °C and stirred for 9 h. Reaction progress was monitored by TLC and LC-MS. Upon completion, the reaction mixture was cooled to 0 °C and quenched with THF/water (1:1, 14 mL) until effervescence stopped. The quenched reaction mixture was then diluted with THF (100 mL), stirred at room temperature for 30 min under an N₂ atmosphere, filtered through a Celite pad, and the filter cake was washed with THF (100 mL). The combined organic filtrates were dried over anhydrous Na₂SO₄ and concentrated under reduced pressure to afford the crude product, which was purified by silica gel column chromatography using 0-10% MeOH in DCM to afford 6-methyl-3-(2-(methylamino)ethyl)-1*H*-indol-4-ol (**39**) as a grey solid (Yield: 0.240 g, 64.45 *%). Note:* The crude compound was directly used for the next step without further characterization.

### Step 5: Preparation of 6-methyl-3-(2-(methylamino)ethyl)-1H-indol-4-ol (39) hemifumarate.

To a stirred solution of 6-methyl-3-(2-(methylamino)ethyl)-1*H*-indol-4-ol (**39;** 0.240 g, 1.17 mmol) in 10% MeOH in DCM (2 mL) was added fumaric acid (0.068 g, 0.587 mmol) at 0 °C over a period of 5 min. The reaction mixture was warmed to room temperature and stirred for 4 h. The reaction mixture was then concentrated under reduced pressure to afford the crude compound. This crude material was triturated using n-pentane (2 mL) and diethyl ether (1 mL) to afford 6-methyl-3-(2-(methylamino)ethyl)-1*H*-indol-4-ol (**3**9**)** hemifumarate as an off-white solid (Yield: 0.203 g, 65.86%). **HRMS** m/z 205.20 [M+1]⁺; **¹H NMR (400 MHz, DMSO-d₆):** δ = 10.51 (s, 2H), 9.42 (br s, 3H), 6.85 (d, *J =* 2.4 Hz, 2H), 6.54 (s, 2H), 6.38 (s, 2H), 6.13 (s, 2H), 3.00-2.94 (m, 8H), 2.43 (s, 6H), 2.24 (s, 6H) ppm.

### Example 23. Preparation of Compound 10

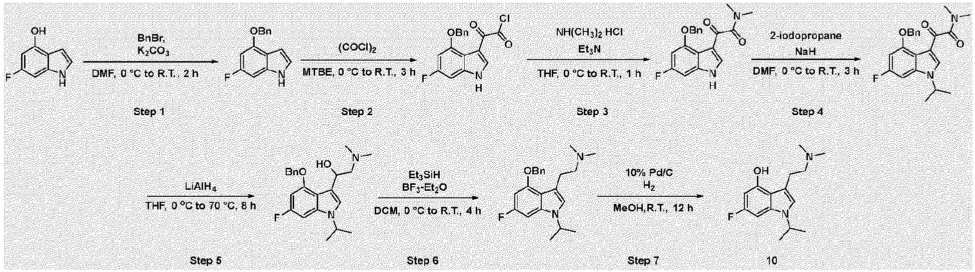

### Step 1: Preparation of 4-(benzyloxy)-6-fluoro-1H-indole.

To a stirred solution of 6-fluoro-1*H*-indol-4-ol (2 g, 13.23 mmol) in anhydrous DMF (20 mL) was added K₂CO₃ (3.66 g, 26.47 mmol) portionwise at 0 °C under an N₂ atmosphere and the resulting mixture was stirred for 15 min. Benzyl bromide (2.36 mL, 19.85 mmol) was then added dropwise over 10 min to the reaction mixture at 0 °C and it was then stirred at room temperature for 2 h. Reaction progress was monitored by TLC and LC-MS. After completion, the reaction mixture was quenched with ice-cold water (10 mL) and extracted with EtOAc (2 x 75 mL). The combined organic layers were washed with brine (50 mL), dried over anhydrous Na₂SO₄, and concentrated under reduced pressure to afford the crude product, which was purified by silica gel chromatography, eluting with a 0-5% EtOAc in hexane gradient, to afford 4-(benzyloxy)-6-fluoro-1H-indole as a white solid **(Yield: 1.9 g, 59 %). HRMS** m/z 242.10 [M+1]⁺; **¹H NMR** (**400 MHz, DMSO-d₆):** *δ* = 11.15 (s, 1H), 7.49 (d, *J =* 7.2 Hz, 2H), 7.41 (t, *J =* 7.2 Hz, 2H), 7.35-7.32 (m, 1H), 7.20 (t, *J* = 2.8 Hz, 1H), 6.78-6.75 (m, 1H), 6.51 (dd, *J* = 2.0 Hz, 12 Hz, 1H), 6.43 (s, 1H), 5.21 (s, 2H) ppm.

### Step 2: Preparation of 2-(4-(benzyloxy)-6-fluoro-1H-indol-3-yl)-2-oxoacetyl chloride.

To a stirred solution of 4-(benzyloxy)-6-fluoro-1*H*-indole (1.9 g, 7.88 mmol) in MTBE (20 mL) at 0 °C was added oxalyl chloride (1.01 mL, 11.81 mmol) dropwise over a period of 5 min and the reaction mixture was stirred at 0 °C for 30 min and then at room temperature for 3 h. The progress of the reaction was monitored by TLC and upon completion, the solvents were removed under reduced pressure to afford 2-(4-(benzyloxy)-6-fluoro-1*H*-indol-3-yl)-2-oxoacetyl chloride crude as a light-yellow liquid (Yield: 1.9 g, 72 *%). Note:* The crude compound was directly used for the next step without further purification.

### Step 3: Preparation of 2-(4-(benzyloxy)-6-fluoro-1H-indol-3-yl)-N,N-dimethyl-2-oxoacetamide.

To a stirred solution of 2-(4-(benzyloxy)-6-fluoro-1*H*-indol-3-yl)-2-oxoacetyl chloride (1.9 g, 5.73 mmol) in anhydrous THF (20 mL) at 0 °C was added dimethylamine hydrochloride (0.700 g, 8.59 mmol). Et₃N (2.40 mL, 17.18 mmol) was then added to the reaction mixture dropwise at 0 °C and the resulting mixture was stirred for 1 h at room temperature. After completion of the reaction (monitored by TLC), the reaction mixture was diluted with water (20 mL) and extracted with DCM (2 x 20 mL). The combined organic layers were dried over anhydrous Na₂SO₄ and concentrated under reduced pressure to afford the crude compound, which was purified by silica gel chromatography using 0-5% MeOH in DCM to afford 2-(4-(benzyloxy)-6-fluoro-1H-indol-3-yl)-N,N-dimethyl-2-oxoacetamide as a yellow solid (Yield: 0.90 g, 46 %). **HRMS** m/z 341.00 [M+1]⁺; **¹H NMR** (**400MHz, DMSO-d₆):** *δ* = 12.30 (s, 1H), 8.14 (s, 1H), 7.70-7.61 (m, 2H), 7.38 (t, *J* = 7.6 Hz, 2H), 7.31-7.23 (m, 1H), 6.87 (d, *J* = 7.6 Hz, 1H), 6.64 (d, *J* = 12 Hz, 1H), 5.27 (s, 2H), 2.89 (d, *J* = 8.0 Hz, 6H) ppm.

### Step 4: Synthesis of 2-(4-(benzyloxy)-6-fluoro-1-isopropyl-1H-indol-3-yl)-N,N-dimethyl-2-oxoacetamide.

To a stirred solution of 2-(4-(benzyloxy)-6-fluoro-1*H*-indol-3-yl)-*N,N*-dimethyl-2-oxoacetamide (0.9 g, 2.64 mmol) in anhydrous DMF (10 mL) was added NaH (0.21 g, 5.29 mmol) portionwise at 0 °C under an N₂ atmosphere and the mixture was stirred for 15 min. 2-Iodopropane (0.674 g, 3.97 mmol) was then added over 10 min to the above reaction mixture at 0 °C and it was stirred for an additional 3 h at room temperature. Reaction progress was monitored by TLC and LC-MS. After completion, the reaction mixture was quenched with ice-cold water (10 mL) and extracted with EtOAc (2 x 25 mL). The combined organic layers were washed with brine (15 mL), dried over anhydrous Na₂SO₄, and concentrated under reduced pressure. The crude product was purified by silica gel chromatography, eluting with 0-3% MeOH in DCM, to afford 2-(4-(benzyloxy)-6-fluoro-1-isopropyl-1*H*-indol-3-yl)-*N,N*-dimethyl-2-oxoacetamide as a yellow sticky solid **(Yield: 0.600 g, 59 %). HRMS m/z 383.15 [M+1]⁺; ¹H NMR (400MHz, DMSO-d₆):** *δ* = 8.18 (s, 1H), 7.56 (d, *J=* 7.2 Hz, 2H), 7.42 (t, *J* = 10 Hz, 2H), 7.38-7.31 (m, 1H), 7.29-7.13 (m, 1H), 6.62 (dd, *J* = 2 Hz, 12 Hz, 1H), 5.29 (s, 2H), 4.78-4.71 (m, 1H), 2.97-2.85 (m, 6H), 1.48-1.39 (m, 6H) ppm.

### Step 5: Synthesis of 1-(4-(benzyloxy)-6-fluoro-1-isopropyl-1H-indol-3-yl)-2-(dimethylamino)ethan-1-ol.

To a stirred solution of LiAlH₄ solution (2.0 M in THF; 6.28 mL, 12.55 mmol) in anhydrous THF (20 mL) was added 2-(4-(benzyloxy)-6-fluoro-1-isopropyl-1*H*-indol-3-yl)-*N,N-*dimethyl-2-oxoacetamide (0.600 g, 1.57 mmol) in THF (10 mL) at 0 °C over a period of 10 min and the mixture was heated to 70 °C for 8 h. Reaction progress was monitored by TLC and LC-MS. After completion, the reaction mixture was cooled to 0 °C and quenched by the addition of THF/water (1:1) until effervescence stopped. Then the reaction mixture was diluted with THF (100 mL), stirred at room temperature for 30 min under N₂ atmosphere, filtered through Celite, and the filter cake was washed with THF (200 mL). The combined organic filtrates were dried over anhydrous Na₂SO₄ and concentrated under reduced pressure to afford the crude product, which was purified by silica gel column chromatography using 0-10% MeOH in DCM to afford 1-(4-(benzyloxy)-6-fluoro-1-isopropyl-1*H*-indol-3-yl)-2-(dimethylamino)ethan-1-ol as a brown liquid (Yield: 0.40 g, 68.8 %). **HRMS** m/z 371.10 [M+1]⁺.

### Step 6: Synthesis of 2-(4-(benzyloxy)-6-fluoro-1-isopropyl-1H-indol-3-yl)-N,N-dimethylethan-1-amine.

To a stirred solution of 1-(4-(benzyloxy)-6-fluoro-1-isopropyl-1*H*-indol-3-yl)-2-(dimethylamino)ethan-1-ol (0.40 g, 1.08 mmol) in DCM (10 mL) was added triethylsilane (1.39 mL, 8.64 mmol) and boron trifluoride diethyl etherate (0.53 mL, 4.32 mmol) at 0 °C under an N₂ atmosphere and the mixture was stirred for 4 h at room temperature. Reaction progress was monitored by TLC and LC-MS. After completion, the reaction mixture was quenched with saturated aqueous NaHCO₃ solution (5 mL) and extracted with DCM (2 x 15 mL). The combined organic layers were washed with brine (15 mL), dried over anhydrous Na₂SO₄, and concentrated under reduced pressure to afford the crude product, which was purified by silica gel chromatography, eluting with 0-5% MeOH in DCM, to afford 2-(4-(benzyloxy)-6-fluoro-1-isopropyl-1*H*-indol-3-yl)-*N,N-*dimethylethan-1-amine as a yellow sticky solid (Yield: 0.335 g, 87%). **HRMS** m/z 355.10 [M+1]⁺.

### Step 7: Synthesis of 3-(2-(dimethylamino)ethyl)-6-fluoro-1-isopropyl-1H-indol-4-ol (10).

To a stirred solution of 2-(4-(benzyloxy)-6-fluoro-1-isopropyl-1*H*-indol-3-yl)-*N,N-*dimethylethan-1-amine (0.335 g, 0.945 mmol) in methanol (10 mL) was added 10 % Pd/C (0.22 g, 0.189 mmol) at room temperature under N₂ atmosphere. The reaction mixture was degassed by sparging with N₂ for 2-3 min and then stirred at room temperature under H₂ atmosphere (balloon) for 12 h. After completion of the reaction (monitored by TLC and LC-MS), the reaction mixture was passed through a Celite pad to remove insoluble catalyst and the filter cake was washed with 10% MeOH in DCM (2 x 30 mL). The combined organic filtrates were concentrated under reduced pressure to afford the crude product, which was triturated using *n*-pentane (5 mL) and diethyl ether (1 mL) to afford 3-(2-(dimethylamino)ethyl)-6-fluoro-1-isopropyl-1*H*-indol-4-ol (**10**) as a light-brown solid (Yield: 0.117 g, 46.8 %). **HRMS** m/z 265.30 [M+1]⁺; **¹H NMR (400MHz, DMSO-d₆):** *δ* = 12.01 (br. s, 1H), 7.05 (s, 1H), 6.66 (dd, *J* = 2.4 Hz, 10.4 Hz, 1H), 6.10 (dd, *J* = 2 Hz, 11.6 Hz, 1H), 4.53-4.46 (m, 1H), 2.84 (t, *J* = *6.4* Hz, 2H), 2.66 (t, *J* = 1.6 Hz, 2H), 2.24 (s, 6H), 1.35 (t, *J* = 6.8 Hz, 6H) ppm.

### Example 24. Preparation of Compound 12

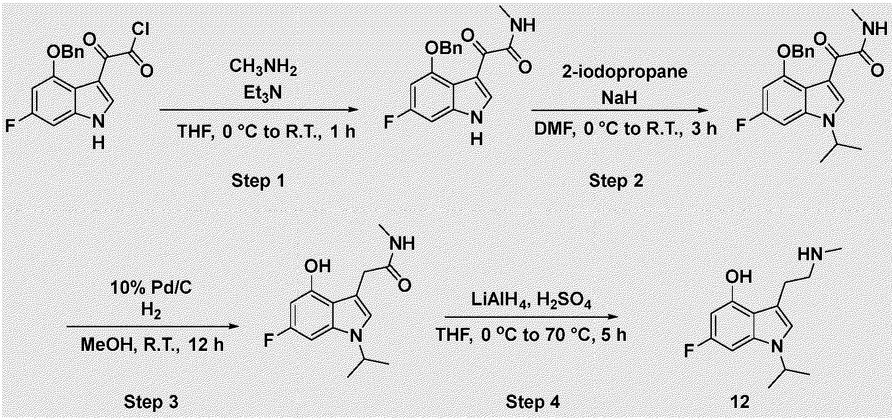

### Step 1: Synthesis of 2-(4-(benzyloxy)-6-fluoro-1H-indol-3-yl)-N-methyl-2-oxoacetamide.

To a stirred solution of 2-(4-(benzyloxy)-6-fluoro-1*H*-indol-3-yl)-2-oxoacetyl chloride (see Example 23; 1.8 g, 5.43 mmol) in anhydrous THF (20 mL) at 0 °C was added CH₃NH₂ (2.0M in THF; 4.07 mL, 8.14 mmol). Et₃N (2.27 mL, 16.28 mmol) was then added to the reaction mixture dropwise at 0 °C and the resulting mixture was stirred for 1 h at room temperature. After completion of the reaction (monitored by TLC), the reaction mixture was diluted with water (20 mL) and extracted with DCM (2 x 20 mL). The combined organic layers were dried over anhydrous Na₂SO₄ and concentrated under reduced pressure to afford the crude product, which was purified by silica gel chromatography using a 0-5% MeOH in DCM gradient to afford 2-(4-(benzyloxy)-6-fluoro-1*H-*indol-3-yl)-N-methyl-2-oxoacetamide as a yellow solid (Yield: 1.1 g, 62.12 %). **HRMS** m/z 327.05 [M+1]⁺; **¹H NMR (400MHz, DMSO-d₆):** *δ* = 12.15 (s, 1H), 8.57 (s, 1H), 8.34 (s, 1H), 7.66 (d, *J* = 7.2 Hz, 2H), 7.39 (t, *J =* 7.2 Hz, 2H), 7.32-7.18 (m, 1H), 6.90-6.88 (m, 1H), 6.70 (d, *J =* 11.6 Hz, 1H), 5.25 (s, 2H), 2.70-2.67 (m, 3H) ppm.

### Step 2: Synthesis of 2-(4-(benzyloxy)-6-fluoro-1-isopropyl-1H-indol-3-yl)-N-methyl-2-oxoacetamide.

To a stirred solution of *N*-methyl-2-oxoacetamide (1.1 g, 3.37 mmol) in anhydrous DMF (10 mL) was added NaH (0.269 g, 6.74 mmol) portionwise at 0 °C under an N₂ atmosphere and the resulting mixture was stirred for 15 min. 2-Iodopropane was then added over 10 min to the above reaction mixture at 0 °C and it was stirred for an additional 3 h at room temperature. Reaction progress was monitored by TLC and LC-MS. After completion, the reaction was quenched with ice-cold water (10 mL) and extracted with EtOAc (2 x 25 mL). The combined organic layers were washed with brine (2 x 15 mL), dried over anhydrous Na₂SO₄, and concentrated under reduced pressure to afford the crude product, which was purified by silica gel chromatography, eluting with 0-5% MeOH in DCM, to afford 2-(4-(benzyloxy)-6-fluoro-1-isopropyl-1*H*-indol-3-yl)-*N*-methyl-2-oxoacetamide as a yellow sticky solid **(Yield: 1.0 g, 80%). HRMS m/z 369.05 [M+1]⁺; ¹H NMR (400MHz, DMSO-d₆):** *δ* = 8.58 (d, *J =* 4.4 Hz, 1H), 8.40 (s, 1H), 7.62 (d, *J* = 7.6 Hz, 2H), 7.39-7.36 (m, 2H), 7.31-7.27 (m, 1H), 7.17 (d, *J =* 9.2 Hz, 1H), 6.72 (d, *J* = 11.6 Hz, 1H), 5.24 (s, 2H), 4.78-4.71 (m, 1H), 2.67 (d, *J* = 4.4 Hz, 3H), 1.47 (d, *J =* 6.4 Hz, 6H) ppm.

### Step 3: Synthesis of 2-(6-fluoro-4-hydroxy-1-isopropyl-1H-indol-3-yl)-N-methylacetamide.

To a stirred solution of 2-(4-(benzyloxy)-6-fluoro-1-isopropyl-1*H*-indol-3-yl)-*N*-methyl-2-oxoacetamide (1.0 g, 2.71 mmol) in methanol (10 mL) was added 10% Pd/C (0.642 g, 0.542 mmol) at room temperature under an N₂ atmosphere. The reaction mixture was degassed by sparging with N₂ for 2-3 min and then stirred at room temperature under H₂ atmosphere (balloon) for 12 h. After completion of the reaction (monitored by TLC and LC-MS), the reaction mixture was passed through a Celite pad to remove insoluble catalyst and the filter cake was washed with 10% MeOH in DCM (2 x 30 mL). The combined organic filtrates were concentrated under reduced pressure to afford the crude product, which was triturated using n-pentane (10 mL) and diethyl ether (3 mL) to afford 2-(6-fluoro-4-hydroxy-1-isopropyl-1*H*-indol-3-yl)-*N*-methylacetamide (Yield: 0.400 g, 55.7 %). **HRMS** m/z 265.10 [M+1]⁺; **¹H NMR (400MHz, DMSO-d₆):** *δ* = 11.68 (s, 1H), 8.20 (br. s, 1H), 7.06 (s, 1H), 6.80-6.70 (m, 1H), 6.21-6.18 (m, 1H), 4.58-4.50 (m, 1H), 3.59 (s, 2H), 2.61 (d, *J* = 4.8 Hz, 3H), 1.40 (t, *J* = 6.8 Hz, 6H) ppm.

### Step 4: Synthesis of 6-fluoro-1-isopropyl-3-(2-(methylamino)ethyl)-1H-indol-4-ol (12).

To a stirred solution of LiAlH₄ solution (2.0M in THF; 6.05 mL, 12.11 mmol) in anhydrous THF (15 mL) was added H₂SO₄ (0.32 mL, 6.05 mmol) dropwise at 0 °C and the resulting mixture was stirred at 0 °C for 30 min. A solution of 2-(6-fluoro-4-hydroxy-1-isopropyl-1*H*-indol-3-yl)-*N-*methylacetamide (0.400 g, 1.51 mmol) in THF (5 mL) was then added at 0 °C over a period of 10 min and the reaction mixture was heated to 70 °C for 5 h. Reaction progress was monitored by TLC and LC-MS. After completion, the reaction was cooled to 0 °C and quenched by the addition of THF/water (1:1) until effervescence stopped. Then the quenched reaction mixture was diluted with THF (100 mL), stirred at room temperature for 30 min under N₂ atmosphere, filtered through Celite, and the filter cake was washed with THF (100 mL). The combined organic filtrates were dried over anhydrous Na₂SO₄ and concentrated under reduced pressure to afford the crude product, which was purified by silica gel column chromatography using 0-10% MeOH in DCM to afford 6-fluoro-1-isopropyl-3-(2-(methylamino)ethyl)-1*H*-indol-4-ol (**12**) as a brown solid (Yield: 0.150 g, 39.6 %). **HRMS** m/z 251.25 [M+1]⁺; **¹H NMR (400MHz, DMSO-d₆):** *δ* = 8.65 (br s, 1H), 7.03 (s, 1H), 6.60 (dd, *J* = 2 Hz, 10.4 Hz, 1H), 6.03 (dd, *J* = 2.4 Hz, 12 Hz, 1H), 4.53-4.45 (m, 1H), 2.82 (t, *J* = 4.8 Hz, 2H), 2.74 (t, *J* = 6 Hz, 2H), 2.28 (s, 3H), 1.36 (d, *J* = 6.4 Hz, 6H).

### Example 25. Metabolic Stability in Human Liver Microsomes

Disclosed compounds were tested for stability in human liver microsomes (HLM), with the results summarized in **Table 1.** Disclosed compounds varied widely in their metabolic stability, but some trends were apparent. For example, compounds bearing a primary amine nitrogen or only a single methyl substitution on the amine were typically among the least stable compounds tested. Further, substitution at the 6 position of the indole, as in Compounds 26, 27, 28, 30, 35, 36, 37, and 38, typically reduced stability compared to the closest analogous compounds with a hydrogen at this position. This variable metabolic stability led certain disclosed compounds, for example, Compounds 4, 7, 27, 28, 35, and 36, to exhibit greater metabolic stability than very short-acting compounds, such as N,N-dimethyltryptamine (DMT), 5-methoxy-N,N-dimethyltryptamine (5-MeO-DMT), and 5-fluoro-N,N-dimethyltryptamine (5-F-DMT), and lesser metabolic stability than longer-acting compounds, such as, psilocin and 4-hydroxy-N-methyl-N-ethyltryptamine (4-HO-MET).

**Test Compounds.** Disclosed compounds were prepared as described above. All other compounds were commercially obtained.

**HLM Stability.** Pooled HLM from adult male and female donors (Corning 452117) were used. Microsomal incubations were carried out in multi-well plates. Liver microsomal incubation medium consisted of PBS (100 mM, pH 7.4), MgCl₂ (1 mM), and NADPH (1 mM), with 0.50 mg of liver microsomal protein per mL. Control incubations were performed by replacing the NADPH-cofactor system with PBS. Test compounds (1 µM, final solvent concentration 1.0%) were incubated with microsomes at 37 °C with constant shaking. Six time points over 60 minutes were analyzed, with 60 µL aliquots of the reaction mixture being drawn at each time point. The reaction aliquots were stopped by adding 180 µL of cold (4 °C) acetonitrile containing 200 ng/mL tolbutamide and 200 ng/mL labetalol as internal standards (IS), followed by shaking for 10 minutes, and then protein sedimentation by centrifugation at 4,000 rpm for 20 minutes at 4 °C. Supernatant samples (80 µL) were diluted with water (240 µL) and analyzed for parent compound remaining using a fit-for-purpose liquid chromatography-tandem mass spectrometry (LC-MS/MS) method.

**Data Analysis.** The elimination constant (kₑₗ), half-life (t_{1/2}), and intrinsic clearance (CLᵢₙₜ) were determined in a plot of ln(AUC) versus time, using linear regression analysis.

**Table 1. Intrinsic clearance (CLᵢₙₜ) and half-life (t_{1/2}) of compounds in the presence of HLM.**

| **Compound** | **Structure** | **Clᵢₙₜ (µL/min/mg)** | **t_{1/2} (min)** |
|---|---|---|---|
| DMT | 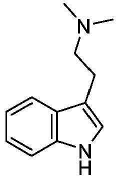 | 198.6 | 7.0 |
| Psilocin | 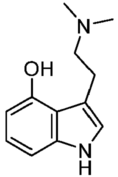 | 12.6 | 109.7 |
| 5-MeO-DMT | 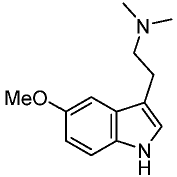 | 101.9 | 13.6 |
| 4 | 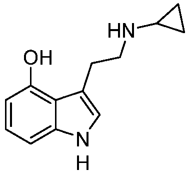 | 17 | 81.7 |
| 5-F-DMT | 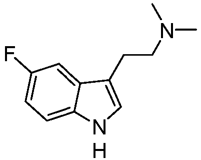 | 299.7 | 4.6 |
| 4-HO-MET | 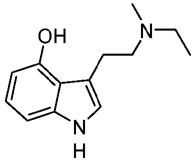 | <9.6 | >145 |
| 1 | 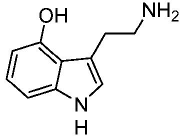 | 40.4 | 34.3 |
| 2 | 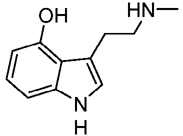 | 104.8 | 13.2 |
| 3 | 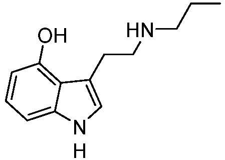 | 11.6 | 119.6 |
| 5 | 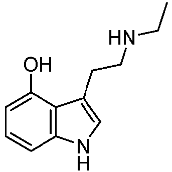 | <9.6 | >145 |
| 6 | 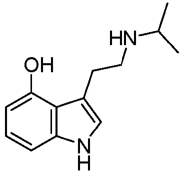 | <9.6 | >145 |
| 26 | 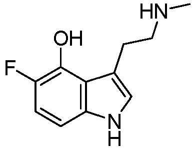 | 1517.7 | 0.9 |
| 7 | 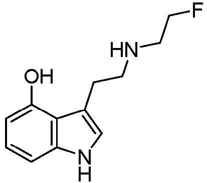 | 18 | 77 |
| 27 | 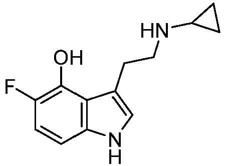 | 70 | 19.8 |
| 23 | 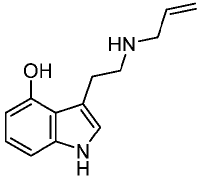 | 11.8 | 117.7 |
| 24 | 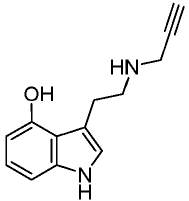 | 15.0 | 92.4 |
| 28 | 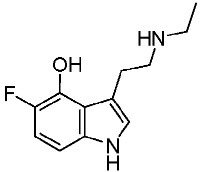 | 32.8 | 42.3 |
| 29 | 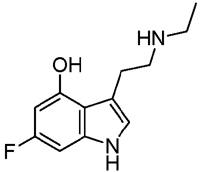 | <9.6 | >145 |
| 30 | 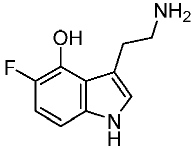 | 149.3 | 9.3 |
| 31 | 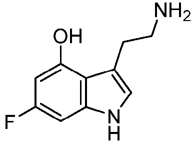 | 81.8 | 16.9 |
| 32 | 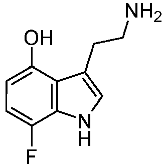 | 86.5 | 16.0 |
| 33 | 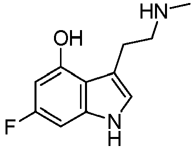 | 137.7 | 10.1 |
| 34 | 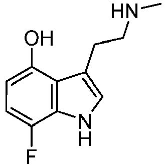 | 209.0 | 6.6 |
| 35 | 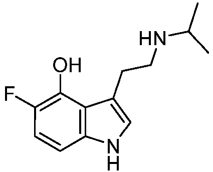 | 30.3 | 45.7 |
| 36 | 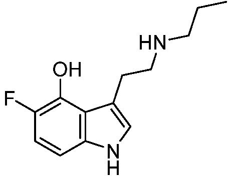 | 27.8 | 49.9 |
| 37 | 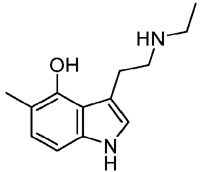 | 16.0 | 86.8 |
| 38 | 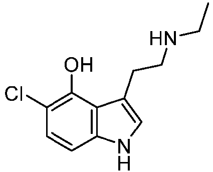 | 105.1 | 13.2 |
| 39 | 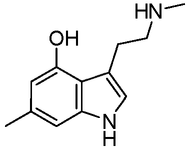 | 139.6 | 9.9 |
| 10 | 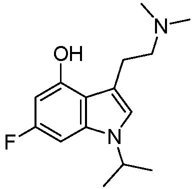 | 12.9 | 107.1 |
| 12 | 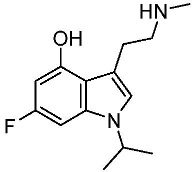 | <9.6 | >145 |

### Example 26. Pharmacokinetics in Mice

The pharmacokinetics (PK) of disclosed compounds were studied in the plasma of mice after subcutaneous (SC) administration (**Table 2** and **Figure 1**). Compound 4 reached much higher peak concentrations than 5-F-DMT and exhibited a half-life intermediate between that of the very short-acting compound 5-F-DMT and the longer-acting compound 4-HO-MET.

**Animals.** Male C57BL/6 mice, aged 8-12 weeks, were used in these studies. Four mice were housed in each cage. Temperature and humidity were maintained at 22 ± 3 °C and 30-70%, respectively, and illumination was controlled to give a 12 h light and 12 h dark cycle. Temperature and humidity were recorded by an auto-controlled data logger system. All animals were provided laboratory rodent diet. Reverse osmosis water treated with ultraviolet light was provided *ad libitum.* Animals were randomly assigned to treatment groups.

**Drugs.** Compound 4 was prepared as described above. All other compounds were commercially obtained. Test compounds were dissolved in a vehicle consisting of normal saline (for salts) or saline acidified with 1.3 molar equivalents of acetic acid (for free bases). They were then administered subcutaneously (SC) at a dose of 10 mg/kg (calculated based on the free base) and at a volume of 5 mL/kg body weight.

**Sample Collection and Bioanalysis.** Blood samples (approximately 60 µL) were collected under light isoflurane anesthesia (Surgivet^{®}) from the retro orbital plexus at 0.08, 0.25, 0.5, 1, 2, 4, 8, and 24 h (4 animals per time point). Immediately after blood collection, plasma was harvested by centrifugation at 4,000 rpm for 10 min at 4 °C and samples were stored at -70±10 °C until bioanalysis. Following blood collection, animals were immediately sacrificed. For bioanalysis, 25 µL aliquots of plasma study samples or spiked plasma calibration standards were added to individual pre-labeled micro-centrifuge tubes followed by 100 µL of an internal standard solution (glipizide, 500 ng/mL in acetonitrile) except for blanks, where 100 µL of acetonitrile was added. Samples were vortexed for 5 minutes and then centrifuged for 10 minutes at 4,000 rpm at 4 °C. Following centrifugation, 100 µL of each clear supernatant was transferred to a 96 well plate and analyzed with a fit-for-purpose LC-MS/MS method, with authentic samples of each analyte used for calibration and identification.

**Data Analysis.** Pharmacokinetic parameters were estimated using the non-compartmental analysis tool of Phoenix^{®} WinNonlin software (Ver 8.0).

**Table 2. Selected pharmacokinetic parameters of compounds in plasma of male C57BL/6 mice after subcutaneous (SC) administration (10 mg/kg).**

| **Compound** | **Tₘₐₓ (h)** | **Cₘₐₓ (ng/mL)** | **AUC₀₋ₗₐₛₜ (h*ng/mL)** | **t_{1/2} (h)** |
|---|---|---|---|---|
| 4 | 0.25 | 1224 | 665 | 0.62 |
| 5-F-DMT | 0.25 | 363 | 247 | 0.27 |
| 4-HO-MET | 0.08 | 1,159 | 1,412 | 1.1 |

### Example 27. Stability in the Presence of Monoamine Oxidases

Disclosed compounds were tested for stability in the presence of monoamine oxidase A and B (MAO-A and MAO-B) in human liver mitochondria preparations, with the results summarized in **Table 3.** Stability in this preparation was governed by a combination of both the indole and amine substituents. For compounds lacking a 4-hydroxy substituent on the indole, compounds with two methyl groups on the amine (e.g., DMT, 5-F-DMT, and 5-MeO-DMT) were rapidly metabolized by MAO, while those with at least one non-methyl substituent on the amine were more stable (e.g., *N-*methyl-*N*-ethyltryptamine (MET) and *N*-ethyl-*N*-propyltryptamine (EPT)). In contrast, 4-hydroxy compounds were generally more stable, as all such compounds with two alkyl groups on the amine (e.g., psilocin and 4-HO-MET) were stable, even when both groups were methyl. However, 4-hydroxy compounds with only a single amine substituent showed variable stability. For instance, *N-*methyl Compound 2 was highly unstable, whereas *N*-propyl Compound 3 was highly stable. Further, substitution at the 6 position of the indole, as in Compounds 26, 27, 28, 30, 35, 36, 37, and 38, typically reduced stability compared to the closest analogous compounds with a hydrogen at this position Interestingly, Compound 4 was of intermediate stability, consistent with its PK profile in mice, and suggesting utility as a psychedelic with intermediate duration of action. Several other compounds met this intermediate stability criterion, including Compounds 27, 28, 31, 32, 35, 38, and 10, but the exact substituents leading to a compound in this intermediate stability range were generally unpredictable.

**Test Compounds.** Disclosed compounds were prepared as described above. All other compounds were commercially obtained.

**Liver Mitochondria Incubations.** Human liver mitochondria (Xenotech H0610.M) were used. Mitochondrial incubations were carried out in multi-well plates. Liver mitochondrial incubation medium consisted of PBS (100 mM, pH 7.4) with 0.30 mg of liver mitochondrial protein per mL. Test compounds (1 µM, final solvent concentration 1.0%) were incubated with liver mitochondrial protein at 37 °C with constant shaking (total reaction volume 100 µL per well). Six time points over 60 minutes were analyzed. At each time point, reactions were stopped by adding 300 µL of cold (4 °C) acetonitrile containing 200 ng/mL tolbutamide and 200 ng/mL labetalol as internal standards (IS), followed by shaking for 10 minutes, and then protein sedimentation by centrifugation at 4,000 rpm for 20 minutes at 4 °C. Supernatant samples (100 µL) were diluted with 5% trichloroacetic acid in water (300 µL) and analyzed for parent compound remaining using a fit-for-purpose liquid chromatography-tandem mass spectrometry (LC-MS/MS) method.

**Data Analysis.** The elimination constant (kₑₗ), half-life (t_{1/2}), and intrinsic clearance (CLᵢₙₜ) were determined in a plot of ln(AUC) versus time, using linear regression analysis.

**Table 3. Intrinsic clearance (CLᵢₙₜ), half-life (t_{1/2}), and percent remaining of compounds in the presence of monoamine oxidases (human mitochondrial preparation).**

| **Compound** | **t_{1/2} (min)** | **Cl_{int(MAO)} (µL/min/mg)** | **Remaining (t = 60 min)** |
|---|---|---|---|
| DMT | 17.1 | 134.9 | 8.9% |
| MET | >145 | <15.9 | 84.5% |
| EPT | >145 | <15.9 | 95.1% |
| Psilocin | >145 | <15.9 | 112.1% |
| 5-MeO-DMT | 52.9 | 43.6 | 47.1% |
| 4 | 114.6 | 20.2 | 72.7% |
| 5-F-DMT | 8.7 | 266 | 0.9% |
| 4-HO-MET | >145 | <15.9 | 112.3% |
| 1 | 70.5 | 32.8 | 51.1% |
| 2 | 18.7 | 123.5 | 12.8% |
| 3 | >145 | <15.9 | 97.3% |
| 5 | >145 | <15.9 | 76.2% |
| 6 | >145 | <15.9 | 80.5% |
| 26 | 6.3 | 369.0 | 0.1% |
| 7 | >145 | <15.9 | 85.2% |
| 27 | 36.2 | 63.9 | 32.1% |
| 23 | >145 | <15.9 | 83.5% |
| 24 | >145 | <15.9 | 87.2% |
| 28 | 56.5 | 40.9 | 46.4% |
| 29 | >145 | <15.9 | 99.1% |
| 30 | 6.5 | 356.2 | 0.0% |
| 31 | 30.1 | 76.8 | 24.6% |
| 32 | 28.4 | 81.4 | 24.0% |
| 33 | 20.2 | 114.3 | 14.3% |
| 34 | 15.0 | 154.3 | 6.4% |
| 35 | 29.0 | 79.6 | 22.8% |
| 36 | 8.5 | 272.2 | 0.9% |
| 37 | 88.7 | 26.1 | 65.1% |
| 38 | 26.1 | 88.6 | 22.6% |
| 39 | 17.8 | 129.6 | 13.5% |
| 10 | 78.7 | 29.4 | 55.7% |
| 12 | >145 | <15.9 | 104.0% |

### Example 28. 5-HT2A Receptor Binding

The binding affinities of disclosed compounds at the ketanserin binding site of the 5-HT2A receptor were determined in radioligand binding experiments.

**Method.** Affinity of the test compounds for the 5-HT2A receptor was determined in radioligand binding experiments with [³H]ketanserin by WuXi AppTec (Hong Kong) Limited, using methods adapted from the literature and under conditions described in **Table 4.**

**Results.** Results of the radioligand binding assays are shown in **Table 5.** Interestingly, cyclopropyl Compound 4 was ~3-fold more potent than the n-propyl Compound 3.

**Table 4. Assay conditions for 5-HT2A receptor radioligand binding assay.**

| **Receptor Source** | HEK293 stable cell line |
|---|---|
| **Vehicle** | 1.0% DMSO |
| **Incubation Time** | 1 h |
| **Incubation Temperature** | 25 °C |
| **Incubation Buffer** | 50 mM Tris-HCl, pH 7.4 |
| **Ligand** | 1 nM [³H]ketanserin |
| **Non-Specific Ligand** | 1 µM ketanserin |

**Table 5. Results of 5-HT2A receptor binding affinity experiments.**

| **Compound** | ***5-HT2A Ki (nM) ([3H]Ketanserin)*** |
|---|---|
| MET | 127.89 |
| 5-F-DMT | 181.17 |
| 4-HO-MET | 106.29 |
| 3 | 1002.41 |
| 4 | 339.23 |
| DMT | 415.90 |
| psilocin | 58.58 |
| 5-MeO-DMT | 39.00 |
| 28 | 318.6 |
| 35 | 445.95 |
| 10 | 1190.1 |
| 12 | >1225.39 |

### Example 29. Functional Activity at Serotonin Receptors

Disclosed compounds were tested for agonist activity at several serotonin receptor subtypes (5-HT2A, 2-HT2B, 5-HT2C, and 5-HT1A) using Ca²⁺ flux functional assays, with the results summarized in **Table 6** All compounds exhibited potent agonist activity at 5-HT2A, suggestive of potential hallucinogenic activity as well as possible therapeutic effects. However, the potency and signaling efficacy at 5-HT2A, as well as the selectivity for this target over other serotonin receptors, varied dramatically with even small changes to the chemical structure. For example, cyclopropyl Compound 4 was more potent at 5-HT2A and more potent and efficacious at 5-HT1A compared to both the n-propyl Compound 3 and the isopropyl Compound 6. Similarly, isopropyl Compound 6 showed partial agonism at 5-HT2B, whereas cyclopropyl Compound 4 did not. Interestingly, Compounds 10 and 12 were both highly selective for the 5-HT2C receptor, exhibiting potent partial agonism at this target, over the 5-HT2A, where they exhibited no significant agonist effect up to 1,000 nM. This finding with Compound 10 contradicted a prior report (WO2006047032) that found this compound to be a selective agonist of the 5-HT2A receptor.

**Test Compounds.** Disclosed compounds were prepared as described above. All other compounds were commercially obtained.

**Functional Assays at 5-HT2A, 5-HT2B, and 5-HT1A.** Agonist activity at 5-HT2A, 5-HT2B, and 5-HT1A receptors was determined using a FLIPR Ca²⁺ flux assay at WuXi AppTec (Hong Kong) Limited according to their standard protocols. Briefly, stably transfected cells expressing the receptor of interest (HEK293 for 5-HT2A and 5-HT2B; CHO cells for 5-HT1A) were grown and plated in a 384 well plate and incubated at 37°C and 5% CO₂ overnight. A solution of 250 mM probenecid in 1mL FLIPR assay buffer was prepared fresh. This was combined with a fluorescent dye (Fluo-4 DirectTM) to make a final assay concentration of 2.5 mM. Compounds were diluted 1:3.16 for 10 points and 750 nL was added to a 384 well compound plate using ECHO along with 30 µL assay buffer. The fluorescent dye was then added to the assay plate along with assay buffer to a final volume of 40 µL. The cell plate was incubated for 50 min at 37 °C and 5% CO₂ and placed into the FLIPR Tetra along with the compound plate. 10 µL of references and compounds were then transferred from the compound plate into the cell plate and the fluorescent signal was read.

**Functional Assays at 5-HT2C.** Agonist activity at 5-HT2C was determined using a FLIPR Ca²⁺ flux assay at Eurofins DiscoverX (Fremont, CA) according to their standard protocols. Briefly, stably transfected cells expressing the human 5-HT2C receptor were grown and plated in a 384 well plate and incubated at 37 °C and 5% CO₂ overnight. Assays were performed in 1x Dye Loading Buffer consisting of 1x Dye, 1x Additive A, and 2.5 mM Probenecid in HBSS / 20 mM Hepes. Probenecid was prepared fresh. Cells were loaded with dye prior to testing and incubated at 37 °C for 30-60 minutes. After dye loading, cells were removed from the incubator and 10 µL HBSS / 20 mM Hepes was added. 3x vehicle was included in the assay buffer. Cells were incubated for 30 mins at room temperature in the dark to equilibrate plate temperature. Intermediate dilution of sample stocks was performed to generate 4x sample in assay buffer. Compound agonist activity was measured on a FLIPR Tetra (MDS). Calcium mobilization was monitored for 2 minutes and 10 µL 4X sample in HBSS / 20 mM Hepes was added to the cells 5 seconds into the assay.

**Table 6. Agonist activity of compounds at select serotonin receptors in Ca²⁺ flux functional assays.**

| **Compound** | **5-HT2A EC₅₀ (nM)** | **5-HT2A %Act @ Max Dose** | **5-HT2B EC₅₀ (nM)** | **5-HT2B %Act @ Max Dose** | **5-HT2C EC₅₀ (nM)** | **5-HT2C %Act @ Max Dose** | **5-HT1A EC₅₀ (nM)** | **5-HT1A %Act @ Max Dose** |
|---|---|---|---|---|---|---|---|---|
| EPT | 30.52 | 85.2 | 40.29 | 80.4 | 33.41 | 90.8 | 88,685 | 75.5 |
| MET | 17.12 | 36.2 | ND | 11.9 | 28.32 | 89.7 | 57,892 | 71.4 |
| 5-F-DMT | 6.94 | 94.7 | 9.71 | 17.6 | 4.94 | 93.9 | 5,716 | 72.9 |
| 4-HO-MET | 15.14 | 103.0 | ND | 1.1 | 37.76 | 95.0 | 14,709 | 73.0 |
| 1 | 33.37 | 99.2 | 151.1 | 83.98 | NT | NT | 92,637 | 49.9 |
| 2 | 15.84 | 89.4 | ND | 1.79 | NT | NT | 22,979 | 64.8 |
| 3 | 29.26 | 90.1 | ND | 1.06 | NT | NT | 33,107 | 10.7 |
| 4 | 8.70 | 95.2 | ND | 4.08 | NT | NT | 6,564 | 58.72 |
| 5 | 14.01 | 107.9 | 275.6 | 18.58 | NT | NT | 27,184 | 38.71 |
| 6 | 22.46 | 83.8 | 998.6 | 21.48 | NT | NT | >100,00 0 | 10.15 |
| DMT | 22.2 | 93.4 | ND | 10.4 | 31.3 | 92.0 | >100,00 0 | 68.1 |
| psilocin | 6.50 | 95.6 | 4,290 | 1.44 | 30.3 | 95.1 | ND | 0.720 |
| 5-MeO-DMT | 1.76 | 106 | 30.1 | 20.5 | 10.1 | 89.8 | 280 | 78.8 |
| 26 | 21.02 | 86.9 | ND | 13.87 | NT | NT | 3,493 | 74.21 |
| 7 | 47.23 | 86.8 | 374.7 | 20.76 | NT | NT | >100,00 0 | 42.13 |
| 27 | 7.367 | 98.2 | 9.531 | 36.08 | NT | NT | 1,230 | 84.02 |
| 23 | 15.88 | 85.46 | 89.02 | 47.88 | NT | NT | >100,00 0 | 9.13 |
| 24 | 20.07 | 96.07 | 88.27 | 48.26 | NT | NT | >100,00 0 | -3.14 |
| 28 | 10.51 | 92.60 | 38.63 | 19.83 | 69.73 | 99.03 | 9,206 | 60.29 |
| 29 | 17.36 | 80.80 | ND | 12.18 | NT | NT | >10,000 | 16.22 |
| 30 | 4.06 | 100.15 | 4.62 | 95.46 | NT | NT | 5,216 | 73.4 |
| 31 | 6.360 | 99.00 | 5.07 | 99.12 | NT | NT | 12,457 | 18.3 |
| 32 | 7.30 | 94.56 | 30.33 | 66.55 | NT | NT | >100,00 0 | 38.64 |
| 33 | 5.80 | 79.51 | 28.48 | 31.76 | NT | NT | >100,00 0 | 3.51 |
| 34 | 16.23 | 77.43 | >10000 | 8.09 | NT | NT | >100,00 0 | 36.72 |
| 35 | 45.73 | 94.57 | 95.87 | 27.19 | 323.6 | 74.81 | 93,794 | 48.74 |
| 36 | 12.09 | 71.78 | 26.17 | 44.90 | NT | NT | >100,00 0 | 15.31 |
| 37 | 56.48 | 91.0 | 274.20 | 16.28 | NT | NT | 6,948 | 64.6 |
| 38 | 10.48 | 103.70 | 42.41 | 43.30 | NT | NT | 1,837 | 77.96 |
| 39 | 78.38 | 76.43 | >10,000 | 10.04 | NT | NT | >100,00 0 | 4.4 |
| 10 | >1,000 | 1.28 | >10,000 | 1.99 | 30.07 | 43.06 | >100,00 0 | 1.85 |
| 12 | >1,000 | 10.55 | >10,000 | 1.74 | 25.31 | 57.17 | >100,00 0 | 3.96 |

| | | | | | | | | |
|---|---|---|---|---|---|---|---|---|
| NT = not tested; ND = not determined due to lack of measurable agonism. | | | | | | | | |

### Example 30. Stability in Mouse Brain Homogenate

Disclosed compounds were tested for stability in mouse brain homogenate **(Table 7).** There was widely variable stability among the test compounds under the conditions of the experiment. Interestingly, Compound 4 was of intermediate stability, consistent with its PK profile in mice, and suggesting utility as a psychedelic with intermediate duration of action. Among the compounds bearing an N-propyl group, Compound 4 was the least stable. More generally, there tended to be a rough correlation between stability in mouse brain homogenate and stability in the presence of monoamine oxidases (see Example 27).

**Test Compounds.** Disclosed compounds were prepared as described above. All other compounds were commercially obtained.

**Mouse Brain Homogenate Stability.** Frozen mouse brain homogenate (pooled from male CD-1 mice, BioreclamationIVT, MSE00BRAINMZA) was thawed in a water bath at 37 °C immediately prior to use. Positive controls and test compounds (final concentration in incubation medium = 1 µM for test compounds and 2 µM for controls, all with 2% DMSO) were incubated in duplicate for each time point (0, 10, 30, 60, and 120 min) in the mouse brain homogenate at a total reaction volume of 100 µL at 37 °C. At the end of each incubation period, reactions were immediately quenched with 400 µL of acetonitrile containing internal standard (200 ng/mL tolbutamine and 200 ng/mL labetalol) and mixed thoroughly. Plates were then sealed, shaken for 20 min, and centrifuged at 4,000 rpm and 4 °C for 20 min. Aliquots of 50 µL of each supernatant were diluted into 100 uL of water and the mixtures were then shaken again for 10 min. The resulting mixtures were analyzed for parent compound remaining using a fit-for-purpose LC-MS/MS method.

**Table 7. Stability of compounds in mouse brain homogenate.**

| **Compound** | **% Compound Remaining (after 120 min)** | **t_{1/2} (min)** |
|---|---|---|
| DMT | 0.0 | 6.4 |
| 5-MeO-DMT | 91.2 | >289.1 |
| psilocin | 97.6 | >289.1 |
| 4 | 19.4 | 53.0 |
| 5-F-DMT | 0.0 | 3.1 |
| 4-HO-MET | 16.7 | 45.8 |
| 1 | 0.0 | 12.6 |
| 2 | 0.2 | 14.0 |
| 3 | 29.2 | 72.6 |
| 5 | 67.2 | 183.3 |
| 6 | 62.6 | 162.9 |
| 26 | 0 | 1.7 |
| 7 | 61.8 | 177.0 |
| 27 | 29.4 | 69.6 |
| 23 | 49.6 | 126.8 |
| 24 | 40.5 | 97.3 |
| 28 | 1.9 | 22.1 |
| 29 | 29.9 | 59.9 |
| 30 | 0 | 6.4 |
| 31 | 1.6 | 20.3 |
| 32 | 4.8 | 29.0 |
| 33 | 0.6 | 16.0 |
| 34 | 1.6 | 21.7 |
| 35 | 40 | 90.5 |
| 36 | 46.2 | 108.0 |
| 37 | 31.3 | 69.9 |
| 38 | 0.9 | 17.5 |
| 39 | 0.4 | 15.5 |
| 10 | 80.9 | >289.1 |
| 12 | 72.3 | 263.5 |

### Example 31. Stability in Rat Brain Homogenate

Disclosed compounds were tested for stability in rat brain homogenate **(Table 8).** There was widely variable stability among the test compounds under the conditions of the experiment. Interestingly, Compound 4 was of intermediate stability, consistent with its PK profile in mice, and suggesting utility as a psychedelic with intermediate duration of action. Among the compounds bearing an N-propyl group, Compound 4 was the least stable. More generally, there tended to be a rough correlation between stability in rat brain homogenate and stability in the presence of monoamine oxidases (see Example 27). Further, as with stability in the presence of monoamine oxidases (see Example 27), substitution at the 6 position of the indole, as in Compounds 26, 27, 28, 30, 35, 36, 37, and 38, typically reduced stability compared to the closest analogous compounds with a hydrogen at this position.

**Test Compounds.** Disclosed compounds were prepared as described above. All other compounds were commercially obtained.

**Rat Brain Homogenate Stability.** Frozen rat brain homogenate (pooled from male Sprague Dawley rats, BioreclamationIVT, RATOOBRAINMZA) was thawed in a water bath at 37 °C immediately prior to use. Positive controls and test compounds (final concentration in incubation medium = 1 µM for test compounds and 2 µM for controls, all with 2% DMSO) were incubated in duplicate for each time point (0, 10, 30, 60, and 120 min) in the rat brain homogenate at a total reaction volume of 100 µL at 37 °C. At the end of each incubation period, reactions were immediately quenched with 400 µL of acetonitrile containing internal standard (200 ng/mL tolbutamine and 200 ng/mL labetalol) and mixed thoroughly. Plates were then sealed, shaken for 20 min, and centrifuged at 4,000 rpm and 4 °C for 20 min. Aliquots of 50 µL of each supernatant were diluted into 100 uL of water and the mixtures were then shaken again for 10 min. The resulting mixtures were analyzed for parent compound remaining using a fit-for-purpose LC-MS/MS method.

**Table 8. Stability of compounds in rat brain homogenate.**

| **Compound** | **% Compound Remaining (after 120 min)** | **t_{1/2} (min)** |
|---|---|---|
| DMT | 0.0 | 5.1 |
| 5-MeO-DMT | 0.3 | 14.4 |
| Psilocin | 46.1 | 114.8 |
| 4 | 25.2 | 63.3 |
| 5-F-DMT | 0.0 | 2.7 |
| 4-HO-MET | 20.9 | 53.0 |
| 1 | 0.0 | 10.0 |
| 2 | 0.2 | 6.3 |
| 3 | 52.5 | 136.9 |
| 5 | 61.5 | 157.9 |
| 6 | 53.8 | 136.2 |
| 26 | 0 | 2.2 |
| 7 | 77.1 | >289.1 |
| 27 | 0.4 | 15.1 |
| 23 | 52.4 | 126.1 |
| 24 | 41.9 | 92.4 |
| 28 | 0.4 | 15.4 |
| 29 | 51.9 | 113.4 |
| 30 | 0.1 | 3.9 |
| 31 | 0 | 11.4 |
| 32 | 0 | 9.5 |
| 33 | 0.4 | 9.1 |
| 34 | 0 | 3.6 |
| 35 | 16.4 | 45.6 |
| 36 | 7.1 | 31.0 |
| 37 | 0.2 | 14.1 |
| 38 | 0 | 9.2 |
| 39 | 0.1 | 6.3 |
| 10 | 71.4 | 269.2 |
| 12 | 70.4 | 248.6 |

### Example 32. Serotonin Release Activity in Synaptosomes

Disclosed compounds were assessed for their ability to release serotonin (5-HT) from rat synaptic vesicles **(Table 9).** Potency and efficacy for 5-HT release varied substantially depending on the specific compound. Compounds with a 4-hydroxy substituent generally exhibited decreased potency and efficacy for 5-HT release compared to compounds with no substituents or only a single fluoro substituent on the indole ring system. However, there was also substantial variability among 4-hydroxy compounds. For example, cyclopropyl Compound 4 was much more potent than n-propyl Compound 3. Similarly, fluorinated Compounds 28 and 35 were significantly more potent and efficacious than their non-fluorinated analogs 5 and 6, respectively.

**Test Compounds.** Disclosed compounds were prepared as described above. All other compounds were commercially obtained.

**Synaptosome 5-HT Release Assay.** Synaptosome release assays were conducted according to modifications of previously described procedures (Partilla et al. (2016). Interrogating the Activity of Ligands at Monoamine Transporters in Rat Brain Synaptosomes. In Neurotransmitter Transporters (pp. 41-52). Springer.). Briefly, synaptosomes were prepared from rat brains. Male Sprague-Dawley rats were rendered unconscious with CO₂ and their brains were immediately removed. The cerebella were discarded and the whole brains (minus striatum) were placed in ice-cold 0.32 M sucrose (10 mL per brain) and gently homogenized by hand. The homogenate of each brain was centrifuged at 1,000 x g at 4 °C for 10 mins and the resulting supernatant was diluted with ice-cold 0.32 M sucrose to a total volume of 10 mL to provide the synaptosome solution. Synaptosomes were then preloaded with 5 nM [³H]5-HT in the presence of selective uptake inhibitors of DAT (50 nM GBR12935), NET (100 nM nomifensine), and VMAT2 (1 µM reserpine) in Krebs phosphate buffer (KPB). The incubations were allowed to reach equilibrium for 2 h at 25 °C. For the release reactions, 425 µL of preloaded synaptosomes were added to test tubes containing 75 µL of test drugs diluted in KPB containing 1 mg/mL BSA. After 10 mins, the release reaction was stopped using a cell harvester by rapid vacuum filtration over GF/B filter paper presoaked in wash buffer (10 mM Tris-HCl, pH 7.4, 150 mM NaCl) and the filters were washed with additional wash buffer. Filters were dried for 1 h at 60 °C and retained radioactivity was quantified using a MicroBeta 2 liquid scintillation counter. The amount of retained radioactivity was inversely proportional to the extent of release.

**Table 9. Effect of compounds on 5-HT release from rat synaptosomes.**

| **Compound** | **5-HT Release EC₅₀ (nM)** | **% Release @ Max Dose (10 µM)** |
|---|---|---|
| DMT | 80.76 | 80.77 |
| EPT | 264.5 | 45.02 |
| MET | 182.7 | 71.00 |
| 5-F-DMT | 40.67 | 81.44 |
| 4-HO-MET | 496.3 | 56.9 |
| 4 | 1,254 | 38.87 |
| 3 | >10,000 | 36.06 |
| 5 (4024) | 4,064 | 53.8 |
| 6 (4025) | >10,000 | 32.8 |
| 28 (4046) | 708.4 | 73.6 |
| 35 (4059) | 6,940 | 53.2 |

### Example 33. Pharmacokinetics in Rats

The pharmacokinetics (PK) of disclosed compounds were studied in the plasma of rats after intravenous (IV) administration (**Table 10**). Half-life in rats generally paralleled stability in the presence of monoamine oxidases (see Example 27), with compounds having very low monoamine oxidase stability, such as 5-F-DMT and Compound 2, having very short half-lives, while compounds with high monoamine oxidase stability, such as psilocin, having much longer half-lives. Compounds 28 and 35, which had intermediate monoamine oxidase stability, also exhibited a half-life intermediate between that of the very short-acting compound 5-F-DMT and the longer-acting compound psilocin, suggesting their potential utility as intermediate duration psychedelic therapies.

**Animals.** Male Sprague Dawley rat, aged 8-12 weeks, were used in these studies. Four rats were housed in each cage. Temperature and humidity were maintained at 22 ± 3 °C and 30-70%, respectively, and illumination was controlled to give a 12 h light and 12 h dark cycle. Temperature and humidity were recorded by an auto-controlled data logger system. All animals were provided laboratory rodent diet. Reverse osmosis water treated with ultraviolet light was provided *ad libitum.* Animals were randomly assigned to treatment groups.

**Drugs.** Disclosed compounds were prepared as described above. All other compounds were commercially obtained. Test compounds were dissolved in a vehicle consisting of normal saline (for salts) or saline acidified with 1.3 molar equivalents of acetic acid (for free bases). They were then administered intravenously (IV) at a dose of 1 mg/kg (calculated based on the free base) and at a volume of 5 mL/kg body weight.

**Sample Collection and Bioanalysis.** Blood samples (approximately 120 µL) were collected under light isoflurane anesthesia (Surgivet^{®}) from the retro orbital plexus at 0.08, 0.25, 0.5, 1, 2, and 4 h (4 animals per time point, 4 h time point only for psilocin). Immediately after blood collection, plasma was harvested by centrifugation at 10,000 rpm for 10 min at 4 °C and samples were stored at -70±10 °C until bioanalysis. Following blood collection, animals were immediately sacrificed. For bioanalysis, 20 µL aliquots of plasma study samples or spiked plasma calibration standards were added to individual pre-labeled micro-centrifuge tubes followed by 200 µL of an internal standard solution (Cetrizine or Rosuvastatin, 50 ng/mL) except for blanks, where 200 µL of acetonitrile was added. Samples were vortexed for 5 minutes and then centrifuged for 10 minutes at 2,500 or 4,000 rpm at 4 °C. Following centrifugation, 200 µL of each clear supernatant was transferred to a 96 well plate and analyzed with a fit-for-purpose LC-MS/MS method, with authentic samples of each analyte used for calibration and identification.

**Data Analysis.** Pharmacokinetic parameters were estimated using the non-compartmental analysis tool of Phoenix^{®} WinNonlin software (Ver 8.0).

**Table 10. Selected pharmacokinetic parameters of compounds in plasma of male Sprague-Dawley rats after intravenous (IV) administration (1 mg/kg).**

| **Compound** | **C₀ (ng/mL)** | **AUC₀₋ₗₐₛₜ (h*ng/mL)** | **t_{1/2} (h)** |
|---|---|---|---|
| 5-F-DMT | 338 | 35.5 | 0.08 |
| psilocin | 179 | 146 | 1.56 |
| 2 | 185 | 21.0 | 0.18 |
| 5 | 350 | 161 | 0.65 |
| 28 | 249 | 86.7 | 0.46 |
| 35 | 348 | 126 | 0.44 |

### Example 34. 5-HT2C Receptor Binding

The binding affinities of disclosed compounds at the mesulergine binding site of the 5-HT2C receptor were determined in radioligand binding experiments.

**Method.** Affinity of the test compounds for the 5-HT2C receptor was determined in radioligand binding experiments with [³H]mesulergine by WuXi AppTec (Hong Kong) Limited, using methods adapted from the literature and under conditions described in **Table 11.**

**Results.** Results of the radioligand binding assays are shown in **Table 12.** Consistent with their high selectivity for 5-HT2C receptor over 5-HT2A receptor in functional assays (see Example 29), Compounds 10 and 12 exhibited potent binding to the 5-HT2C receptor that was much more potent than that observed at the 5-HT2A receptor (see Example 28). Interestingly, this finding with Compound 10 contradicted a prior report (WO2006047032) that found this compound to be a selective agonist of the 5-HT2A receptor.

**Table 11. Assay conditions for 5-HT2C receptor radioligand binding assay.**

| | |
|---|---|
| **Receptor Source** | HEK293 stable cell line |
| **Vehicle** | 0.5% DMSO |
| **Incubation Time** | 1 h |
| **Incubation Temperature** | 25 °C |
| **Incubation Buffer** | 50 mM Tris-HCl, pH 7.4, 10 mM |
| | MgCl₂, 1 mM EDTA, 0.1% BSA |
| **Ligand** | 2 nM [³H]mesulergine |
| **Non-Specific Ligand** | 1 µM SB 206553 |

**Table 12. Results of 5-HT2C receptor binding affinity experiments.**

| **Compound** | ***5-HT2C Ki (nM) ([3H]Mesulergine)*** |
|---|---|
| 10 | 10.5 |
| 12 | 30.8 |

### Example 35. Synthesis of Additional Compounds

Additional disclosed compounds may be prepared by standard methods known to those skilled in the art of organic synthesis, for example, those presented in **Schemes 1-5** and **Examples 1-24.**

While there have been shown and described what are at present considered the preferred embodiments of the invention, those skilled in the art may make various changes and modifications which remain within the scope of the invention defined by the appended claims.
The present invention is further defined by the following items:
1. A compound having the following structure: 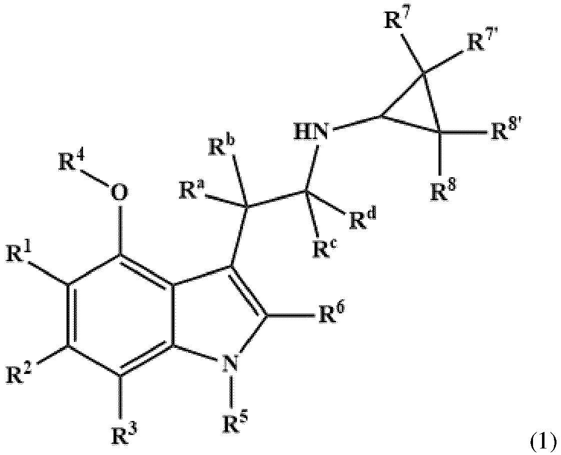 wherein:
   R¹, R², R³, and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms;
   R⁴ is selected from the group consisting of H and esterifying groups that result in OR⁴ being an ester group;
   R⁵ is selected from the group consisting of H and hydrocarbon groups containing 1-5 carbon atoms (R);
   R⁷, R^{7'}, R⁸, and R^{8'} are independently selected from H and F atoms;
   R^{a}, R^{b}, R^{c}, and R^{d} are independently selected from H and D atoms;
   and pharmaceutically acceptable salts thereof.
2. The compound of item 1, wherein R⁴ is H.
3. The compound of item 1, wherein R⁴ is an esterifying group that results in OR⁴ being an ester group.
4. The compound of item 3, wherein R⁴ is -C(O)R to result in OR⁴ being -OC(O)R, wherein R is defined in item 1.
5. The compound of item 3, wherein R⁴ is -P(O)(OR')₂, wherein R' is independently selected from H and R, to result in OR⁴ being -OP(O)(OR')₂, wherein R is defined in item 1.
6. The compound of item 1, wherein the compound has the following structure: 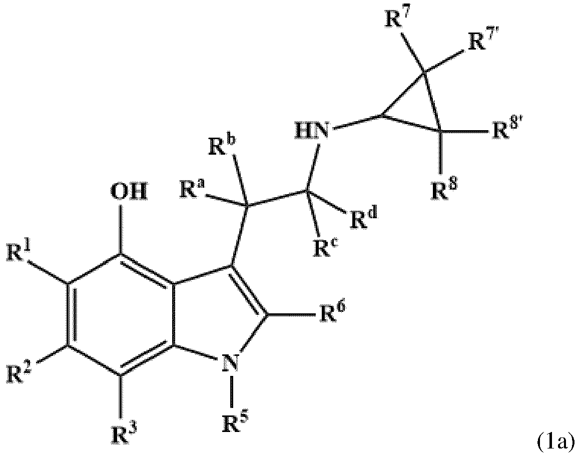 and pharmaceutically acceptable salts thereof.
7. The compound of item 1, wherein the compound has the following structure: 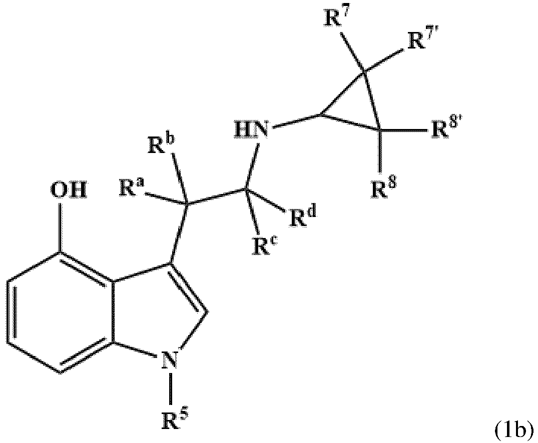 and pharmaceutically acceptable salts thereof.
8. The compound of item 1, wherein the compound has the following structure: 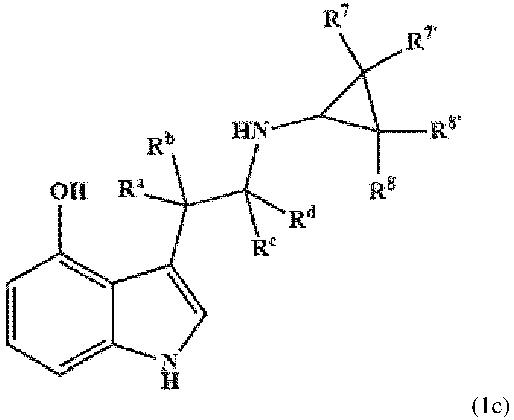 and pharmaceutically acceptable salts thereof.
9. The compound of item 1, wherein the compound has the following structure: 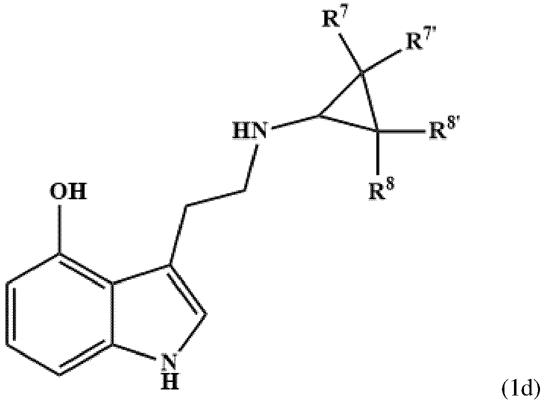 and pharmaceutically acceptable salts thereof.
10. The compound of item 1, wherein the compound has the following structure: 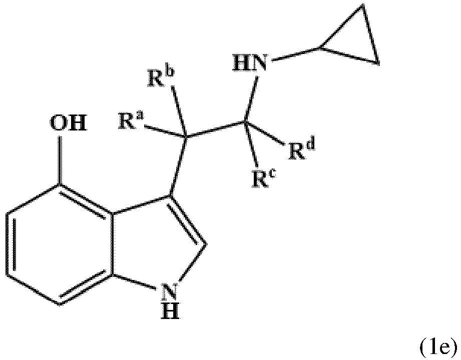 and pharmaceutically acceptable salts thereof.
11. The compound of item 1, wherein the compound has the following structure: 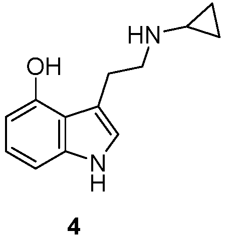 and pharmaceutically acceptable salts thereof.
12. The compound of item 1, wherein the compound has the following structure: 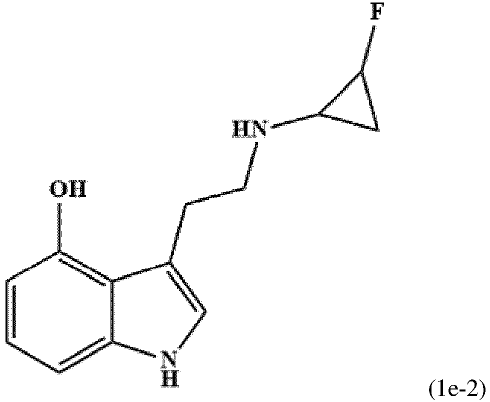 and pharmaceutically acceptable salts thereof.
13. The compound of item 1, wherein the compound has the following structure: 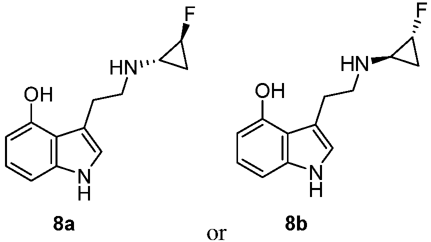 and pharmaceutically acceptable salts thereof.
14. The compound of item 1, wherein the compound has the following structure: 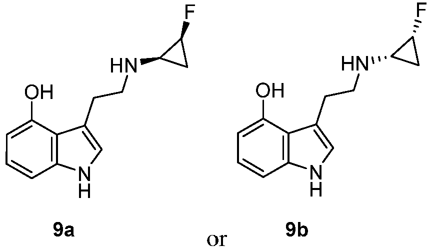 and pharmaceutically acceptable salts thereof.
15. A compound having the following structure: 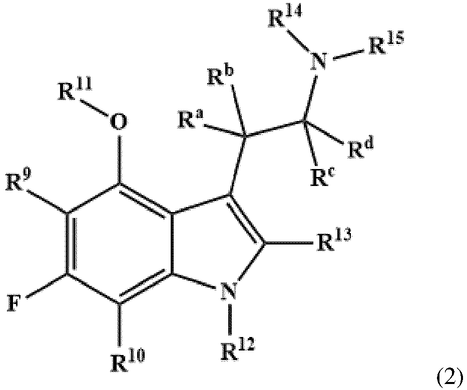 wherein:
   R⁹, R¹⁰, and R¹³ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms;
   R¹¹ is selected from the group consisting of H and esterifying groups that result in OR¹¹ being an ester group;
   R¹² is isopropyl or cyclopropyl with optional substitution with one or more fluorine atoms;
   R¹⁴ and R¹⁵ are independently selected from the group consisting of H and hydrocarbon groups containing 1-5 carbon atoms (R), which are optionally substituted with one or more fluorine atoms;
   R^{a}, R^{b}, R^{c}, and R^{d} are independently selected from H and D atoms;
   provided that, when R⁹, R¹⁰, R¹¹, R¹³, R^{a}, R^{b}, R^{c}, and R^{d} are H and R¹² is isopropyl, then R¹⁴ and R¹⁵ are not both methyl;
   and pharmaceutically acceptable salts thereof.
16. The compound of item 15, wherein R¹¹ is H.
17. The compound of item 15, wherein R¹¹ is an esterifying group that results in OR¹¹ being an ester group.
18. The compound of item 17, wherein R¹¹ is -C(O)R to result in OR¹¹ being -OC(O)R, wherein R is defined as in item 15.
19. The compound of item 17, wherein R¹¹ is -P(O)(OR')₂, wherein R' is independently selected from H and R, to result in OR¹¹ being -OP(O)(OR')₂, wherein R is defined as in item 15.
20. The compound of item 15, wherein the compound has the following structure: 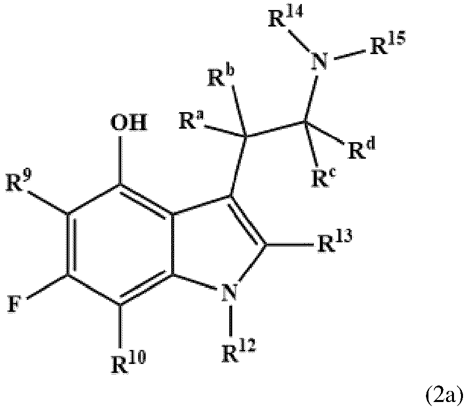 and pharmaceutically acceptable salts thereof.
21. The compound of item 15, wherein the compound has the following structure: 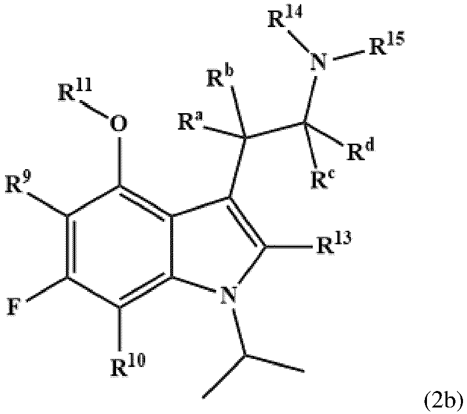 and pharmaceutically acceptable salts thereof.
22. The compound of item 15, wherein the compound has the following structure: 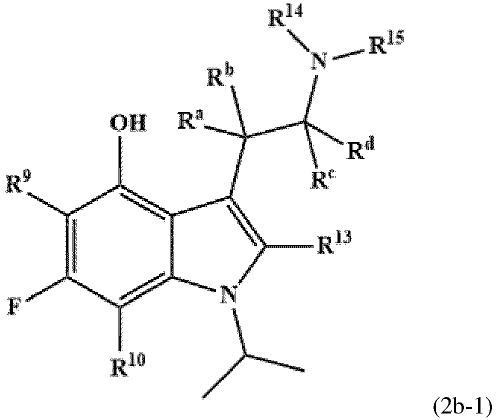 and pharmaceutically acceptable salts thereof.
23. The compound of item 15, wherein the compound has the following structure: 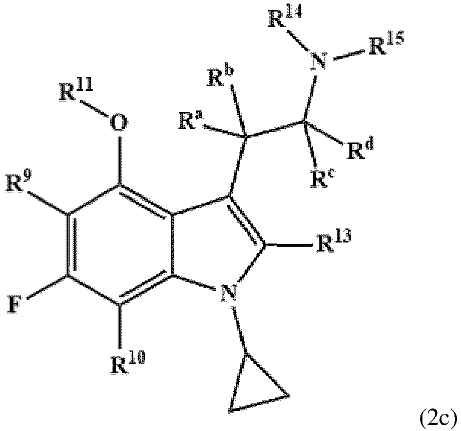 and pharmaceutically acceptable salts thereof.
24. The compound of item 15, wherein the compound has the following structure: 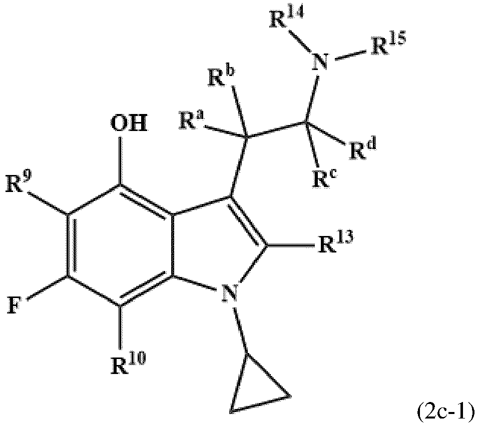 and pharmaceutically acceptable salts thereof.
25. The compound of item 15, wherein the compound is selected from the group consisting of the following compounds: 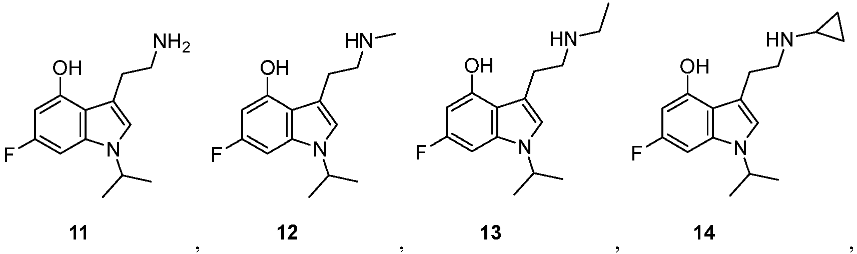 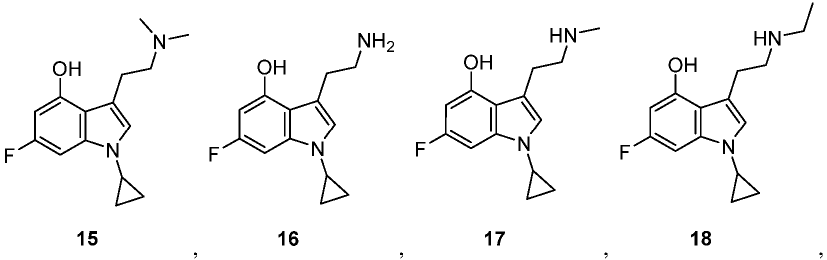 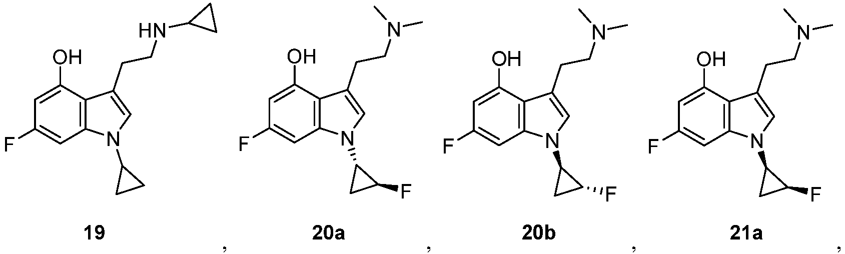 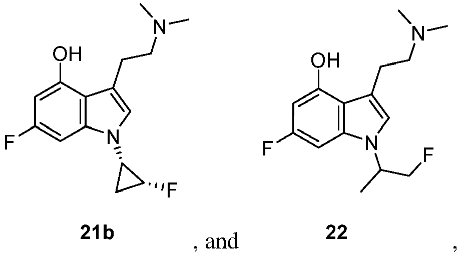 and pharmaceutically acceptable salts thereof.
26. A compound having the following structure: 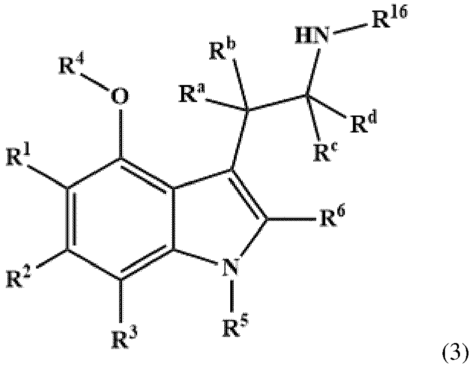 wherein:
   R¹, R², R³, and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms;
   R⁴ is selected from the group consisting of H and esterifying groups that result in OR⁴ being an ester group;
   R⁵ is selected from the group consisting of H and hydrocarbon groups containing 1-5 carbon atoms (R);
   R^{a}, R^{b}, R^{c}, and R^{d} are independently selected from H and D atoms;
   R¹⁶ is an allyl or propargyl group;
   provided that, when R¹, R², R³, R⁴, R⁵, R⁶, R^{a}, R^{b}, R^{c}, and R^{d} are all H, then R¹⁶ is not allyl;
   and pharmaceutically acceptable salts thereof.
27. The compound of item 26, wherein R⁴ is H.
28. The compound of item 26, wherein R⁴ is an esterifying group that results in OR⁴ being an ester group.
29. The compound of item 28, wherein R⁴ is -C(O)R to result in OR⁴ being -OC(O)R, wherein R is defined as in item 26.
30. The compound of item 28, wherein R⁴ is -P(O)(OR')₂, wherein R' is independently selected from H and R, to result in OR⁴ being -OP(O)(OR')₂, wherein R is defined as in item 26
31. The compound of item 26, wherein the compound has the following structure: 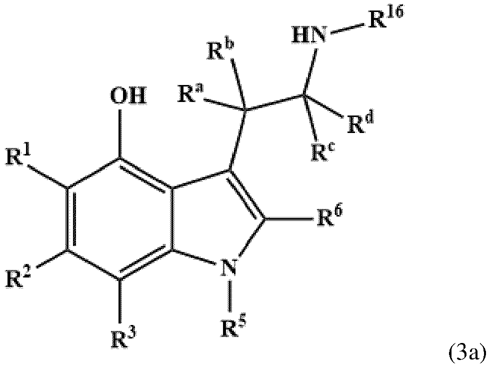 and pharmaceutically acceptable salts thereof.
32. The compound of item 26, wherein the compound has the following structure: 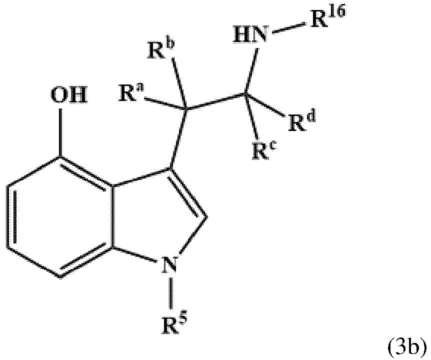 and pharmaceutically acceptable salts thereof.
33. The compound of item 26, wherein the compound has the following structure: 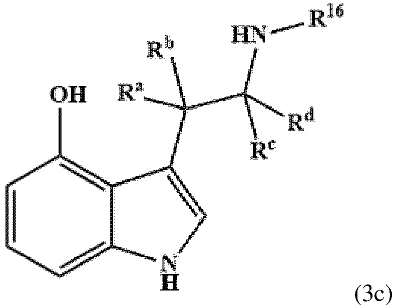 and pharmaceutically acceptable salts thereof.
34. The compound of item 26, wherein the compound has the following structure: 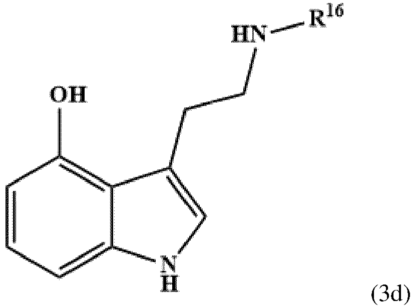 and pharmaceutically acceptable salts thereof.
35. The compound of item 26, wherein the compound has the following structure: 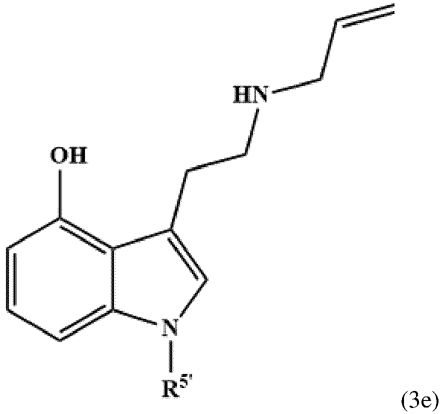
   wherein R^{5'} is selected from hydrocarbon groups containing 1-5 carbon atoms (R);
   and pharmaceutically acceptable salts thereof.
36. The compound of item 26, wherein the compound has the following structure: 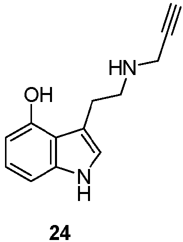 and pharmaceutically acceptable salts thereof.
37. A compound having the following structure: 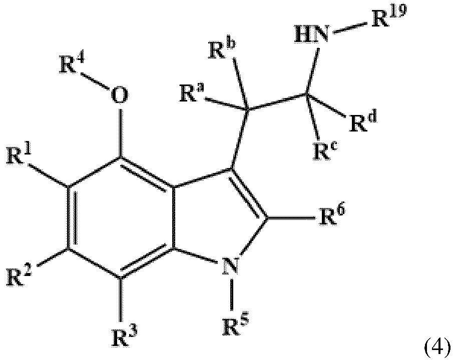 wherein:
   R¹ is selected from the group consisting of: F, Cl, and methyl;
   R², R³, and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms;
   R⁴ is selected from the group consisting of H and esterifying groups that result in OR⁴ being an ester group;
   R⁵ is selected from the group consisting of H and hydrocarbon groups containing 1-5 carbon atoms (R);
   R^{a}, R^{b}, R^{c}, and R^{d} are independently selected from H and D atoms;
   R¹⁹ is selected from the group consisting of H and hydrocarbon groups containing 1-5 carbon atoms (R), which are optionally substituted with one or more fluorine atoms;
   provided that, when R¹ is F or methyl, and R², R³, R⁴, R⁶, R¹⁹, R^{a}, R^{b}, R^{c}, and R^{d} are all H, then R⁵ is not H or methyl;
   further provided that, when R¹ is Cl, and R², R³, R⁴, R⁶, R¹⁹, R^{a}, R^{b}, R^{c}, and R^{d} are all H, then R⁵ is not H, methyl, or ethyl;
   further provided that, when R¹ is F, and R², R³, R⁴, R⁶, R^{a}, R^{b}, R^{c}, and R^{d} are all H, then R⁵ and R¹⁹ are not both methyl;
   and pharmaceutically acceptable salts thereof.
38. The compound of item 37, wherein the compound has the following structure: 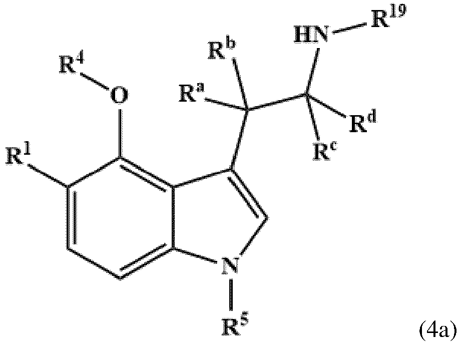 and pharmaceutically acceptable salts thereof.
39. The compound of item 37, wherein the compound has the following structure: 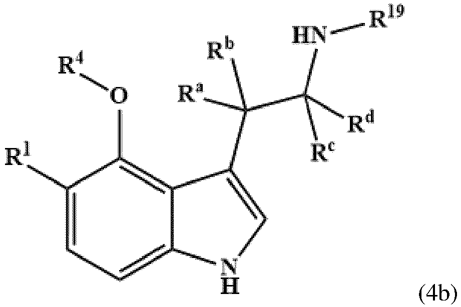 and pharmaceutically acceptable salts thereof.
40. The compound of item 37, wherein the compound has the following structure: 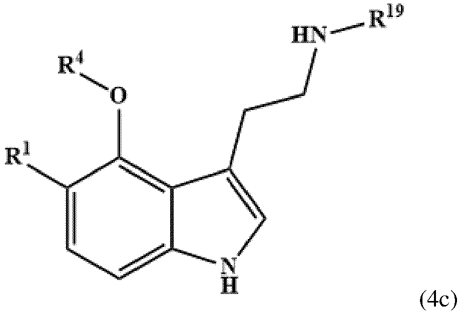 and pharmaceutically acceptable salts thereof.
41. The compound of any one of items 37-40, wherein R⁴ is H.
42. The compound of any one of items 37-40, wherein R⁴ is an esterifying group that results in OR⁴ being an ester group.
43. The compound of item 42, wherein R⁴ is -C(O)R to result in OR⁴ being -OC(O)R, wherein R is a hydrocarbon group containing 1-5 carbon atoms and optionally substituted with one or more fluorine atoms.
44. The compound of item 42, wherein R⁴ is -P(O)(OR')₂, wherein R' is independently selected from H and R, to result in OR⁴ being -OP(O)(OR')₂, wherein R is a hydrocarbon group containing 1-5 carbon atoms and optionally substituted with one or more fluorine atoms.
45. The compound of any one of items 37-44, wherein R₁ is F.
46. The compound of any one of items 37-44, wherein R₁ is Cl.
47. The compound of any one of items 37-44, wherein R₁ is methyl.
48. The compound of item 37, wherein the compound is selected from the group consisting of the following compounds: 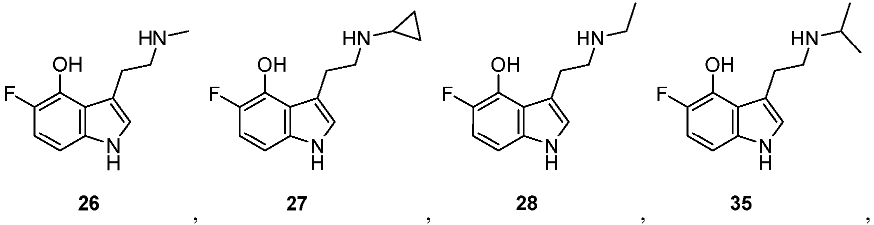 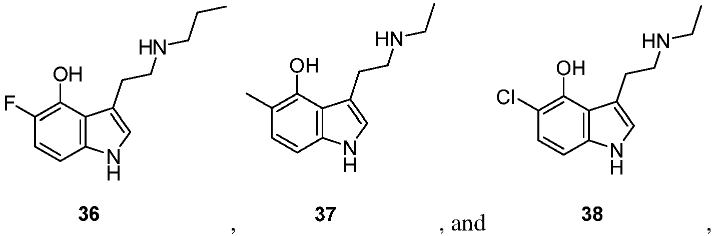 and pharmaceutically acceptable salts thereof.
49. A compound having the following structure: 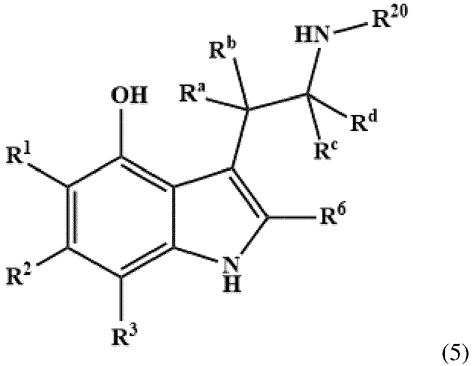 wherein:
   R¹, R³, and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms;
   R² is selected from the group consisting of: F, Cl, and methyl;
   R^{a}, R^{b}, R^{c}, and R^{d} are independently selected from H and D atoms;
   R²⁰ is selected from the group consisting of H and hydrocarbon groups containing 1-5 carbon atoms (R), which are optionally substituted with one or more fluorine atoms;
   provided that when R² is F, R³ is not methyl or OH;
   and pharmaceutically acceptable salts thereof.
50. The compound of item 49, wherein the compound has the following structure: 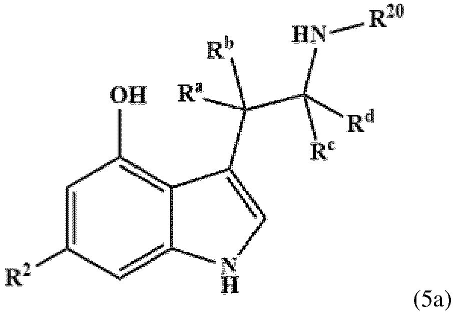 and pharmaceutically acceptable salts thereof.
51. The compound of item 49, wherein the compound has the following structure: 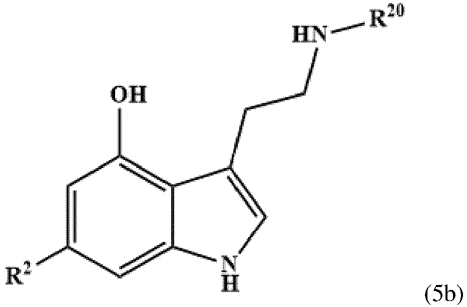 and pharmaceutically acceptable salts thereof.
52. The compound of any one of items 49-51, wherein R₂ is F.
53. The compound of any one of items 49-51, wherein R₂ is Cl.
54. The compound of any one of items 49-51, wherein R₂ is methyl.
55. The compound of item 49, wherein the compound is selected from the group consisting of the following compounds: 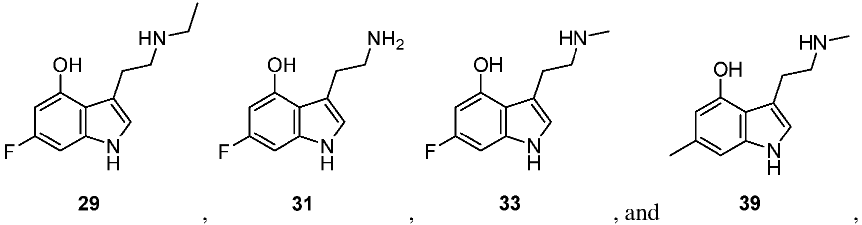 and pharmaceutically acceptable salts thereof.
56. A compound having the following structure: 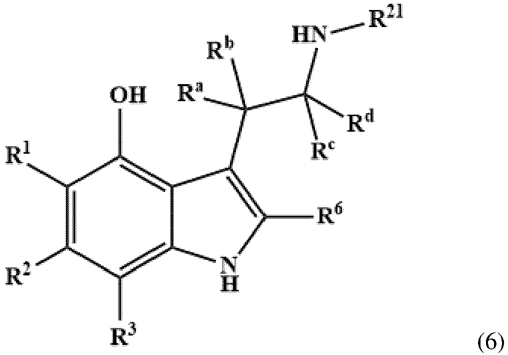 wherein:
   R¹, R², and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms;
   R³ is selected from the group consisting of: F, Cl, and methyl.
   R^{a}, R^{b}, R^{c}, and R^{d} are independently selected from H and D atoms;
   R²¹ is selected from the group consisting of H and hydrocarbon groups containing 1-5 carbon atoms (R), which are optionally substituted with one or more fluorine atoms;
   provided that when R³ is methyl, R² is not F and R⁶ is not methyl;
   further provided that when R³ is methyl, and R¹, R², R⁶, R^{a}, R^{b}, R^{c}, and R^{d} are all H, then R²¹ is not H, methyl, or ethyl;
   further provided that when R³ is For Cl, and R¹, R², R⁶, R^{a}, R^{b}, R^{c}, and R^{d} are all H, then R²¹ is not H;
   and pharmaceutically acceptable salts thereof.
57. The compound of item 56, wherein the compound has the following structure: 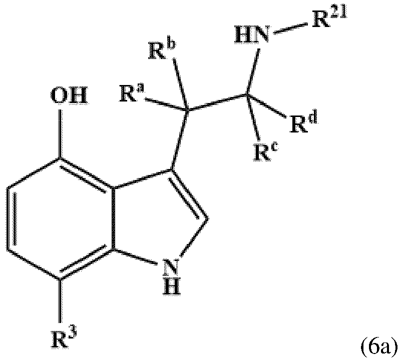 and pharmaceutically acceptable salts thereof.
58. The compound of item 56, wherein the compound has the following structure: 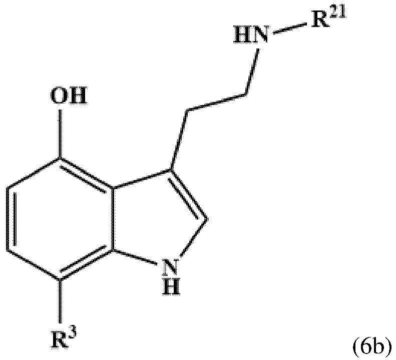 and pharmaceutically acceptable salts thereof.
59. The compound of any one of items 56-58, wherein R₃ is F.
60. The compound of any one of items 56-58, wherein R₃ is Cl.
61. The compound of any one of items 56-58, wherein R₃ is methyl.
62. The compound of item 56, wherein the compound has the following structure: 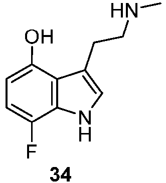 and pharmaceutically acceptable salts thereof.
63. A compound having the following structure: 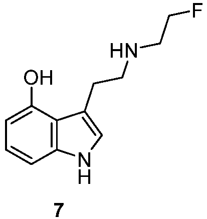 and pharmaceutically acceptable salts thereof.
64. A pharmaceutical composition comprising a compound of any one of items 1-63 and a pharmaceutically acceptable carrier.
65. A method of treating a mood disorder comprising administering a therapeutically effective amount of a compound according to any one of items 1-63 or a pharmaceutical composition according to item 64 to a subject in need thereof.
66. The method of item 65, wherein the mood disorder is selected from the group consisting of depressive disorders and bipolar disorders.
67. The method of item 65, wherein the mood disorder is a depressive disorder.
68. The method of item 67, wherein the depressive disorder is a treatment-resistant depressive disorder.
69. The method of item 65, wherein the mood disorder is selected from the group consisting of major depressive disorder, persistent depressive disorder, postpartum depression, premenstrual dysphoric disorder, seasonal affective disorder, psychotic depression, disruptive mood dysregulation disorder, substance/medication-induced depressive disorder, and depressive disorder due to another medical condition.
70. The method of item 65, wherein the mood disorder is a substance-related disorder.
71. The method of item 65, wherein the mood disorder is a substance-use disorder.
72. The method of item 65, wherein the mood disorder is an anxiety disorder.
73. The method of item 65, wherein the mood disorder is selected from the group consisting of obsessive-compulsive and related disorders, trauma- and stressor-related disorders, feeding and eating disorders, borderline personality disorder, attention-deficit/hyperactivity disorder, and autism spectrum disorder.
74. The method according to any one of items 65-73, wherein the method comprises administering about 0.5 mg to 150 mg of the compound according to any one of items 1-63.
75. The method according to any one of items 65-73, wherein the method comprises administering about 2 mg to 5 mg of the compound according to any one of items 1-63.
76. The method according to any one of items 65-73, wherein the method comprises administering about 5 mg to 10 mg of the compound according to any one of items 1-63.
77. The method according to any one of items 65-73, wherein the method comprises administering about 10 mg to 20 mg of the compound according to any one of items 1-63.
78. The method according to any one of items 65-73, wherein the method comprises administering about 20 mg to 40 mg of the compound according to any one of items 1-63.
79. The method according to any one of items 65-73, wherein the method comprises administering about 40 mg to 80 mg of the compound according to any one of items 1-63.
80. The method according to any one of items 65-73, wherein the method comprises administering about 80 mg to 100 mg of the compound according to any one of items 1-63.
81. The method according to any one of items 65-73, wherein the method comprises administering about 100 mg to 120 mg of the compound according to any one of items 1-63.
82. The method according to any one of items 65-73, wherein the method comprises administering about 120 mg to 150 mg of the compound according to any one of items 1-63.
83. The method according to any one of items 65-82, wherein the method provides improvement in at least one symptom selected from the group consisting of sadness or lethargy or lassitude, depressed mood, inability to feel, anxious worried feelings, fears, feeling tense, feeling restlessness, diminished interest in all or nearly all activities, difficulty initiating activities, significant increased or decreased appetite leading to weight gain or weight loss, insomnia, irritability, fatigue, feelings of worthlessness or low self-esteem, strongly held negative beliefs or pessimistic thoughts about self, others or world, feelings of helplessness, inability to concentrate or distractibility, recurrent thoughts of death or suicide, feelings of guilt, memory complaints, difficulty experiencing positive feelings, feeling cut off or distant from people, hypervigilance, risk taking behavior, avoidance of thoughts about a stressful or traumatic event, pains and aches, ruminations and obsessive thoughts, compulsive behaviors, talking to people you don't know well or strangers, being center of attention, disturbing intrusive thoughts, can't get through week without drug use, guilty about drug use, problems with friends or family due to drug use, and withdrawal symptoms due to drug use.
84. The method according to any one of items 65-83, wherein the compound according to any one of items 1-63 or the pharmaceutical composition of item 64 is administered via a route selected from the group consisting of oral, buccal, sublingual, inhaled mist, topical, intranasal, subcutaneous, intramuscular, and intravenous.
85. The method according to any one of items 65-84, wherein the compound according to any one of items 1-63 or the pharmaceutical composition of item 64 is administered from one to four times per day.
86. The method according to any one of items 65-84, wherein the compound according to any one of items 1-63 or the pharmaceutical composition of item 64 is administered from one to ten times per month.
87. The method according to any one of items 65-84, wherein the compound according to any one of items 1-63 or the pharmaceutical composition of item 64 is administered from one to ten times per year.

## Claims

1. A compound having the following structure: 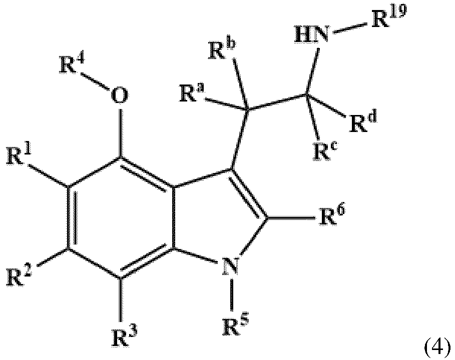 wherein:
R¹ is selected from the group consisting of: F, Cl, and methyl;
R², R³, and R⁶ are independently selected from the group consisting of: (i) H; (ii) hydrocarbon groups containing 1-5 carbon atoms (R); (iii) alkoxy groups (OR); (iv) thioalkoxy groups (SR); (v) CN; (vi) NH₂; (vii) OH; and (viii) halogen atoms;
R⁴ is selected from the group consisting of H and esterifying groups that result in OR⁴ being an ester group;
R⁵ is selected from the group consisting of H and hydrocarbon groups containing 1-5 carbon atoms (R);
R^{a}, R^{b}, R^{c}, and R^{d} are independently selected from H and D atoms;
R¹⁹ is selected from the group consisting of H and hydrocarbon groups containing 1-5 carbon atoms (R), which are optionally substituted with one or more fluorine atoms;
provided that, when R¹ is F or methyl, and R², R³, R⁴, R⁶, R¹⁹, R^{a}, R^{b}, R^{c}, and R^{d} are all H, then R⁵ is not H or methyl;
further provided that, when R¹ is Cl, and R², R³, R⁴, R⁶, R¹⁹, R^{a}, R^{b}, R^{c}, and R^{d} are all H, then R⁵ is not H, methyl, or ethyl;
further provided that, when R¹ is F, and R², R³, R⁴, R⁶, R^{a}, R^{b}, R^{c}, and R^{d} are all H, then R⁵ and R¹⁹ are not both methyl;
and pharmaceutically acceptable salts thereof.

2. The compound of claim 1, wherein the compound has the following structure: 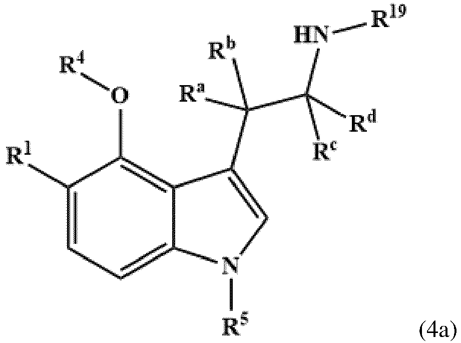 and pharmaceutically acceptable salts thereof.

3. The compound of claim 1, wherein the compound has the following structure: 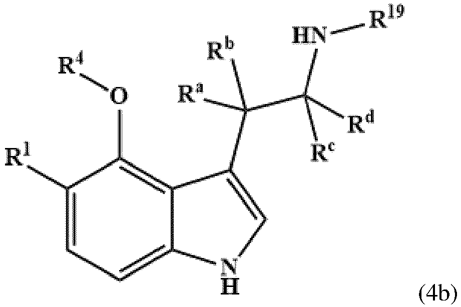 and pharmaceutically acceptable salts thereof.

4. The compound of claim 1, wherein the compound has the following structure: 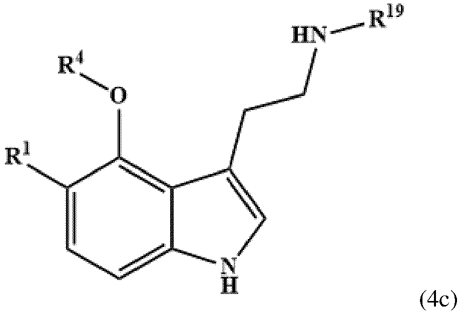 and pharmaceutically acceptable salts thereof.

5. The compound of any one of claims 1-4, wherein R⁴ is H.

6. The compound of any one of claims 1-4, wherein R⁴ is an esterifying group that results in OR⁴ being an ester group.

7. The compound of claim 6, wherein R⁴ is -C(O)R to result in OR⁴ being -OC(O)R, wherein R is a hydrocarbon group containing 1-5 carbon atoms and optionally substituted with one or more fluorine atoms.

8. The compound of claim 6, wherein R⁴ is -P(O)(OR')₂, wherein R' is independently selected from H and R, to result in OR⁴ being -OP(O)(OR')₂, wherein R is a hydrocarbon group containing 1-5 carbon atoms and optionally substituted with one or more fluorine atoms.

9. The compound of any one of claims 1-8, wherein R₁ is F.

10. The compound of any one of claims 1-8, wherein R₁ is Cl.

11. The compound of any one of claims 1-8, wherein R₁ is methyl.

12. The compound of claim 1, wherein the compound is selected from the group consisting of the following compounds: 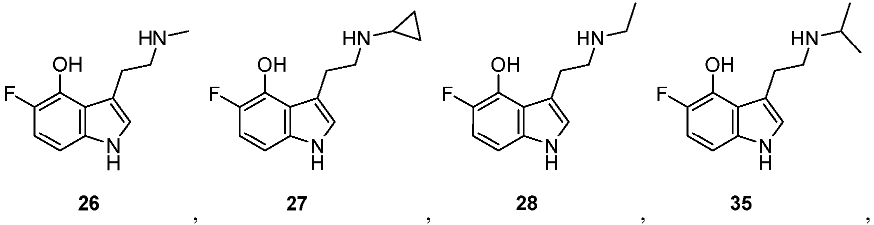 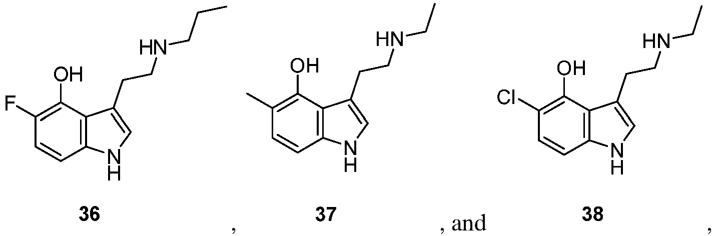 and pharmaceutically acceptable salts thereof.

13. A pharmaceutical composition comprising a compound of any one of claims 1-12 and a pharmaceutically acceptable carrier.

14. A compound according to any one of claims 1-12 or a pharmaceutical composition according to claim 13 for use in a method of treating a mood disorder, by administering a therapeutically effective amount of the compound or the pharmaceutical composition to a subject in need thereof.

15. The compound or pharmaceutical composition for use of claim 14, wherein the mood disorder is selected from the group consisting of depressive disorders and bipolar disorders.

16. The compound or pharmaceutical composition for use of claim 14, wherein the mood disorder is a depressive disorder.

17. The compound or pharmaceutical composition for use method of claim 16, wherein the depressive disorder is a treatment-resistant depressive disorder.

18. The compound or pharmaceutical composition for use of claim 14, wherein the mood disorder is selected from the group consisting of major depressive disorder, persistent depressive disorder, postpartum depression, premenstrual dysphoric disorder, seasonal affective disorder, psychotic depression, disruptive mood dysregulation disorder, substance/medication-induced depressive disorder, and depressive disorder due to another medical condition.

19. The compound or pharmaceutical composition for use of claim 14, wherein the mood disorder is a substance-related disorder.

20. The compound or pharmaceutical composition for use of claim 14, wherein the mood disorder is a substance-use disorder.

21. The compound or pharmaceutical composition for use of claim 14, wherein the mood disorder is an anxiety disorder.

22. The compound or pharmaceutical composition for use of claim 14, wherein the mood disorder is selected from the group consisting of obsessive-compulsive and related disorders, trauma- and stressor-related disorders, feeding and eating disorders, borderline personality disorder, attention-deficit/hyperactivity disorder, and autism spectrum disorder.

23. The compound or pharmaceutical composition for use according to any one of claims 14-22, wherein the method comprises administering about 0.5 mg to 150 mg of the compound according to any one of claims 1-12.

24. The compound or pharmaceutical composition for use according to any one of claims 14-22, wherein the method comprises administering about 2 mg to 5 mg of the compound according to any one of claims 1-12.

25. The compound or pharmaceutical composition for use according to any one of claims 14-22, wherein the method comprises administering about 5 mg to 10 mg of the compound according to any one of claims 1-12.

26. The compound or pharmaceutical composition for use according to any one of claims 14-22, wherein the method comprises administering about 10 mg to 20 mg of the compound according to any one of claims 1-12.

27. The compound or pharmaceutical composition for use according to any one of claims 14-22, wherein the method comprises administering about 20 mg to 40 mg of the compound according to any one of claims 1-12.

28. The compound or pharmaceutical composition for use according to any one of claims 14-22, wherein the method comprises administering about 40 mg to 80 mg of the compound according to any one of claims 1-12.

29. The compound or pharmaceutical composition for use according to any one of claims 14-22, wherein the method comprises administering about 80 mg to 100 mg of the compound according to any one of claims 1-12.

30. The compound or pharmaceutical composition for use according to any one of claims 14-22, wherein the method comprises administering about 100 mg to 120 mg of the compound according to any one of claims 1-12.

31. The compound or pharmaceutical composition for use according to any one of claims 14-22, wherein the method comprises administering about 120 mg to 150 mg of the compound according to any one of claims 1-12.

32. The compound or pharmaceutical composition for use according to any one of claims 14-31, wherein the method provides improvement in at least one symptom selected from the group consisting of sadness or lethargy or lassitude, depressed mood, inability to feel, anxious worried feelings, fears, feeling tense, feeling restlessness, diminished interest in all or nearly all activities, difficulty initiating activities, significant increased or decreased appetite leading to weight gain or weight loss, insomnia, irritability, fatigue, feelings of worthlessness or low self-esteem, strongly held negative beliefs or pessimistic thoughts about self, others or world, feelings of helplessness, inability to concentrate or distractibility, recurrent thoughts of death or suicide, feelings of guilt, memory complaints, difficulty experiencing positive feelings, feeling cut off or distant from people, hypervigilance, risk taking behavior, avoidance of thoughts about a stressful or traumatic event, pains and aches, ruminations and obsessive thoughts, compulsive behaviors, talking to people you don't know well or strangers, being center of attention, disturbing intrusive thoughts, can't get through week without drug use, guilty about drug use, problems with friends or family due to drug use, and withdrawal symptoms due to drug use.

33. The compound or pharmaceutical composition for use according to any one of claims 14-32, wherein the compound or the pharmaceutical composition is administered via a route selected from the group consisting of oral, buccal, sublingual, inhaled mist, topical, intranasal, subcutaneous, intramuscular, and intravenous.

34. The compound or pharmaceutical composition for use according to any one of claims 14-33, wherein the compound or the pharmaceutical composition is administered from one to four times per day.

35. The compound or pharmaceutical composition for use according to any one of claims 14-33, wherein the compound or the pharmaceutical composition is administered from one to ten times per month.

36. The compound or pharmaceutical composition for use according to any one of claims 14-33, wherein the compound or the pharmaceutical composition is administered from one to ten times per year.
